# EUROPEAN PATENT APPLICATION

(11) **EP 2 236 614 A2**
(43) Date of publication of application: **06.10.2010**
(21) Application number: 10168014.8
(22) Date of filing: 15.08.2003
(51) Int. Cl.: C12Q 1/00, C12Q 1/68, C12Q 1/70, G01N 33/53, G01N 33/567, G01N 33/569

(54) **Brain endothelial cell expression patterns**

(30) Priority: 15.08.2002 US 403390 P; 01.04.2003 US 458978 P
(62) Divisional of application: 03788531.6
(71) Applicant: GENZYME CORPORATION, Framingham, Massachusetts 01701 (US); The John Hopkins University, Baltimore MD 21201 (US)
(72) Inventor: Madden, Stephen, Sudbury, MA 01776 (US); Wang, Clarence, Arlington, MA 02474 (US); Cook, Brian, Northboro, MA 01532 (US); Lattera, John, Baltimore, MA 21201 (US); Walter, Kevin, Pittsburgh, PA 15213 (US)
(74) Representative: Wallis, Naomi Rachel

(57) **Abstract**

To gain a better understanding of brain tumor angiogenesis, new techniques for isolating brain endothelial cells (ECs) and evaluating gene expression patterns were developed. When transcripts from brain ECs derived from normal and malignant colorectal tissues were compared with transcripts from non-endothelial cells, genes predominantly expressed in the endothelium were identified. Comparison between normal- and tumor-derived endothelium revealed genes that were specifically elevated in tumor-associated brain endothelium. These results confirm that neoplastic and normal endothelium in human brains are distinct at the molecular level, and have significant implications for the development of anti-angiogenic therapies in the future.

## Description

This application claims the benefit of provisional applications serial numbers 60/403,390 filed August 15, 2002 and 60/458,978 filed April 1, 2003. The disclosures of each are expressly incorporated herein.

### TECHNICAL FIELD OF THE INVENTION

This invention is related to the area of angiogenesis and anti-angiogenesis. In particular, it relates to genes which are characteristically expressed in brain glioma endothelial cells.

### BACKGROUND OF THE INVENTION

Brain cancers represent an infrequent but deadly form of cancer that has seen little improvement in survivability over the last 30 years. Tumor excision followed by therapies relying on outdated cytotoxins and radiation inevitably results in a diminished quality of life. Gliomas represent the most common brain neoplasms with highly vascular and invasive characteristics defining gliomas as one of the most aggressive tumors known. Classifications of gliomas derive from both the cellular origin and staged aggressiveness. Derived from either astrocytes or oligodendrocytes, astrocytomas and oligodendrogliomas constitute the most common types of gliomas. As is common to other tumor type classifications, glioma increases in aggressiveness from the first to third stages of disease with stage IV, gliobastoma multiforme, being the most aggressive. Moreover, glioblastoma tumors constitute one of the most vascular tumors known.

Vascular permeability within the brain is limited in comparison to other organs. Similarily, the accessibility of brain malignancies to immune surveillance was thought to be restricted as well although more recent evidence suggests the brain is not wholly immunologically privileged. This so called "blood-brain barrier" is thought to derive primarily from a combination of brain-specific capillary transport systems and astrocyte-regulated cross-talk with the endothelial cell-based vasculature (for reviews, see Bart, J., Groen, H. J., Hendrikse, N. H., van der Graaf, W. T., Vaalburg, W., and de Vries, E. G. (2000). The blood-brain barrier and oncology: new insights into function and modulation. Cancer Treat Rev 26, 449-62.) The presence of tight junctions and an observed high electrical resistance both contribute to restricted transvascular molecular exchange. The existence of a therapeutically impermeable vasculature has resulted in a comparatively limited amount of work aimed at intervening in brain malignancies and other CNS diseases. Defining proteins preferentially expressed on either normal or diseased brain endothelial cells holds promise for expanding CNS therapeutic regimens.

The vascular microenvironment within gliomas has been studied primarily through morphological, circulatory and perfusion based experiments (for review see Vajkoczy, P., and Menger, M. D. (2000). Vascular microenvironment in gliomas. J Neurooncol 50, 99-108; and Bart, J., Groen, H. J., Hendrikse, N. H., van der Graaf, W. T., Vaalburg, W., and de Vries, E. G. (2000). The blood-brain barrier and oncology: new insights into function and modulation. Cancer Treat Rev 26, 449-62.) These studies demonstrate profound changes in vascualture architecture associated with tumor progression. Increased fenestrations, malperfusion, hyperpermeability, and reduced leukocyte-EC interaction are all phenotypic observations linked to glioma microvasculature Bernsen, H. J., Rijken, P. F., Oostendorp, T., and van der Kogel, A. J. (1995). Vascularity and perfusion of human gliomas xenografted in the athymic nude mouse. Br J Cancer 71, 721-6; Vick, N. A., and Bigner, D. D. (1972). Microvascular abnormalities in virally-induced canine brain tumors. Structural bases for altered blood-brain barrier function. J Neurol Sci 17, 29-39; and Hobbs, S. K., Monsky, W. L., Yuan, F., Roberts, W. G., Griffith, L., Torchilin, V. P., and Jain, R. K. (1998). Regulation of transport pathways in tumor vessels: role of tumor type and microenvironment. Proc Natl Acad Sci U S A 95, 4607-12. It is also suggested that higher grade gliomas utilize intussuceptive capillary growth to a much larger degree than earlier staged gliomas that primarily utilize both sprouting an cooption to advance vessel growth. Vajkoczy, P., Schilling, L., Ullrich, A., Schmiedek, P., and Menger, M. D. (1998). Characterization of angiogenesis and microcirculation of high-grade glioma: an intravital multifluorescence microscopic approach in the athymic nude mouse. J Cereb Blood Flow Metab 18, 510-20. The molecular characterization of glioma ECs has thus far been limited to the evaluation of common growth factors or previously defined brain EC transporters. Holash, J., Maisonpierre, P. C., Compton, D., Boland, P., Alexander, C. R., Zagzag, D., Yancopoulos, G. D., and Wiegand, S. J. (1999). Vessel cooption, regression, and growth in tumors mediated by angiopoietins and VEGF. Science 284, 1994-8; Guerin, C., Wolff, J. E., Laterra, J., Drewes, L. R., Brem, H., and Goldstein, G. W. (1992). Vascular differentiation and glucose transporter expression in rat gliomas: effects of steroids. Ann Neurol 31, 481-7.

To date, global gene expression profiles from endothelial cell-specific populations is limited to normal and tumorigenic colon tissue. St Croix, B., Rago, C., Velculescu, V., Traverso, G., Romans, K. E., Montgomery, E., Lal, A., Riggins, G. J., Lengauer, C., Vogelstein, B., and Kinzler, K. W. (2000). Genes expressed in human tumor endothelium. Science 289, 1197-202.
There is a need in the art for analysis of endothelial cells from other tissue, so that diagnostic and therapeutic for non-colonic tumors can be developed.

### SUMMARY OF THE INVENTION

According to one embodiment of the invention a method is provided to aid in diagnosing glioma. An expression product of at least one gene in a first brain tissue sample suspected of being neoplastic is detected. The at least one gene is selected from the group consisting of signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(sp1); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein 516; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; 5100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I;
and Homo sapiens, clone IMAGE:3908182, mRNA, partial cds. Expression of the at least one gene in the first brain tissue sample is compared to expression of the at least one gene in a second brain tissue sample which is normal. Increased expression of the at least one gene in the first brain tissue sample relative to the second tissue sample identifies the first brain tissue sample as likely to be neoplastic.
According to another embodiment of the invention a method is provided of treating a glioma. Cells of the glioma are contacted with an antibody. The antibody specifically binds to an extracellular epitope of a protein selected from the group consisting of plasmalemma associated protein; KIAA0726 gene product; osteonectin: laminin, alpha 5; collagen, type IV, alpha 1; insulin-like growth factor binding protein 7; Thy-1 cell surface antigen; dysferlin, limb girdle muscular dystrophy 2B; integrin, alpha 5; matrix metalloproteinase 9; Lutjheran blood group, integrink, alpha 10, collagen, type VI, alpha 2; glioma endothelial marker 1 precursor; translocase of inner mitochondrial membrane 17 homolog A; heparan sulfate proteoglycan 2; annexin A2; matrix metalloproteinase 10; G protein-coupled receptor; matrix metalloproteinase 14; solute carrier family 29, member 1; CD59 antigen p18-20; KIAA 1870 protein; plexin B2; lectin, glactoside-binding, soluble, 8; integrin beta 4 binding protein; acetyl LDL receptor; laminin, gamma 3; macrophage migration inhibitory factor; gap junction p roein, alpha 1, 43 kD; aquaporin 1; protease, serine, 11; collagen, type IV, alpha 2; apolipoprotein D; plasminogen activator, urokinase; insulin-like growth factor binding protein 3; regulator of G-protein signaling 12; prosaposin; laminin, alpha 4; lectin, galactoside-binding, soluble, 3 binding protein; glycophorin C; endothelin receptor type B; biglycan; transmembrane 4 superfamilyh member 2; lysyl osidase-like 2; TEK tyrosine kinase, endothelial; insulin receptore; cell membrane glycoprotein, 110000M( r ); jagged 1; plasmalemma vesicle associated protein; TEM13, Thy-1 cell surface antigen; coagulation factor II (thrombin) receptor-like 3; dysferlin, limb girdle muscular dystrophy 2B (autosomal recessive); sema domain, transmembrane domain (TM), and cytoplasmic domain, (semaphorin) 6B; integrin, alpha 5 (fibronectin receptor, alpha polypeptide); likely ortholog of rat vacuole membrane protein 1; nerve growth factor receptor (TNFR superfamily, member 16); degenerative spermatocyte homolog, lipid desaturase (Drosophila); TEM1, endosialin; heme oxygenase (decycling) 1; G protein-coupled receptor; C-type (calcium dependent, carbohydrate-recognition domain) lectin, superfamily member 9; matrix metalloproteinase 14 (membrane-inserted); solute carrier family 29 (nucleoside transporters), member 1; likely ortholog of mouse embryonic epithelial gene 1; major histocompatibility complex, class I, C; likely ortholog of mouse fibronectin type III repeat containing protein 1; sprouty homolog 4 (Drosophila); KIAA0620 protein; coagulation factor III (thromboplastin, tissue factor); aquaporin 1 (channel-forming integral protein, 28kDa); major histocompatibility complex, class I, B; Lysosomal-associated multispanning membrane protein-5; endothelin receptor type B; insulin receptor; complement component 1, q subcomponent, receptor 1; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16)and complement component 1, q subcomponent, receptor 1. Immune destruction of cells of the glioma is thereby triggered.

According to still another embodiment of the invention a method is provided for identifying a test compound as a potential anti-cancer or anti-glioma drug. A test compound is contacted with a cell which expresses at least one gene selected from the group consisting of: signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(spl); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; S100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds. An expression product of the at least one gene is monitored. The test compound is identified as a potential anti-cancer drug if it decreases the expression of the at least one gene.

According to yet another embodiment of the invention a method is provided to aid in diagnosing glioma. An mRNA of at least one gene in a first brain tissue sample suspected of being neoplastic is detected. The at least one gene is identified by a tag selected from the group consisting of SEQ ID NO: 1-32. Expression of the at least one gene in the first brain tissue sample is compared to expression of the at least one gene in a second brain tissue sample which is normal. If increased expression of the at least one gene in the first brain tissue sample relative to the second tissue sample if found, the first brain tissue sample is identified as likely to be neoplastic.

Another embodiment of the invention is a method of identifying a test compound as a potential anti-cancer or anti-glioma drug. A test compound is contacted with a cell. The cell expresses an mRNA of at least one gene identified by a tag selected from the group consisting of SEQ ID NO: 1-32. An mRNA of the at least one gene is monitored. The test compound is identified as a potential anti-cancer drug if it decreases the expression of at least one gene.

Still another embodiment of the invention is a method to induce an immune response to glioma. A protein or nucleic acid encoding a protein is administered to a mammal, preferably a human. The protein is selected from the group consisting of: signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(sp1); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7 hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; S100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds. An immune response to the protein is thereby induced.

The present invention thus provides the art with methods of diagnosing and treating gliomas and other brain tumors.

### DETAILED DESCRIPTION OF THE INVENTION

Using SAGE (Serial Analysis of Gene Expression) profiling, this study was able to identify previously unrecognized, angiogenesis-specific markers that discriminate between non-proliferative and pathologic endothelial cells. We identified 255 human genes that were expressed at significantly higher levels in brain tumor endothelium than in normal brain endothelium. See Table 1. We have named these markers GEMs (glioma **e**ndothelial **m**arkers). Any of the GEMs disclosed in any of the tables can be used in the methods of the present invention, according to the discretion of the skilled artisan.

ECs represent only a minor fraction of the total cells within normal or tumor tissues, and only those EC transcripts expressed at the highest levels would be expected to be represented in libraries constructed from unfractionated tissues. The genes described in the current study should therefore provide a valuable resource for basic and clinical studies of human brain angiogenesis in the future. Genes which have been identified as expressed more in glioma endothelial cells than in normal brain endothelial cells (GEMS) include those which correspond to tags shown in SEQ ID NOS: 1-32. The tags correspond to the segment of the cDNA that is 3' of the 3' most restriction endonuclease site for the restriction enzyme NlaIII which was used as the anchoring enzyme. The tag shown is the same strand as the mRNA. Other such genes are listed in Tables 1 and 2.

**Table 1**

| **StdTag** | **SEQ** | **LongTag** | **SEQ ID** | **Function** |
|---|---|---|---|---|
| AAACCATTCT | 1 | AAACCATTCTCCTCCGC | 256 | |
| AAGGCAGGGA | 2 | AAGGCAGGGAGGGAGG | 257 | |
| ACACAGCAAG | 3 | ACACAGCAAGACGAGAA | 258 | |
| AGCTGGAGTC | 4 | AGCTGGAGTCCTAGGCA | 259 | |
| AGCTGGCACC | 5 | AGCTGGCACCAGAGCCC | 260 | |
| ATAAATGAGG | 6 | ATAAATGAGGTAAGGTC | 261 | |
| CAAGCACCCC | 7 | CAAGCACCCCCGTTCCA | 262 | |
| CACTACCCAC | 8 | CACTACCCACCAGACGC | 263 | |
| CACTACTCAC | 9 | CACTACTCACCAGGCGC | 264 | |
| CCCACCTCCA | 10 | CCCACCTCCAGTCCAGC | 265 | |
| CCCGCCTCTT | 11 | CCCGCCTCTTCACGGGC | 266 | |
| CCTCAGA TGT | 12 | CCTCAGATGTTTGAAAA | 267 | |
| CGCTACTCAC | 13 | CGCTACTCACCAGACGC | 268 | |
| CTAAGACCTC | 14 | CTAAGACCTCACCAGTC | 269 | |
| CTAAGACTTC | 15 | CTAAGACTTCACCGGTC | 270 | |
| GAGTGGGTGC | 16 | GAGTGGGTGCAGCCTCC | 271 | |
| GGGACAGCTG | 17 | GGGACAGCTGTCTGTGG | 272 | |
| GGGTTGGCTT | 18 | GGGTTGGCTTGAAACCA | 273 | |
| GTAAGTGTAC | 19 | GTAAGTGTACTGGAAGT | 274 | |
| GTAAGTGTAC | 20 | GTAAGTGTACTGGTAAG | 275 | |
| GTAGGGGTAA | 21 | GTAGGGGTAAAAGGAGG | 276 | |
| TAACCACTGC | 22 | TAACCACTGCACTTTCC | 277 | |
| TACTGCTCGG | 23 | TACTGCTCGGAGGTCGG | 278 | |
| TCAGGCTGAA | 24 | TCAGGCTGAAGTCAGGC | 279 | |
| TCCATACACC | 25 | TCCATACACCTATCCCC | 280 | |
| TCCTTTTAAA | 26 | TCTTTTAAAACAAAAC | 281 | |
| TGATTAAGGT | 27 | TGATTAAGGTCGGCGCT | 282 | |
| TGGTATCACA | 28 | TGGTATCACACAAGGGG | 283 | |
| TGGTGTATGC | 29 | TGGTGTATGCATCGGGG | 284 | |
| TGTCACTGGG | 30 | TGTCACTGGGCAGGCGG | 285 | |
| TGTGGGAGGC | 31 | TGTGGGAGGCTGATGGG | 286 | |
| TTTAACGGCC | 32 | TTTAACGGCCGCGGTAC | 287 | |
| GCTCTCTATG | 33 | GCTCTCTATGCTGACGT | 288 | signal sequence receptor, delta (translocon-associated protein delta) |
| AGAATGAAAC | 34 | AGAATGAAACTGCCGGG | 289 | DC2 protein |
| AAGTGGAATAH | 35 | AGTGGAATAAACTGCC | 290 | KIAA0404 protein |
| GATGACGACT | 36 | GATGACGACTCGGGGCT | 291 | symplekin; Huntingtin interacting protein I |
| CCCTTTCACA | 37 | CCCTTTCACACACACTT | 292 | plasmalemma vesicle associated protein |
| TCCTGGGGCA | 38 | TCCTGGGGCAGGGGCG | 293 | KIAA0726 gene product |
| TCTATTGATG | 39 | TCTATTGATGTGTATGC | 294 | latexin protein |
| GGGGCTGTAT | 40 | GGGGCTGTATTTAAGGA | 295 | transforming growth factor, beta 1 |
| CCCAGGACAC | 41 | CCCAGGACACCAGCTGG: | 296 | hypothetical protein FLJ22215 |
| GGAGCTGCTG | 42 | GGAGCTGCTGCTTGTGG | 297 | Rag C protein |
| TGGACAGCAG | 43 | TGGACAGCAGGGACCTG | 298 | hypothetical protein FLJ23471 |
| TCTGGGAACAH | 44 | TCTGGGAACAGGGACGG | 299 | N-myristoltransferase 1 |
| CCTGTGTATG | 45 | CCTGTGTATGTGTGTAA | 300 | hypothetical protein dJ1181N3.1 |
| GGCAAGAAGA | 46 | GGCAAGAAGAAGATCGC | 301 | ribosomal protein L27 |
| AAATGCTTGG | 47 | AAATGCTTGGAGGTGAA | 302 | secreted protein, acidic, cysteine-rich (osteonectin) |
| CTAAAAACCT | 48 | CTAAAAACCTTATGACA | 303 | secreted protein, acidic, cysteine-rich (osteonectin) |
| GAGCATTGCA | 49 | GAGCATTGCACCACCCG | 304 | secreted protein, acidic, cysteine-rich (osteonectin) |
| GGTGGACACG | 50 | GGTGGACACGGATCTGC | 305 | secreted protein, acidic, cysteine-rich (osteonectin) |
| GCTCCTGAGC | 51 | GCTCCTGAGCCCCGGCC | 306 | ESTs, Weakly similar to 165992 gene MLL protein [H.sapiens] |
| AAGAAGTGGAHH | 52 | AAGAAGTGGAGATTGTC | 307 | ESTs |
| TGGGAAGTGG | 53 | TGGGAAGTGGGCTCCTT | 308 | maternally expressed 3 |
| ACTCGCTCTG | 54 | ACTCGCTCTGTGGAGGT | 309 | laminin, alpha 5 |
| TTTCAGGGGA | 55 | TTTCAGGGGAGGGGGAA | 310 | protective protein for beta-galactosidase (galactosialidosis) |
| ACAACGTCCA | 56 | ACAACGTCCAGCTGGTG | 311 | Melanoma associated gene |
| GTCTCAGTGC | 57 | GTCTCAGTGCTGAGGCG | 312 | Melanoma associated gene |
| CCCCCTGCCC | 58 | CCCCCTGCCCCTCTGCC | 313 | E3 ubiquitin ligase SMURF1 |
| AGAAACCACG | 59 | AGAAACCACGGAAATGG | 314 | collagen, type IV, alpha 1 |
| GACCGCAGGA | 60 | GACCGCAGGAGGGCAGA | 315 | collage type IV, alpha 1 |
| GTGCTACTTC | 61 | GTGCTACTTCTTCTTCT | 316 | collagen, type IV, alpha 1 |
| GATAACTACA | 62 | GATAACTACATTACCTG | 317 | Nnsulin-iike growth factor binding protein |
| TGGCTGTGAC | 63 | TGGCTGTGACTGTGACT | 318 | gene predicted from cDNA with a complete coding sequence |
| GAGTGAGACC | 64 | GAGTGAGACCCAGGAGC | 319 | Tthy-1 cell surface antigen |
| GAGTGGCTAC | 65 | GAGTGGCTACCCGCCGC | 320 | ESTs, Weakly similar to T28770 hypothetical protein W03D2.1-Caenorhabditis elegans |
| GACTCAGGGA | 66 | GACTCAGGGATTTGTTG | 321 | GTP binding protein 2 |
| GTTATATGCC | 67 | GTTATATGCCCGGGAGA | 322 | Homo sapiens mRNA; cDNA DKFZp586D0918 (from clone DKFZp586D0918) |
| GAGGCGCTGC | 68 | GAGGCGCTGCTGCCACC | 323 | cutaneous T-cell lymphoma-associated tumor antigen se20-4; differentially expressed nucleolar TGF-beta1 target protein (DENTT) |
| GAGCTCTGAG | 69 | GAGCTCTGAGATCACCC | 324 | dysferlin, limb girdle muscular dystrophy 2B (autosomal recessive) |
| GCCAGCCAGT | 70 | GCCAGCCAGTGGCAAGC | 325 | Smoothelin |
| ATGGCAACAG | 71 | ATGGCAACAGATCTGGA | 326 | integrin, alpha 5 (fibronectin receptor, alpha polypeptide) |
| AAGGAGTTAC | 72 | AAGGAGTTACACTAGTC | 327 | putative translation initiation factor |
| TCCCACAAGG | 73 | TCCCACAAGGCTGCTTG | 328 | retinoic acid induced 14 |
| TAAATCCCCA | 74 | TAAATCCCCACTGGGAC | 329 | matrix metalloproteinase 9 (gelatinase B, 92kD gelatinase, 92kD type IV collagenase) |
| CCCGCCCCCG | 75 | CCCGCCCCCGCCTTCCC | 330 | Lutheran blood group (Auberger b antigen included) |
| CCCGAGGCAG | 76 | CCCGAGGCAGAGTCGGG | 331 | stanniocalcin 2 |
| CTACGTGATG | 77 | CTACGTGATGAAGATGG | 332 | nuclear factor (erythroid-derived 2)-like 2 |
| ATGGGTTTGC | 78 | ATGGGTTTGCATTTTAG | 333 | protein tyrosine phosphatase, non-receptor type 1 |
| GGCATTGTCT | 79 | GGCATTGTCTCTGTTTC | 334 | integrin, alpha 10 |
| GTGCTAAGCG | 80 | GTGCTAAGCGGGCCCGG | 335 | Collagen, type alpha 2 |
| ACCGTTTGCA | 81 | ACCGTTTGCATTCGAAA | 336 | chromosome 21 open reading frame 25 |
| CAGCGCTGCA | 82 | CAGCGCTGCATTGACTC | 337 | CDC37 (cell division cycle 37, S. cerevisiae, homolog) |
| GAAGACACTT | 83 | GAAGACACTTGGTTTGA | 338 | ESTs |
| CGCTGGGCGT | 84 | CGCTGGGCGTCTGGGAC | 339 | Rho guanine nucleotide exchange factor (GEF) 7 |
| CACCCCTGAT | 85 | CACCCCTGATGTTCGCC | 340 | creatine kinase, brain |
| GCCCCCCTGC | 86 | GCCCCCCTGCCCCGTGC | 341 | hypothetical protein FLJ10297 |
| CCCCCTGCCC | 87 | CCCCCTGCCCTCGCCTG | 342 | hypothetical protein FLJ10350 |
| AGCATAAAAA | 88 | AGCATAAAAATGCGTGC | 343 | TNF-induced protein |
| GGGCTGGACG | 89 | GGGCTGGACGGCTGCGT | 344 | tumor necrosis factor receptor superfamily, member 12 (translocating chain-association membrane protein) |
| CTGCCAACTT | 90 | CTGCCAACTTCTAACCG | 345 | cofilin 1 (non-muscle) |
| AAGTGGATAG | 91 | AAGTGGATAGATACTTC | 346 | splicing factor proline/glutamine rich (polypyrimidine tract-binding protein-associated) |
| CGTACTGAGC | 92 | CGTACTGAGCGCTTTGG | 347 | splicing factor proline/glutamine rich (polypyrimidine tract-binding protein-associated) |
| CCGCTTACTC | 93 | CCGCTTACTCTGTTGGG | 348 | v-ets avian erythroblastosis virus E26 oncogene homolog 1 |
| GGGGCTTCTG | 94 | GGGGCTTCTGTAGCCCC | 349 | protease, cysteine, 1 (legumain) |
| CCCGTCCGGA | 95 | CCCGTCCGGAACGTCTA | 350 | ribosomal protein L13 |
| AGTTCCACCA | 96 | AGTTCCACCAGAAAGCC | 351 | chromosome 22 open reading frame 5 |
| GGCCTCCAGC | 97 | GGCCTCCAGCCACCCAC | 352 | zinc finger protein 144 (Mel-18) |
| GGAGGCTGAG | 98 | GGAGGCTGAGGTGGGA | 353 | degenerative spermatocyte (homolog Drosophila; lipid desaturase) |
| CAGAGGCGTC | 99 | CAGAGGCGTCCGCAGGT | 354 | eukaryotic translation initiation factor 2C, 2 |
| GACCAGCCTT | 100 | GACCAGCCTTCAGATGG | 355 | mitochondrial ribosomal protein L45 |
| GAGGATGGTG | 101 | GAGGATGGTGTCCTGAG | 356 | prostate tumor over expressed gene 1 |
| TCGTCGCAGA | 102 | TCGTCGCAGAAGGCGCT | 357 | NADH dehydrogenase (ubiquinone) 1 alpha subcomplex, 7 (14.5kD, B14.5a) |
| GGGGCTGCCC | 103 | GGGGCTGCCCAGCTGGA | 358 | tumor endothelial marker 1 precursor |
| CTGTACATAC | 104 | CTGTACATACTTTTTGG | 359 | NS1-binding protein |
| GCGACGAGGC | 105 | GCGACGAGGCGCGCTG | 360 | ribosomal protein L38 |
| GCCAAGTGAA | 106 | GCCAAGTGAACTGTGGC | 361 | protein |
| AAGATAAACT | 107 | AAGATAAACTCTGGGCC | 362 | HLA class II region expressed gene KE2 |
| GAGAGTGTAC | 108 | GAGAGTGTACTGGCACT | 363 | of inner mitochondrial membrane 17 homolog A (yeast) |
| CCACTGCACT | 109 | CCACTGCACTCCGGCCT | 364 | sudD (suppressor of bimD6, Aspergillus nidulans) homolog |
| CCACCCTCAC | 110 | CCACCCTCACACACACA | 365 | heparan sulfate proteoglycan 2 (perlecan) |
| CAGACCATTG | 111 | CAGACCATTGTTTGATC | 366 | SEC24 (S. cerevisiae) related gene family, member A |
| GGGAGCTGCG | 112 | GGGAGCTGCGCCAACGG | 367 | NADH dehydrogenase (ubiquinone) Fe-S protein 7 (20kD) (NADH-coenzyme Q reductase) |
| GGGATTTCTG | 113 | GGGATTTCTGTGTCTGC | 368 | DNA segment on chromosome X and Y (unique) 155 expressed sequence |
| CTTCCAGCTA | 114 | CTTCCAGCTAACAGGTC | 369 | annexin A2 |
| CAGAAACAGA | 115 | CAGAAACAGACTGGGGG | 370 | Homo sapiens clone 24670 mRNA sequence |
| TCTGTGCTCA | 116 | TCTGTGCTCAGGAAGAG | 371 | hypothetical protein |
| TGCAATAGGT | 117 | TGCAATAGGTGAGAGAA | 372 | matrix metalloproteinase 10 (stromelysin 2) |
| ATGGCCAACT | 118 | ATGGCCAACTTCCACCT | 373 | KIAA 1049 protein |
| TCACACAGTG | 119 | TCACACAGTGCCTGTCG | 374 | G protein-coupled receptor |
| GGCTTAGGAT | 120 | GGCTTAGGATGTGAATG | 375 | hypothetical protein FLJ20401 |
| GGGAGGGGTG | 121 | GGGAGGGGTGGGGGTG | 376 | matrix metalloproteinase 14 (membrane-inserted) |
| GAAGTAGAAG | 122 | GAAGTAGAAGGTAAGGA | 377 | KIAA0470 gene product |
| CACCCTGTAC | 123 | CACCCTGTACAGTTGCC | 378 | solute carrier family 29 (nucleoside transporters), member 1 |
| ATGTTTACAA | 124 | ATGTTTACAAGATGGCG | 379 | stanniocalcin 1 |
| CAAACTGGTC | 125 | CAAACTGGTCTAGGTCA | 380 | stanniocalcin 1 |
| GTAATGACAG | 126 | GTAATGACAGATGCAAG | 381 | stanniocalcin 1 |
| ACCTGCCGAC | 127 | ACCTGCCGACAGTGTTG | 382 | tumor suppressor deleted in oral cancer-related 1 |
| TGATGCGCGC | 128 | TGATGCGCGCTTTGTTG | 383 | tumor suppressor deleted in oral cancer-related 1 |
| TGGCCCCAGG | 129 | TGGCCCCAGGTGCCACC | 384 | apolipoprotein C-1 |
| GCCTGCTGGG | 130 | GCCTGCTGGGCTTGGCT | 385 | glutathione peroxidase 4 (phospholipid hydroperoxidase) |
| TGCCTGTGGT | 131 | TGCCTGTGGTCCCAGCT | 386 | ESTs |
| GAGGGTATAC | 132 | GAGGGTATACTGAGGGG | 387 | transcription factor binding to IGHM enhancer 3 |
| GGAGCCAGCT | 133 | GGAGCCAGCTGACCTGC | 388 | hypothetical protein DKFZp762A227 |
| GAGCCTCAGG | 134 | GAGCCTCAGGTGCTCCC | 389 | hypothetical protein FLJ22362 |
| TACTTCACAT | 135 | TACTTCACATACAGTGC | 390 | CD59 antigen p18-20 (antigen identified by monoclonal antibodies 16.3A5, EJ16, EJ30, EL32 and G344) |
| TAATCCCAGC | 136 | TAATCCCAGCACTTTGG | 391 | PRO0628 protein |
| CACCTTCCAG | 137 | CACCTTCCAGCCCGGGG | 392 | melanoma-associated antigen recognised by cytotoxic T lymphocytes |
| GAGTCTGTTC | 138 | GAGTCTGTTCGTGACTC | 393 | LOC88745 |
| GGATTTTGGT | 139 | GGATTTTGGTCTCTGTC | 394 | Homo sapiens beta-1,3-galactosyltransferase-6 (B3GALT6) mRNA, |
| TGCCTGTAGT | 140 | TGCCTGTAGTCCTAGTT | 395 | sprouty (Drosophila) homolog 4 |
| TTACAAACAG | 141 | TTACAAACAGAAAAGCT | 396 | sprouty (Drosophila) homolog 4 |
| TCTTCTTTCA | 142 | TCTTCTTTCAGAATGGG | 397 | Homo sapiens mRNA; cDNA DKFZp434E1515 (from clone |
| AGCACATTTG | 143 | AGCACATTTGATATAGC | 398 | coactosin-like protein |
| CAGGGCTCGC | 144 | CAGGGCTCGCGTGCGG | 399 | hypothetical protein FLJ21865 |
| GCTGGTCCCA | 145 | GCTGGTCCCAGGGCCAG | 400 | ESTs, Weakly similar to T31613 hypothetical protein Y50E8A.i-Caenorhabditis elegans [C.elegans] |
| TCCACGCCCT | 146 | TCCACGCCCTTCCTGGC | 401 | KIAA0685 gene product |
| TTGCAATAGC | 147 | GCAATAGCAAAACCC | 402 | hypothetical protein FLJ10980 |
| AGGGCTTCCA | 148 | AGGGCTTCCAATGTGCT | 403 | ribosomal protein L10 |
| CTGGGTTAAT | 149 | CTGGGTTAATAAATTGC | 404 | ribosomal in S19 |
| AACCTGGGAG | 150 | AACCTGGGAGGTGGAGG | 405 | ESTs |
| GGCAACGTGG | 151 | GGCAACGTGGTAGAGGC | 406 | Huntingtin interacting protein K |
| GGATGCGCAG | 152 | GGATGCGCAGGGGAGG | 407 | Homo sapiens mRNA full length insert cDNA clone EUROIMAGE 50374 |
| CACCTGTAGT | 153 | CACCTGTAGTCCTAGCT | 408 | EST |
| GTGGTGGGCG | 154 | GTGGTGGGCGCCTGTAG | 409 | EST |
| GCAGGGTGGG | 155 | GCAGGGTGGGGAGGGG | 410 | v-akt murine thymoma viral oncogene homolog 2 |
| CAAGCATCCC | 156 | CAAGCATCCCCGTTCCA | 411 | EST |
| TGGGGGCCGA | 157 | TGGGGGCCGATGGGCA | 412 | transducin-like enhancer of split 2, homolog of Drosophila E(sp1) |
| TCAGTGTATT | 158 | TCAGTGTATTAAAACCC | 413 | KIAA1870 protein |
| GGCAAGCCCC | 159 | GGCAAGCCCCAGCGCCT | 414 | ribosomal protein L10a |
| CCTAGCTGGA | 160 | CCTAGCTGGATTGCAGA | 415 | peptidylprolyl isomerase A (cyclophilin A) |
| GCAAAACCCT | 161 | GCAAAACCCTGCTCTCC | 416 | ESTs, Weakly similar to ubiquitous TPR motif, Y isoform [H.sapiens] |
| GCTGGTTCCT | 162 | GCTGGTTCCTGAGTGGC | 417 | hypothetical protein FLJ23239 |
| GCACCTCAGC | 163 | GCACCTCAGCCAGGGGT | 418 | hypothetical protein DKFZp761H221 |
| ACCAGCTGTC | 164 | ACCAGCTGTCCAGGGGC | 419 | KIAA1887 protein |
| TTTGAATCAG | 165 | TTTGAATCAGTGCTAGA | 420 | Homo sapiens mRNA full length insert cDNA clone EUROIMAGE 701679 |
| AGACTAGGGG | 166 | AGACTAGGGGCCGGAGC | 421 | Homo sapiens cDNA FLJ30634 fis, clone CTONG2002453 |
| AGCTCAGTGA | 167 | AGCTCAGTGAGAAGGGC | 422 | Homo sapiens cDNA FLJ32203 fis, clone PLACE6003038, weakly similar to ZINC FINGER PROTEIN 84 |
| GGCCAACATT | 168 | GGCCAACATTTGGTCCA | 423 | Homo sapiens mRNA full length insert cDNA clone EUROIMAGE 1035904 |
| TTTGTGGGCA | 169 | TTTGTGGGCAGTCAGGC | 424 | hypothetical protein LOC57333 |
| ATTGTAGACA | 170 | ATTGTAGACAATGAGGG | 425 | myosin ID |
| CCCTAGGTTG | 171 | CCCTAGGTTGGGCCCCT | 426 | plexin B2 |
| AAATCACCAA | 172 | AAATCACCAATCAAGGC | 427 | lectin, galactoside-binding, soluble, 8 (galectin 8) |
| GGCTGCAGTC | 173 | GGCTGCAGTCTTCTTCC | 428 | double ing-finger protein, Dorfin |
| GTGGCAGDCG | 174 | GTGGCAGGCGCCTGTAG | 429 | DKFZP434B168 protein |
| TAAAGGCACA | 175 | TAAAGGCACAGTGGCTC | 430 | LIM domain binding 2 |
| GGCTCCTGGC | 176 | GGCTCCTGGCTCTGGAC | 431 | integrin beta 4 binding protein |
| ATATTAGGAA | 177 | ATATTAGGAAGTCGGGG | 432 | Synaptopodin |
| GCTTCAGTGG | 178 | GCTTCAGTGGGGGAGAG | 433 | ESTs |
| TGATTAAAAC | 179 | TGATTAAAACAAGTTGC | 434 | insulin induced gene |
| ASGCCACCACG | 180 | AGCCACCACGCCTGGTC | 435 | acetyl LDL receptor; SREC |
| GGCGGCTGCA | 181 | GGCGGCTGCAGAGCCTG | 436 | excision repair cross-complementing rodent repair deficiency, complementation group 1 (includes overlapping antisense sequence) |
| TGTTTGGGGG | 182 | TGTTTGGGGGCTTTTAG | 437 | hypothetical protein FLJ22329 |
| CCTGCCTCGT | 183 | CCTGCCTCGTAGTGAAG | 438 | schwannomin-interacting protein 1 |
| AGGCCTGGGC | 184 | AGGCCTGGGCCTCTGCG | 439 | PTEN induced putative kinase 1 |
| CAAAACTGTT | 185 | CAAAACTGTTTGTTGGC | 440 | myosin X |
| GAGAGGACAT | 186 | GAGAGGACATTGGAGGG | 441 | Homo sapiens cDNA FLJ32424 fis, clone SKMUS2000954, moderately similar to Homo sapiens F-box protein Fbx25 (FBX25) 97 |
| GAGTTAGGCA | 187 | GAGTTAGGCACTTCCTG | 442 | golgi phosphoprotein 1 |
| CCGTAGTGCC | 188 | CCGTAGTGCCTTTATGG | 443 | splicing factor, arginine/serine-rich 6 |
| CATAAACGGG | 189 | CATAAACGGGCACACCC | 444 | laminin, gamma 3 |
| TCCCTGGCAG | 190 | TCCCTGGCAGAGGGCTT | 445 | cysteine-rich proteins 2 |
| GAGGCCATCC | 191 | GAGGCCATCCCCAACCC | 446 | U6 snRNA-associated Sm-like protein LSm7 |
| TTGCCTGGGA | 192 | TTGCCTGGGATGCTGGT | 447 | hypothetical protein FLJ10707 |
| CTGTCAGCGG | 193 | CTGTCAGCGGCTGCCCC | 448 | Homo sapiens, Similar to RIKEN cDNA 2310012N15 gene, clone IMAGE:3342825, mRNA, partial cds |
| AACGCGGCCA | 194 | AACGCGGCCAATGTGGG | 449 | macrophage migration inhibitory factor (glycosylation-inhibiting factor) |
| GGTTTGGCTT | 195 | GGTTTGGCTTAGGCTGG | 450 | ubiquinol-cytochrome c reductase hinge protein |
| GATTTTTGTG | 196 | ATTTTTGTGGTGTGGG | 451 | gap junction protein, alpha 1, 43kD (connexin 43) |
| GGCTGCCCTG | 197 | GGCTGCCCTGGGCAGCC | 452 | dihydropyrimidinase-like 3 |
| ATGGCAACAG | 198 | ATGGCAACAGAAACCAA | 453 | aquaporin 1 (channel-forming integral protein, 28kD) |
| CGCTGTGGGG | 199 | CGCTGTGGGGTGCAGAC | 454 | protein expressed in thyroid |
| GGCAGCCAGA | 200 | GGCAGCCAGAGCTCCAA | 455 | macrophage myristoylated alanine-rich C kinase substrate |
| AGAGCAAACC | 201 | AGAGCAAACCGTAGTCC | 456 | procollagen-lysine, 2-oxoglutarate 5-dioxygenase (lysine hydroxylase, Ehlers-Danlos syndrome type VI) |
| TTTCCCTCAA | 202 | TTTCCCTCAAAGACTCT | 457 | protease, serine, 11 (IGF binding) |
| TCCCCGTGGC | 203 | TCCCCGTGGCTGTGGGG | 458 | 24-dehydrocholesterol reductase |
| TTCTCCCAAA | 204 | TTCTCCCAAATACCGTT | 459 | collagen, type IV, alpha 2 |
| GGCTGGGGGC | 205 | GGCTGGGGGCCAGGGC | 460 | profilin 1 |
| CCCTACCCTG | 206 | CCCTACCCTGTTACCTT | 461 | apolipoprotein D |
| TAGGACCCTG | 207 | TAGGACCCTGCAGGGGG | 462 | hyaluronoglucosaminidase 2 |
| GTTTTTGCTT | 208 | GTTTTTGCTTCAGCGGC | 463 | hypothetical protein FLJ22678 |
| CTTGATTCCC | 209 | CTTGATTCCCACGCTAC | 464 | quiescin Q6 |
| GCTTGGCTCC | 210 | GCTTGGCTCCCAAAGGG | 465 | ras homolog gene family, member A |
| GGTGGCACTC | 211 | GGTGGCACTCAGTCTCT | 466 | ras homolog gene family, member A |
| ACCTGTGACC | 212 | ACCTGTGACCAGCACTG | 467 | plasminogen activator, urokinase |
| ACTGAGGAAA | 213 | ACTGAGGAAAGGAGCTC | 468 | insulin-like growth factor binding protein 3 |
| TGCAGCGCCT | 214 | TGCAGCGCCTGCGGCCT | 469 | uridine phosphorylase |
| CTGGGGGGAA | 215 | CTGGGGGGAAGGGACTG | 470 | KIAA0638 protein |
| GTGCTATTCT | 216 | GTGCTATTCTGGGGCTG | 471 | B7 homolog 3 |
| GGAGGGGGCT | 217 | GGAGGGGGCTTGAAGCC | 472 | lamin A/C |
| GTGCCTGAGA | 218 | GTGCCTGAGAGGCAGGC | 473 | lamin A/C |
| TCACAGGGTC | 219 | TCACAGGGTCCCCGGGG | 474 | lamin A/C |
| GGGCTCCCTG | 220 | GGGCTCCCTGGCCCTGG | 475 | regulator of G-protein signalling 12 |
| GCCCCAGGTA | 221 | GCCCCAGGTAGGGGGAC | 476 | proteasome (prosome, macropain) 26S subunit, non-ATPase, 8 |
| GAAAGTGGCT | 222 | GAAAGTGGCTGTCCTGG | 477 | Homo sapiens, Similar to RIKEN cDNA 5730528L 13 gene, clone MGC:17337 IMAGE:4213591, mRNA, complete cds |
| TCCCTGGCTG | 223 | TCCCTGGCTGTTGAGGC | 478 | prosaposin (variant Gaucher disease and variant metachromatic |
| ACAGAGCACA | 224 | ACAGAGCACAGCTGCCC | 479 | laminin, alpha 4 |
| CTTTGCACTC | 225 | CTTTGCACTCTCCTTTG | 480 | transcription elongation factor A (SII), 1 |
| ATGCTCCCTG | 226 | ATGCTCCCTGAGGAGCT | 481 | lectin, galactoside-binding, soluble, 3 binding protein |
| CCGTCCAAGG | 227 | CCGTCCAAGGGTCCGCT | 482 | ribosomal protein S16 |
| GGGCCCCCTG | 228 | GGGCCCCCTGGGCAGTG | 483 | glycophorin C (Gerbich blood group) |
| CTTATGCTGC | 229 | CTTATGCTGCTGGTGCC | 484 | endothelin receptor type B |
| GGTTATTTTG | 230 | GGTTATTTTGGAGTGTA | 485 | serine (or cysteine) proteinase inhibitor, clade E (nexin, plasminogen activator inhibitor type 1), member 1 |
| GCCTGTCCCT | 231 | GCCTGTCCCTCCAAGAC | 486 | Biglycan |
| AAGATGAGGG | 232 | AAGATGAGGGGGCAGGC | 487 | small nuclear ribonucleoprotein polypeptide B" |
| CCAACAAGAA | 233 | CCAACAAGAATGCATTG | 488 | transmembrane 4 superfamily member 2 |
| AAGGATGCGG | 234 | AAGGATGCGGTGATGGC | 489 | TAF11 RNA polymerase II, TATA box binding protein (TBP)-associated factor, 28 kD |
| TGTCATCACA | 235 | TGTCATCACAGACACTT | 490 | lysyl oxidase-like 2 |
| CAGGCTTTTT | 236 | CAGGCTTTTTGGCTTCC | 491 | SRY (sex determining region Y)-box 4 |
| TCAAGTTCAC | 237 | TCAAGTTCACTGCCTGT | 492 | SOX4 SPY (sex determining region Y)-box 4 |
| TCCCTGGGCA | 238 | TCCCTGGGCAGCTTCAG | 493 | SRY (sex determining region Y)-box 4 |
| CAGGAGTTCA | 239 | CAGGAGTTCAAAGAAGG | 494 | actin related protein 2/3 complex, subunit 2 (34 kD) |
| CAGGTGGTTC | 240 | CAGGTGGTTCTGCCATC | 495 | Homo sapiens cDNA: FLJ23507 fis, clone LNG03128 |
| GCCCACATCC | 241 | GCCCACATCCGCTGAGG | 496 | hypothetical protein FLJ12442 |
| GCTGGGGTGG | 242 | GCTGGGGTGGGGGTGG | 497 | Fas (TNFRSF6)-associated via death domain |
| GACCTCCTGC | 243 | GACCTCCTGCCCTGGGG | 498 | mitogen-activated protein kinase kinase kinase 11 |
| AGTGAATAAA | 244 | AGTGAATAAATGTCTTG | 499 | TEK tyrosine kinase, endothelial (venous malformations, multiple cutaneous and mucosal) |
| AAGGTTCTTC | 245 | AAGGTTCTTCTCAAGGG | 500 | insulin receptor |
| AGCCTGGACT | 246 | AGCCTGGACTGAGCCAC | 501 | cell membrane glycoprotein, 110000M(r) (surface antigen) |
| CAACCCAGAT | 247 | CAACCCAGATTGGGGTG | 502 | Homo sapiens cDNA FLJ11863 fis, clone HEMBA1006926 |
| TGCTTCTGCC | 248 | TGCTTCTGCCACCCTGC | 503 | jagged 1 (Alagille syndrome) |
| CAGGTGACAA | 249 | CAGGTGACAAGGGCCCT | 504 | KIAA0304 gene product |
| GGCCGGGGGC | 250 | GGCCGGGGGCAGTTCTC | 505 | pre-B-cell leukemia transcription factor 2 |
| GTGCGCTAGG | 251 | GTGCGCTAGGGCCCCGG | 506 | Homo sapiens cDNA FLJ31238 fis, clone KIDNE2004864 |
| AGGCTGTCCA | 252 | AGGCTGTCCAGGCTCTG | 507 | p53-induced protein |
| TGTTATGTCC | 253 | TGTTATGTCCATTTTGC | 508 | complement component 1, q subcomponent, receptor 1 |
| TTTCCCAAAC | 254 | TTTCCCAAACTGTGAGG | 509 | complement component 1, q subcomponent, receptor 1 |
| GGGGATGGGG | 255 | GGGGATGGGGTACTGCC | 510 | Homo sapiens, clone IMAGE:3908182, mRNA, partial cds |

**Table 2.**

| **SEQ ID NO:** | **Unigene ID** | **OMIMID** | **gene symbol** | **locuslink id** | **Cellular Component** |
|---|---|---|---|---|---|
| 33 | Hs.102135 | 300090 | SS | 6748 | endoplasmic reticulum, membrane |
| 34 | Hs.103180 | | | | |
| 35 | Hs.105850 | | KIAA0404 | 23130 | |
| 36 | Hs.107019 | 602388 | SPK | 8189 | cytoplasm, nucleoplasm |
| 37 | Hs.107125 | | | | membrane |
| 38 | Hs.107809 | | KIAA0726 | 9746 | membrane |
| 39 | Hs.109276 | | | | |
| 40 | Hs.1103 | 190180 | TGFB1 | 7040 | |
| 41 | Hs.110443 | | | | |
| 42 | Hs.110950 | | | | |
| 43 | Hs.110964 | | | | |
| 44 | Hs.111039 | 160993 | NMT1 | 4836 | |
| 45 | Hs.11114 | | DJ1181N3 | 58476 | |
| 46 | Hs.111611 | | RPL27 | 6155 | intracellular, ribosome |
| 47 | Hs.111779 | 182120 | SPARC | 6678 | basement membrane |
| 48 | Hs.111779 | 182120 | SPARC | 6678 | basement membrane |
| 49 | Hs.111779 | 182120 | SPARC | 6678 | basement membrane |
| 50 | Hs.111779 | 182120 | SPARC | 6678 | basement membrane |
| 51 | Hs.111988 | | | | |
| 52 | Hs.112238 | | | | |
| 53 | Hs.112844 | | | | |
| 54 | Hs.11669 | 601033 | LAMA5 | 3911 | basement lamina |
| 55 | Hs.118126 | 256540 | PPGB | 5476 | endoplasmic reticulum, lysosome |
| 56 | Hs.118893 | 600134 | D2S448 | 7837 | cellular_component unknown |
| 57 | Hs.118893 | 600134 | D2S44 | 7837 | cellular_component unknown |
| 58 | Hs.119120 | 605568 | SMURF1 | 57154 | intracellular |
| 59 | Hs.119129 | 120130 | COL4A1 | 1282 | collagen |
| 60 | Hs.119129 | 120130 | COL4A1 | 1282 | collagen |
| 61 | Hs.119129 | 120130 | COL4A1 | 1282 | collagen |
| 62 | Hs.119206 | 602867 | IGFBP7 | 3490 | extracellular |
| 63 | Hs.124 | | | | |
| 64 | Hs.125359 | 188230 | THY1 | 7070 | integral plasma membrane protein |
| 65 | Hs.127824 | | | | |
| 66 | Hs.13011 | | GTPBP2 | 54676 | |
| 67 | Hs.13350 | | | | |
| 68 | Hs.136164 | | | | |
| 69 | Hs.143897 | 603009 | DYSF | 8291 | plasma membrane |
| 70 | Hs.149098 | 602127 | SMTN | 6525 | actin cytoskeleton |
| 71 | Hs.149609 | 135620 | ITGA5 | 3678 | cytoskeleton, extracellular matrix, |
| 72 | Hs.150580 | | SUI1 | 10209 | cellular_component unknown |
| 73 | Hs.15165 | | | | |
| 74 | Hs.151738 | 120361 | MMP9 | 4318 | extracellular matrix, extracellular |
| 75 | Hs.155048 | 111200 | LU | 4059 | integral plasma membrane protein, |
| 76 | Hs.155223 | 603665 | STC2 | 8614 | |
| 77 | Hs.155396 | 600492 | NFE2L2 | 4780 | nucleus |
| 78 | Hs.155894 | 176885 | PTPN1 | 5770 | cytoplasm, soluble fraction |
| 79 | Hs.158237 | 604042 | ITGA10 | 8515 | cytoskeleton, extracellular matrix, |
| 80 | Hs.159263 | 120240 | COL6A2 | 1292 | extracellular matrix |
| 81 | Hs.16007 | | | | |
| 82 | Hs.160958 | 605065 | CDC37 | 11140 | |
| 83 | Hs.16450 | | | | |
| 84 | Hs.172813 | 605477 | P85SPR | 8874 | |
| 85 | Hs.173724 | 123280 | CKB | 1152 | cytoplasm |
| 86 | Hs.173739 | | | | |
| 87 | Hs.177596 | | | | |
| 88 | Hs.17839 | | GG2 | 25816 | |
| 89 | Hs.180338 | 603366 | TNFRSF12 | 8718 | integral plasma membrane protein, |
| 90 | Hs.180370 | 601442 | CFL1 | 1072 | cytoskeleton, nucleus |
| 91 | Hs.180610 | 605199 | SFPQ | 6421 | nucleus |
| 92 | Hs.180610 | 605199 | SFPQ | 6421 | nucleus |
| 93 | Hs.18063 | 164720 | ETS1 | 2113 | nucleus |
| 94 | Hs.18069 | 602620 | PRSC1 | 5641 | |
| 95 | Hs.180842 | 113703 | RPL13 | 6137 | cytosolic ribosome, intracellular |
| 96 | Hs.182626 | | | | |
| 97 | Hs.184669 | 600346 | ZNF144 | 7703 | nucleus |
| 98 | Hs.185973 | | DEGS | 8560 | endoplasmic reticulum, integral |
| 99 | Hs.193053 | 606229 | EIF2C2 | 27161 | cellular_component unknown |
| 100 | Hs.19347 | | MRPL45 | 84311 | mitochondrion |
| 101 | Hs.19555 | | | | |
| 102 | Hs.19561 | 602139 | NDUFA7 | 4701 | membrane fraction mitochondrion, |
| 103 | Hs.195727 | 606064 | TEM1 | 57124 | extracellular matrix |
| 104 | Hs.197298 | | NS1 | 10625 | spliceosome, transcription factor |
| 105 | Hs.2017 | 604182 | RPL38 | 6169 | 60S ribosomal subunit, intracellular, |
| 106 | Hs.20225 | | | | |
| 107 | Hs.205736 | 605660 | HKE2 | 10471 | prefoldin |
| 108 | Hs.20716 | 605057 | TIM17 | 10440 | integral plasma membrane protein, |
| 109 | Hs.209061 | 603579 | SUDD | 8780 | |
| 110 | Hs.211573 | 14246 | HSPG2 | 3339 | basement membrane, extracellular |
| 111 | Hs.211612 | | SEC24A | 10802 | COPII vesicle coat, endoplasmic |
| 112 | Hs.211914 | 601825 | NDUFS7 | 4727 | mitochondrion, NADH dehydrogenase |
| 113 | Hs.21595 | 312095 | DXYS155E | 8227 | cellular_component unknown |
| 114 | Hs.217493 | 151740 | ANXA2 | 302 | plasma membrane, soluble fraction |
| 115 | Hs.21906 | | | | |
| 116 | Hs.22129 | | | | |
| 117 | Hs.2258 | 185260 | MMP10 | 4319 | extracellular matrix, extracellular |
| 118 | Hs.227835 | | | | |
| 119 | Hs.23016 | | RDC1 | 57007 | integral membrane protein, membrane |
| 120 | Hs.233955 | | | | |
| 121 | Hs.2399 | 600754 | MMP14 | 4323 | extracellular matrix, integral plasma |
| 122 | Hs.25132 | | | | |
| 123 | Hs.25450 | 602193 | SLC29A1 | 2030 | integral plasma membrane protein, |
| 124 | Hs.25590 | 601185 | STC1 | 6781 | |
| 125 | Hs.25590 | 601185 | STC1 | 6781 | |
| 126 | Hs.25590 | 601185 | STC1 | 6781 | |
| 127 | Hs.25664 | | DOC | 10263 | |
| 128 | Hs.25664 | | DOC | 10263 | |
| 129 | Hs.268571 | 107710 | APOC1 | 341 | |
| 130 | Hs.2706 | 138322 | GPX4 | 2879 | mitochondrion |
| 131 | Hs.272106 | | | | |
| 132 | Hs.274184 | 314310 | TFE3 | 7030 | nucleus |
| 133 | Hs.274453 | | | | |
| 134 | Hs.27836 | | | | |
| 135 | Hs.278573 | 107271 | CD59 | 966 | membrane fraction, plasma membrane |
| 136 | Hs.278941 | | | | |
| 137 | Hs.279869 | 604853 | MAAT1 | 10573 | |
| 138 | Hs.283636 | | | | |
| 139 | Hs.284284 | | | | |
| 140 | Hs.285814 | | | | |
| 141 | Hs.285814 | | | | |
| 142 | Hs.287830 | | | | |
| 143 | Hs.289092 | | CLP | 23406 | intracellular |
| 144 | Hs.29288 | | | | |
| 145 | Hs.296234 | | | | |
| 146 | Hs.296406 | | | | |
| 147 | Hs.29716 | | | | |
| 148 | Hs.29797 | 312173 | RPL10 | 6134 | 60S ribosomal subunit, intracellular, |
| 149 | Hs.298262 | 603474 | RPS19 | 6223 | 40S ribosomal subunit, intracellular, ribosome |
| 150 | Hs.299257 | | | | |
| 151 | Hs.300954 | | | | |
| 152 | Hs.302741 | | | | |
| 153 | Hs.311780 | | | | |
| 154 | Hs.312191 | | | | |
| 155 | Hs.326445 | 164731 | AKT2 | 208 | |
| 156 | Hs.327884 | | | | |
| 157 | Hs.332173 | 601041 | TLE2 | 7089 | nucleus |
| 158 | Hs.334604 | | KIAA1870 | 85301 | collagen |
| 159 | Hs.334895 | | RPL10A | 4736 | 60S ribosomal subunit, intracellular, |
| 160 | Hs.342389 | 123840 | PPIA | 5478 | cytoplasm |
| 161 | Hs.344224 | | | | |
| 162 | Hs.34516 | | | | |
| 163 | Hs.347297 | | | | |
| 164 | Hs.348428 | | | | |
| 165 | Hs.348967 | | | | |
| 166 | Hs.350065 | | | | |
| 167 | Hs.351706 | | | | |
| 168 | Hs.36353 | | | | |
| 169 | Hs.39619 | | LOC57333 | 57333 | |
| 170 | Hs.39871 | 606539 | MYO1D | 4642 | myosin |
| 171 | Hs.3989 | 604293 | PLXNB2 | 23654 | membrane |
| 172 | Hs.4082 | 606099 | LGALS8 | 3964 | extracellular space |
| 173 | Hs.48320 | | DORFIN | 25897 | centrosome |
| 174 | Hs.48604 | | | | |
| 175 | Hs.4980 | 603450 | LDB2 | 9079 | nucleus |
| 176 | Hs.5215 | 602912 | ITGB4BP | 3692 | extrinsic membrane protein, |
| 177 | Hs.5307 | | | | |
| 178 | Hs.54828 | | | | |
| 179 | Hs.56205 | 602055 | INSIG1 | 3638 | |
| 180 | Hs.57735 | | SREC | 8578 | membrane |
| 181 | Hs.59544 | 126380 | ERCC1 | 2067 | nucleus |
| 182 | Hs.61478 | | | | |
| 183 | Hs.61490 | | | | |
| 184 | Hs.6163 | | PINK1 | 65018 | |
| 185 | Hs.61638 | | | | |
| 186 | Hs.61661 | | | | |
| 187 | Hs.6831 | | | | |
| 188 | Hs.6891 | 601944 | SFRS6 | 6431 | nucleus |
| 189 | Hs.69954 | 604349 | LAMC3 | 10319 | extracellular matrix, membrane |
| 190 | HS.70327 | 601183 | CRIP2 | 1397 | |
| 191 | Hs.70830 | | LOC51690 | 51690 | nucleus, small nucleolar |
| 192 | Hs.7187 | | | | |
| 193 | Hs.7247 | | | | |
| 194 | Hs.73798 | 153620 | MIF | 4282 | extracellular space |
| 195 | Hs.73818 | | UQCRH | 7388 | mitochondrial electron transport chain |
| 196 | Hs.74471 | 121014 | GJA1 | 2697 | connexon, integral plasma membrane |
| 197 | Hs.74566 | 601168 | DPYSL3 | 1809 | |
| 198 | Hs.74602 | 107776 | AQP1 | 358 | integral plasma membrane protein, |
| 199 | Hs.7486 | | | | |
| 200 | Hs.75061 | | MLP | 65108 | |
| 201 | Hs.75093 | 153454 | PLOD | 5351 | endoplasmic reticulum |
| 202 | Hs.75111 | 602194 | PRSS11 | 5654 | extracellular space |
| 203 | Hs.75616 | | | | |
| 204 | Hs.75617 | 120090 | COL4A2 | 1284 | collagen, collagen type IV |
| 205 | Hs.75721 | 176610 | PFN1 | 5216 | actin cytoskeleton |
| 206 | Hs.75736 | 107740 | APOD | 347 | extracellular space |
| 207 | Hs.76873 | 603551 | HYAL2 | 8692 | lysosome |
| 208 | Hs.7718 | | | | |
| 209 | Hs.77266 | 603120 | QSCN6 | 5768 | |
| 210 | Hs.77273 | 165390 | ARHA | 387 | cytoskeleton |
| 211 | Hs.77273 | 165390 | ARHA | 387 | cytoskeleton |
| 212 | Hs.77274 | 191840 | PLAU | 5328 | extracellular space |
| 213 | Hs.77326 | 146732 | IGFBP3 | 3486 | extracellular space |
| 214 | Hs.77573 | 191730 | UP | 7378 | |
| 215 | Hs.77864 | | | | |
| 216 | Hs.77873 | 605715 | B7 | 80381 | cellular_component unknown |
| 217 | Hs.77886 | 150330 | LMNA | 4000 | lamin , nuclear lamina, nucleus |
| 218 | Hs.77886 | 150330 | LMNA | 4000 | lamin, nuclear lamina, nucleus |
| 219 | Hs.77886 | 150330 | LMNA | 4000 | lamin, nuclear lamina, nucleus |
| 220 | Hs.78281 | 602512 | RGS12 | 6002 | extrinsic plasma membrane protein, |
| 221 | Hs.78466 | | PSMD8 | 5714 | 19S proteasome regulatory particle |
| 222 | Hs.78531 | | | | |
| 223 | Hs.78575 | 176801 | PSAP | 5660 | extracellular space, integral membrane |
| 224 | Hs.78672 | 600133 | LAMA4 | 3910 | basement lamina |
| 225 | Hs.78869 | 601425 | TCEA1 | 6917 | nucleus |
| 226 | Hs.79339 | 600626 | LGALS3BP | 3959 | extracellular space, membrane |
| 227 | Hs.80617 | 603675 | RPS16 | 6217 | 40S ribosomal subunit, intracellular, |
| 228 | Hs.81994 | 110750 | GYPC | 2995 | integral plasma membrane protein, |
| 229 | Hs.82002 | 131244 | EDNRB | 1910 | integral plasma membrane protein, |
| 230 | Hs.82085 | 173360 | SERPINE1 | 5054 | |
| 231 | Hs.821 | 301870 | BGN | 633 | extracellular matrix |
| 232 | Hs.82575 | 603520 | SNRPB2 | 6629 | nucleus, snRNP U2e |
| 233 | Hs.82749 | 300096 | TM4SF2 | 7102 | integral plasma membrane protein, |
| 234 | Hs.83126 | 600772 | TAF2I | 6882 | nucleus, TFIID complex |
| 235 | Hs.83354 | | LOXL2 | 4017 | extracellular space, membrane |
| 236 | Hs.83484 | 184430 | SOX4 | 6659 | nucleus |
| 237 | Hs.83484 | | | | |
| 238 | Hs.83484 | 184430 | SOX4 | 6659 | nucleus |
| 239 | Hs.83583 | 604224 | ARPC2 | 10109 | actin cytoskeleton, Arp2/3 protein |
| 240 | Hs.84063 | | | | |
| 241 | Hs.84753 | | | | |
| 242 | Hs.86131 | 602457 | FADD | 8772 | cytoplasm |
| 243 | Hs.89449 | 600050 | MAP3K11 | 4296 | |
| 244 | Hs.89640 | 600221 | TEK | 7010 | integral plasma membrane protein, |
| 245 | Hs.89695 | 147670 | INSR | 3643 | integral plasma membrane protein, |
| 246 | Hs.90107 | | GP110 | 11047 | integral plasma membrane protein, |
| 247 | Hs.9096 | | | | |
| 248 | Hs.91143 | 601920 | JAG1 | 182 | membrane |
| 249 | Hs.92236 | | KIAA0304 | 9757 | nucleus |
| 250 | Hs.93728 | 176311 | PBX2 | 5089 | nucleus |
| 251 | Hs.9408 | | | | |
| 252 | Hs.96908 | | PIG11 | 9537 | |
| 253 | Hs.97199 | 120577 | C1QR | 22918 | integral plasma membrane protein, |
| 254 | Hs.97199 | 120577 | C1QR | 22918 | integral plasma membrane protein, |
| 255 | Hs.99093 | | | | |

Isolated and purified nucleic acids, according to the present invention are those which are not linked to those genes to which they are linked in the human genome. Moreover, they are not present in a mixture such as a library containing a multitude of distinct sequences from distinct genes. They may be, however, linked to other genes such as vector sequences or sequences of other genes to which they are not naturally adjacent. Tags disclosed herein, because of the way that they were made, represent sequences which are 3' of the 3' most restriction enzyme recognition site for the tagging enzyme used to generate the SAGE tags. In this case, the tags are 3' of the most 3' most NlaIII site in the cDNA molecules corresponding to mRNA. Nucleic acids corresponding to tags may be RNA, cDNA, or genomic DNA, for example. Such corresponding nucleic acids can be determined by comparison to sequence databases to determine sequence identities. Sequence comparisons can be done using any available technique, such as BLAST, available from the National Library of Medicine, National Center for Biotechnology Information. Tags can also be used as hybridization probes to libraries of genomic or cDNA to identify the genes from which they derive. Thus, using sequence comparisons or cloning, or combinations of these methods, one skilled in the art can obtain full-length nucleic acid sequences. Genes corresponding to tags will contain the sequence of the tag at the 3' end of the coding sequence or of the 3' untranslated region (UTR), 3' of the 3' most recognition site in the cDNA for the restriction endonuclease which was used to make the tags. The nucleic acids may represent either the sense or the anti-sense strand. Nucleic acids and proteins although disclosed herein with sequence particularity, may be derived from a single individual. Allelic variants which occur in the population of humans are included within the scope of such nucleic acids and proteins. Those of skill in the art are well able to identify allelic variants as being the same gene or protein. Griven a nucleic acid, one of ordinary skill in the art can readily determine an open reading frame present, and consequently the sequence of a polypeptide encoded by the open reading frame and, using techniques well known in the art, express such protein in a suitable host. Proteins comprising such polypeptides can be the naturally occurring proteins, fusion proteins comprising exogenous sequences from other genes from humans or other species, epitope tagged polypeptides, etc. Isolated and purified proteins are not in a cell, and are separated from the normal cellular constituents, such as nucleic acids, lipids, etc. Typically the protein is purified to such an extent that it comprises the predominant species of protein in the composition, such as greater than 50, 60 70, 80, 90, or even 95% of the proteins present.

Using the proteins according to the invention, one of ordinary skill in the art can readily generate antibodies which specifically bind to the proteins. Such antibodies can be monoclonal or polyclonal. They can be chimeric, humanized, or totally human. Any functional fragment or derivative of an antibody can be used including Fab, Fab', Fab2, Fab'2, and single chain variable regions. So long as the fragment or derivative retains specificity of binding for the endothelial marker protein it can be used. Antibodies can be tested for specificity of binding by comparing binding to appropriate antigen to binding to irrelevant antigen or antigen mixture under a given set of conditions. If the antibody binds to the appropriate antigen at least 2, 5, 7, and preferably 10 times more than to irrelevant antigen or antigen mixture then it is considered to be specific.

Techniques for making such partially to fully human antibodies are known in the art and any such techniques can be used. According to one particularly preferred embodiment, fully human antibody sequences are made in a transgenic mouse which has been engineered to express human heavy and light chain antibody genes. Multiple strains of such transgenic mice have been made which can produce different classes of antibodies. B cells from transgenic mice which are producing a desirable antibody can be fused to make hybridoma cell lines for continuous production of the desired antibody. See for example, Nina D. Russel, Jose R. F. Corvalan, Michael L. Gallo, C. Geoffrey Davis, Liise-Anne Pirofski. Production of Protective Human Antipneumococcal Antibodies by Transgenic Mice with Human Immunoglobulin Loci Infection and Immunity April 2000, p. 1820-1826; Michael L. Gallo, Vladimir E. Ivanov, Aya Jakobovits, and C. Geoffrey Davis. The human immunoglobulin loci introduced into mice: V (D) and J gene segment usage similar to that of adult humans European Journal of Immunology 30: 534-540, 2000; Larry L. Green. Antibody engineering via genetic engineering of the mouse: XenoMouse strains are a vehicle for the facile generation of therapeutic human monoclonal antibodies Journal of Immunological Methods 231 11-23, 1999; Yang X-D, Corvalan JRF, Wang P, Roy CM-N and Davis CG. Fully Human Anti-interleukin-8 Monoclonal Antibodies: Potential Therapeutics for the Treatment of Inflammatory Disease States. Journal of Leukocyte Biology Vol. 66, pp401-410 (1999); Yang X-D, Jia X-C, Corvalan JRF, Wang P, CG Davis and Jakobovits A. Eradication of Established Tumors by a Fully Human Monoclonal Antibody to the Epidermal Growth Factor Receptor without Concomitant Chemotherapy. Cancer Research Vol. 59, Number 6, pp1236-1243 (1999) ; Jakobovits A. Production and selection of antigen-specific fully human monoclonal antibodies from mice engineered with human Ig loci. Advanced Drug Delivery Reviews Vol. 31, pp: 33-42 (1998); Green L and Jakobovits A. Regulation of B cell development by variable gene complexity in mice reconstituted with human immunoglobulin yeast artificial chromosomes. J. Exp. Med. Vol. 188, Number 3, pp: 483-495 (1998); Jakobovits A. The long-awaited magic bullets: therapeutic human monoclonal antibodies from transgenic mice. Exp. Opin. Invest. Drugs Vol. 7(4), pp : 607-614 (1998) ; Tsuda H, Maynard-Currie K, Reid L, Yoshida T, Edamura K, Maeda N, Smithies O, Jakobovits A. Inactivation of Mouse HPRT locus by a 203-bp retrotransposon insertion and a 55-kb gene-targeted deletion: establishment of new HPRT-Deficient mouse embryonic sGEM cell lines. Genomics Vol. 42, pp: 413-421 (1997) ; Sherman-Gold, R. Monoclonal Antibodies: The Evolution from '80s Magic Bullets To Mature, Mainstream Applications as Clinical Therapeutics. Genetic Engineering News Vol. 17, Number 14 (August 1997); Mendez M, Green L, Corvalan J, Jia X-C, Maynard-Currie C, Yang X-d, Gallo M, Louie D, Lee D, Erickson K, Luna J, Roy C, Abderrahim H, Kirschenbaum F, Noguchi M, Smith D, Fukushima A, Hales J, Finer M, Davis C, Zsebo K, Jakobovits A. Functional transplant of megabase human immunoglobulin loci recapitulates human antibody response in mice. Nature Genetics Vol. 15, pp: 146-156 (1997); Jakobovits A. Mice engineered with human immunoglobulin YACs: A new technology for production of fully human antibodies for autoimmunity therapy. Weir's Handbook of Experimental Immunology, The Integrated Immune System Vol. IV, pp: 194.1-194.7 (1996) ; Jakobovits A. Production of fully human antibodies by transgenic mice. Current Opinion in Biotechnology Vol. 6, No. 5, pp: 561-566 (1995) ; Mendez M, Abderrahim H, Noguchi M, David N, Hardy M, Green L, Tsuda H, Yoast S, Maynard-Currie C, Garza D, Gemmill R, Jakobovits A, Klapholz S. Analysis of the structural integrity of YACs comprising human immunoglobulin genes in yeast and in embryonic sGEM cells. Genomics Vol. 26, pp: 294-307 (1995); Jakobovits A. YAC Vectors: Humanizing the mouse genome. Current Biology Vol. 4, No. 8, pp: 761-763 (1994); Arbones M, Ord D, Ley K, Ratech H, Maynard-Curry K, Otten G, Capon D, Tedder T. Lymphocyte homing and leukocyte rolling and migration are impaired in L-selectin-deficient mice. Immunity Vol. 1, No. 4, pp: 247-260 (1994); Green L, Hardy M, Maynard-Curry K, Tsuda H, Louie D, Mendez M, Abderrahim H, Noguchi M, Smith D, Zeng Y, et. al. Antigen-specific human monoclonal antibodies from mice engineered with human Ig heavy and light chain YACs. Nature Genetics Vol. 7, No. 1, pp: 13-21 (1994); Jakobovits A, Moore A, Green L, Vergara G, Maynard-Curry K, Austin H, Klapholz S. Germ-line transmission and expression of a human-derived yeast artificial chromosome. Nature Vol. 362, No. 6417, pp: 255-258 (1993) ; Jakobovits A, Vergara G, Kennedy J, Hales J, McGuinness R, Casentini-Borocz D, Brenner D, Otten G. Analysis of homozygous mutant chimeric mice: deletion of the immunoglobulin heavy-chain joining region blocks B-cell development and antibody production. Proceedings of the National Academy of Sciences USA Vol. 90, No. 6, pp: 2551-2555 (1993); Kucherlapati et al., U.S. 6,1075,181.

Antibodies can also be made using phage display techniques. Such techniques can be used to isolate an initial antibody or to generate variants with altered specificity or avidity characteristics. Single chain Fv can also be used as is convenient. They can be made from vaccinated transgenic mice, if desired. Antibodies can be produced in cell culture, in phage, or in various animals, including but not limited to cows, rabbits, goats, mice, rats, hamsters, guinea pigs, sheep, dogs, cats, monkeys, chimpanzees, apes.

Antibodies can be labeled with a detectable moiety such as a radioactive atom, a chromophore, a fluorophore, or the like. Such labeled antibodies can be used for diagnostic techniques, either *in vivo,* or in an isolated test sample. Antibodies can also be conjugated, for example, to a pharmaceutical agent, such as chemotherapeutic drug or a toxin. They can be linked to a cytokine, to a ligand, to another antibody. Suitable agents for coupling to antibodies to achieve an anti-tumor effect include cytokines, such as interleukin 2 (IL-2) and Tumor Necrosis Factor (TNF); photosensitizers, for use in photodynamic therapy, including aluminum (III) phthalocyanine tetrasulfonate, hematoporphyrin, and phthalocyanine; radionuclides, such as iodine-131 (¹³¹I), yttrium-90 (⁹⁰Y), bismuth-212 (²¹²Bi), bismuth-213 (²¹³Bi), technetium-99m (^{99m}Tc), rhenium-186 (¹⁸⁶Re), and rhenium-188 (¹⁸⁸Re); antibiotics, such as doxorubicin, adriamycin, daunorubicin, methotrexate, daunomycin, neocarzinostatin, and carboplatin; bacterial, plant, and other toxins, such as diphtheria toxin, pseudomonas exotoxin A, staphylococcal enterotoxin A, abrin-A toxin, ricin A (deglycosylated ricin A and native ricin A), TGF-alpha toxin, cytotoxin from chinese cobra (naja naja atra), and gelonin (a plant toxin); ribosome inactivating proteins from plants, bacteria and fungi, such as restrictocin (a ribosome inactivating protein produced by *Aspergillus restrictus*), saporin (a ribosome inactivating protein from *Saponaria officinalis*), and RNase; tyrosine kinase inhibitors; ly207702 (a difluorinated purine nucleoside); liposomes containing antitumor agents (e.g., antisense oligonucleotides, plasmids which encode for toxins, methotrexate, etc.); and other antibodies or antibody fragments, such as F(ab).

Those of skill in the art will readily understand and be able to make such antibody derivatives, as they are well known in the art. The antibodies may be cytotoxic on their own, or they may be used to deliver cytotoxic agents to particular locations in the body. The antibodies can be administered to individuals in need thereof as a form of passive immunization.

Characterization of extracellular regions for the cell surface and secreted proteins from the protein sequence is based on the prediction of signal sequence, transmembrane domains and functional domains. Antibodies are preferably specifically immunoreactive with membrane associated proteins, particularly to extracellular domains of such proteins or to secreted proteins. Such targets are readily accessible to antibodies, which typically do not have access to the interior of cells or nuclei. However, in some applications, antibodies directed to intracellular proteins may be useful as well. Moreover, for diagnostic purposes, an intracellular protein may be an equally good target since cell lysates may be used rather than a whole cell assay.

Computer programs can be used to identify extracellular domains of proteins whose sequences are known. Such programs include SMART software (Schultz et al., Proc. Natl. Acad. Sci. USA 95: 5857-5864, 1998) and Pfam software (BaGEMan et al., Nucleic acids Res. 28: 263-266, 2000) as well as PSORTII. Typically such programs identify transmembrane domains; the extracellular domains are identified as immediately adjacent to the transmembrane domains. Prediction of extracellular regions and the signal cleavage sites are only approximate. It may have a margin of error + or - 5 residues. Signal sequence can be predicted using three different methods (Nielsen et al, Protein Engineering 10: 1-6 ,1997, Jagla et. al, Bioinformatics 16: 245-250 , 2000, Nakai, K and Horton, P. Trends in Biochem. Sci. 24:34-35, 1999) for greater accuracy. Similarly transmembrane (TM) domains can be identified by multiple prediction methods. (Pasquier, et. al, Protein Eng. 12:381-385, 1999, Sonnhammer et al., In Proc. of Sixth Int. Conf. on Intelligent Systems for Molecular Biology, p. 175-182 , Ed J. Glasgow, T. Littlejohn, F. Major, R. Lathrop, D. Sankoff, and C. Sensen Menlo Park, CA: AAAI Press, 1998 , Klein, et.al, Biochim. Biophys. Acta, 815:468, 1985, Nakai and Kanehisa Genomics, 14: 897-911 , 1992). In ambiguous cases, locations of functional domains in well characterized proteins are used as a guide to assign a cellular localization.

Putative functions or functional domains of novel proteins can be inferred from homologous regions in the database identified by BLAST searches (Altschul et. al. Nucleic Acid Res. 25: 3389-3402, 1997) and/or from a conserved domain database such as Pfam (BaGEMan et.al, Nucleic Acids Res. 27:260-262 1999) BLOCKS (Henikoff, et. al, Nucl. Acids Res. 28:228-230, 2000) and SMART (Ponting, et. al, Nucleic Acid Res. 27,229-232, 1999). Extracellular domains include regions adjacent to a transmembrane domain in a single transmembrane domain protein (out-in or type I class). For multiple transmembrane domains proteins, the extracellular domain also includes those regions between two adjacent transmembrane domains (in-out and out-in). For type II transmembrane domain proteins, for which the N-terminal region is cytoplasmic, regions following the transmembrane domain is generally extracellular. Secreted proteins on the other hand do not have a transmembrane domain and hence the whole protein is considered as extracellular.

Membrane associated proteins can be engineered to delete the transmembrane domains, thus leaving the extracellular portions which can bind to ligands. Such soluble forms of transmembrane receptor proteins can be used to compete with natural forms for binding to ligand. Thus such soluble forms act as inhibitors. and can be used therapeutically as anti-angiogenic agents, as diagnostic tools for the quantification of natural ligands, and in assays for the identification of small molecules which modulate or mimic the activity of a GEM:ligand complex.

Alternatively, the endothelial markers themselves can be used as vaccines to raise an immune response in the vaccinated animal or human. For such uses, a protein, or immunogenic fragment of such protein, corresponding to the intracellular, extracellular or secreted GEM of interest is administered to a subject. The immogenic agent may be provided as a purified preparation or in an appropriately expressing cell. The administration may be direct, by the delivery of the immunogenic agent to the subject, or indirect, through the delivery of a nucleic acid encoding the immunogenic agent under conditions resulting in the expression of the immunogenic agent of interest in the subject. The GEM of interest may be delivered in an expressing cell, such as a purified population of glioma endothelial cells or a populations of fused glioma endothelial and dendritic cells. Nucleic acids encoding the GEM of interest may be delivered in a viral or non-viral delivery vector or vehicle. Non-human sequences encoding the human GEM of interest or other mammalian homolog can be used to induce the desired immunologic response in a human subject. For several of the GEMs of the present invention, mouse, rat or other ortholog sequences are described herein or can be obtained from the literature or using techniques well within the skill of the art.

Endothelial cells can be identified using the markers which are disclosed herein as being endothelial cell specific. These include the human markers identified by SEQ ID NOS: 1-510. Antibodies specific for such markers can be used to identify such cells, by contacting the antibodies with a population of cells containing some endothelial cells. The presence of cross-reactive material with the antibodies identifies particular cells as endothelial. Similarly, lysates of cells can be tested for the presence of cross-reactive material. Any known format or technique for detecting cross-reactive material can be used including, immunoblots, radioimmunoassay, ELISA, immunoprecipitation, and immunohistochemistry. In addition, nucleic acid probes for these markers can also be used to identify endothelial cells. Any hybridization technique known in the art including Northern blotting, RT-PCR, microarray hybridization, and in situ hybridization can be used.

One can identify glioma endothelial cells for diagnostic purposes, testing cells suspected of containing one or more GEMs. One can test both tissues and bodily fluids of a subject. For example, one can test a patient's blood for evidence of intracellular and membrane associated GEMS, as well as for secreted GEMS. Intracellular and/or membrane associated GEMs may be present in bodily fluids as the result of high levels of expression of these factors and/or through lysis of cells expressing the GEMs.

Populations of various types of endothelial cells can also be made using the antibodies to endothelial markers of the invention. The antibodies can be used to purify cell populations according to any technique known in the art, including but not limited to fluorescence activated cell sorting. Such techniques permit the isolation of populations which are at least 50, 60, 70, 80, 90, 92, 94, 95, 96, 97, 98, and even 99 % the type of endothelial cell desired, whether normal, tumor, or pan-endothelial. Antibodies can be used to both positively select and negatively select such populations. Preferably at least 1, 5, 10, 15, 20, or 25 of the appropriate markers are expressed by the endothelial cell population.

Populations of endothelial cells made as described herein, can be used for screening drugs to identify those suitable for inhibiting the growth of tumors by virtue of inhibiting the growth of the tumor vasculature.

Populations of endothelial cells made as described herein, can be used for screening candidate drugs to identify those suitable for modulating angiogenesis, such as for inhibiting the growth of tumors by virtue of inhibiting the growth of endothelial cells, such as inhibiting the growth of the tumor or other undesired vasculature, or alternatively, to promote the growth of endothelial cells and thus stimulate the growth of new or additional large vessel or microvasculature.

Inhibiting the growth of endothelial cells means either regression of vasculature which is already present, or the slowing or the absence of the development of new vascularization in a treated system as compared with a control system. By stimulating the growth of endothelial cells, one can influence development of new (neovascularization) or additional vasculature development (revascularization). A variety of model screening systems are available in which to test the angiogenic and/or anti-angiogenic properties of a given candidate drug. Typical tests involve assays measuring the endothelial cell response, such as proliferation, migration, differentiation and/or intracellular interaction of a given candidate drug. By such tests, one can study the signals and effects of the test stimuli. Some common screens involve measurement of the inhibition of heparanase, endothelial tube formation on Matrigel, scratch induced motility of endothelial cells, platelet-derived growth factor driven proliferation of vascular smooth muscle cells, and the rat aortic ring assay (which provides an advantage of capillary formation rather than just one cell type).

Drugs can be screened for the ability to mimic or modulate, inhibit or stimulate, growth of tumor endothelium cells and/or normal endothelial cells. Drugs can be screened for the ability to inhibit tumor endothelium growth but not normal endothelium growth or survival. Similarly, human cell populations, such as normal endothelium populations or glioma endothelial cell populations, can be contacted with test substances and the expression of glioma endothelial markers and/or normal endothelial markers determined. Test substances which decrease the expression of glioma endothelial markers (GEMs) are candidates for inhibiting angiogenesis and the growth of tumors. In cases where the activity of a GEM is known, agents can be screened for their ability to decrease or increase the activity.

For those glioma endothelial markers identified as containing transmembrane regions, it is desirable to identify drug candidates capable of binding to the GEM receptors found at the cell surface. For some applications, the identification of drug candidates capable of blocking the GEM receptor from its native ligand will be desired. For some applications, the identification of a drug candidate capable of binding to the GEM receptor may be used as a means to deliver a therapeutic or diagnostic agent. For other applications, the identification of drug candidates capable of mimicking the activity of the native ligand will be desired. Thus, by manipulating the binding of a transmembrane GEM receptor:ligand complex, one may be able to promote or inhibit further development of endothelial cells and hence, vascularization.

For those glioma endothelial markers identified as being secreted proteins, it is desirable to identify drug candidates capable of binding to the secreted GEM protein. For some applications, the identification of drug candidates capable of interfering with the binding of the secreted GEM it is native receptor. For other applications, the identification of drug candidates capable of mimicking the activity of the native receptor will be desired. Thus, by manipulating the binding of the secreted GEM:receptor complex, one may be able to promote or inhibit futher development of endothelial cells, and hence, vascularization.

Expression can be monitored according to any convenient method. Protein or mRNA can be monitored. Any technique known in the art for monitoring specific genes' expression can be used, including but not limited to ELISAs, SAGE, microarray hybridization, Western blots. Changes in expression of a single marker may be used as a criterion for significant effect as a potential pro-angiogenic, anti-angiogenic or anti-tumor agent. However, it also may be desirable to screen for test substances which are able to modulate the expression of at least 5, 10, 15, or 20 of the relevant markers, such as the tumor or normal endothelial markers. Inhibition of GEM protein activity can also be used as a drug screen. Human and mouse GEMS can be used for this purpose.

Test substances for screening can come from any source. They can be libraries of natural products, combinatorial chemical libraries, biological products made by recombinant libraries, etc. The source of the test substances is not critical to the invention. The present invention provides means for screening compounds and compositions which may previously have been overlooked in other screening schemes. Nucleic acids and the corresponding encoded proteins of the markers of the present invention can be used therapeutically in a variety of modes. GEMs can be used to stimulate the growth of vasculature, such as for wound healing or to circumvent a blocked vessel. The nucleic acids and encoded proteins can be administered by any means known in the art. Such methods include, using liposomes, nanospheres, viral vectors, non-viral vectors comprising polycations, etc. Suitable viral vectors include adenovirus, retroviruses, and sindbis virus. Administration modes can be any known in the art, including parenteral, intravenous, intramuscular, intraperitoneal, topical, intranasal, intrarectal, intrabronchial, etc.

Specific biological antagonists of GEMs can also be used to therapeutic benefit. For example, antibodies, T cells specific for a GEM, antisense to a GEM, and ribozymes specific for a GEM can be used to restrict, inhibit, reduce, and/or diminish tumor or other abnormal or undesirable vasculature growth. Such antagonists can be administered as is known in the art for these classes of antagonists generally. Anti-angiogenic drugs and agents can be used to inhibit tumor growth, as well as to treat diabetic retinopathy, rheumatoid arthritis, psoriasis, polycystic kidney disease (PKD), and other diseases requiring angiogenesis for their pathologies.

Mouse counterparts to human GEMS can be used in mouse cancer models or in cell lines or *in vitro* to evaluate potential anti-angiogenic or anti-tumor compounds or therapies. Their expression can be monitored as an indication of effect. Mouse GEMs can be used as antigens for raising antibodies which can be tested in mouse tumor models. Mouse GEMs with transmembrane domains are particularly preferred for this purpose. Mouse GEMs can also be ued as vaccines to raise an immunological response in a human to the human ortholog.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLE 1

In this study we employ SAGE transcript profiling to derive the transcriptomes from normal and neoplastic brain tissue. Moreover, we have employed a new version of SAGE, long SAGE, allowing for the derivation of 21 bp SAGE tags. These longer tags allow for the direct interrogation of genomic DNA, identifying unique locations of cell-specific transcription. Endothelial cells from normal brain and different stages of gliomas were expression profiled and compared to each other and to the colon endothelial cell data. Distinct sets of genes define global tumor and normal endothelial cell markers as well as defining glioma-specific endothelial markers. This expanded tumor endothelial cell database will likely provide further insights into the complex regulatory mechanisms governing tumor angiogenesis.

### EXAMPLE 2

Tissue procurement and endothelial cell isolation. Five separate brain tissue samples (Table 1) were resected and immediately subjected to endothelial cell isolation with slight modifications to the protocol described previously. St Croix, B., Rago, C., Velculescu, V., Traverso, G., Romans, K. E., Montgomery, E., Lal, A., Riggins, G. J., Lengauer, C., Vogelstein, B., and Kinzler, K. W. (2000). Genes expressed in human tumor endothelium. Science 289, 1197-202.

Briefly, samples were surgically excised and submerged in DMEM. The samples were minced into 2 centimeter cubes and subjected to tissue digestion with a collagenase cocktail. Samples were mixed at 37°C until dissolved. Cells were spun down and washed two times with PBSBSA and filtered through successive nylon mesh filters of 250, 100 and 40 microns. Samples were resuspended in PBS/BSA and applied to a 30% Percoll gradient centrifuging for 15 minutes at 800g. 5 ml off the top of the percoll gradient was diluted in 50 ml DMEM and cells pelleted, washed with PBS and resuspended in 3 ml PBSBSA. Cells were filtered through falcon blue top filter tubes, spun down and resuspended in 1 ml PBS/BSA. 100 microliters of prewashed ant-CD45 magnetic beads (Dynal) were added and the solution allowed to gently mix for ten minutes. Bead-bound cells were discarded and the supernatant transferred to a fresh microcentrifuge tube. 10 microliters of P1H12 mAB (1:100) (Brain N1, T1, and T2 samples) or UEA-I lectin (Brain N2 and T3 samples) was added and the samples were mixed gently at 4°C for 45 minutes. Cells were pelleted and washed 3 times in PBSBSA and resuspended in 500 microliters PBS/BSA. Prewashed goat anti-mouse M450 dynabeads were added to each tube and allowed to mix for 15 minutes at 4°C. Bead-bound cells were washed 8 times with PBS/BSA and resuspended in a final volume of 500 microliters PBS. Cells were counted and frozen at-70°C prior to RNA extraction.

### EXAMPLE 3

RNA isolation and SAGE library generation. RNA was isolated from the selected cells and initially subjected to RT-PCR analysis to determine the relative abundance of specific, known endothelial cell markers. The microSAGE protocol St Croix, B., Rago, C., Velculescu, V., Traverso, G., Romans, K. E., Montgomery, E., Lal, A., Riggins, G. J., Lengauer, C., Vogelstein, B., and Kinzler, K. W. (2000). Genes expressed in human tumor endothelium. Science 289, 1197-202 ( server www, domain name sagenet.org, directory sage_protocol) was used to generate high-quality longSAGE libraries employing the tagging enzyme MmeI instead of BsmFI. 21 base tags were defined by capillary sequencing using a combination of an ABI 3700 and ABI 3100. The sample descriptions and sequencing depth are shown in Table 3.

### EXAMPLE 4

Data analysis. Long SAGE tags derived from the brain endothelial samples were reduced to short tags to allow for the integration of colon endothelial SAGE data. Aggregate short tags were derived from the long tags. Any short tag counts that had more than one corresponding long tag representative were summed and the counts represented as one short tag. Both sequencing errors and legitimate long tag derivatives contribute to the generation of multiple long tags. For transcript and genome mapping, differential long tags were employed. Differential gene expression was evaluated as follows: For the two normal brain samples, either the maximum or minimum value was used for determining tumor/normal and normal/tumor ratios, respectively. For the three brain tumor samples, the median value was used for the tumor/normal whereas the maximum value was used for the normal/tumor ratios. A two parameter family of beta distributions was used to assess the probability of observing two fold differences in the observed SAGE tag abundances. Chen, H., Centola, M., Altschul, S. F., and Metzger, H. (1998). Characterization of gene expression in resting and activated mast cells. J Exp Med 188, 1657-68.

### EXAMPLE 5

The following provides a detailed protocol useful for isolating brain endothelial cells. All steps were done at 4° C in cold room and in centrifuge except digestion.
1) Take sample from operating room and submerge in known volume of DMEM+ in 50 ml conical tube to measure tumor volume by displacement. Cut off 2 small pieces of tumor on dry ice and store at -70° C for mRNA extraction/immunohistochemistry/in situ analysis.
2) Take sample from conical and place in small amount of DMEM+ in 10 cm Tissue Culture dish in hood. Mince specimen into 2 mm cubes with sterile scalpel.
3) Transfer minced specimen to small autoclaved erlenmeyer flask and add 5X volume of digestion cocktail. Sample volumes > 5 ml should be split into multiple flasks.
4) Mix in bacterial shaker or in 37° C room on rotating shaker for 45 minutes or until sample is dissolved. Titrate with 10 ml piper every 15 minutes. Once a good cell suspension is obtained, remove and transfer to 50 ml conical.
Remainder of protocol done at 4°C.
5) Spin down at 1500 RPM (600xg) at 4° C for 5 minutes.
6) Wash 2X with PBS/BSA and spin down again. Pool samples.
7) Filter through Nylon Mesh (250, 100, 40 micron).
8) Spin down.
9) Resuspend n PBSBSA at ½ the original tumor volume.
10) Aply sample in 500 ul aliquots to preformed 30% Percoll gradient (Gradients needed = volume of original sample).
11) Spin at 1750 RPM (800g) for 15 minutes.
12) Remove top 5 ml Percoll from each tube and dilute with DMEM to 50 ml volume.
13) Pellet cells in centrifuge at 1500 RPM. Pool pelleted cells.
14) Wash 2X with PBSBSA and resuspend in 3 ml PBS/BSA.
15) Filter through Falcon Blue Top Filter tube.
16) Spin down and resuspend in 1 ml PBS/BSA in a 1.5 ml microcentrigufe tube.
17) Add 100 µl of prewashed anti-CD45 beads (hematopoietic depletion) to solution and rotate end over end in cold room for ten minutes. [For brain tissue isolation, an additional negative selection with BerEP4 epithelial depletion is not needed]
18) Remove bead-bound cells and transfer supernatant toa fresh microcentrifuge tube. Save bead-bound sample by freezing at -70° C. Repeat extraction to ensure complete removal of all beads.
19) Add 10 ul of P1H12 mAb (1:100) to cells and mix in cold room with end-over-end roataion for 45 minutes.
   [As an alternative, selection using UEA1 lectin also provides quality endothelial cell selection.] 20) Pellet cells and wash 3X with PBS/BSA.
21) Resuspend cells in 500 ul of PBS/BSA.
22) Divide sample into four 1.5 ml microcentrifuge tubes (125 ul per tube) and bring volume up to 800 ul. Add 50 ul of prewashed goat anti-mouse M450 dynabeads to each tube.
23) Rotate tubes in cold room for 15 minutes.
24) Separate with magnet and save supernatant as staining control, tumor/brain fraction.
25) Rinse 8X with PBS/BSA.
26) Pool beads into single microcentrifuge tube.
27) Resuspend final cells in 500 ul plain PBS.
28) Take 5 ul of solution and combine with 5 ul of Magic DAPI and count on hemacytometer.
29) Remove 10k cells for staining for quality control based on hemacytometer results
30) Separate beads again and freeze remainder at-70° C for mRNA extraction.

### EXAMPLE 6

This example describes the preparation of SAGE tags from mRNA extracted from brain endothelial cells. The preparation is described with reference to standard SAGE tag preparation procedures as are known in the art.
All of the template was used in the PCR SAGE ditag step. Usually we take only a small portion of our template, dilute it and perform ∼300 PCR
reactions. For these libraries we used all of our material, diluted it and
performed ∼ 1200 PCR reactions.
* During the post-amplified PCR product purification step we normally
do a standard large volume phenol/chloroform extraction and remove the
aqueous layer which contains the product of interest. For these libraries
we used Eppendorf's Phase Lock product which creates a physical barrier
between the aqueous and organic layers thereby decreasing the amount of
product you leave behind. This product was used for all P/C extractions in
the second half of the protocol.
* Digesting the amplified PCR products with NlaIII to release the
ditag of interest is usually done in one reaction. For these libraries I
divided the material into thirds and performed 3 NlaIII reactions in the
hopes of yielding more released ditag.
* Due to the low amount of material, upon entering the concatemer and
digested pZERO ligation reaction, I modified the recipe for this reaction to
accommodate this. Standard reaction calls for 6ul of concatemers, 2 ul of
5x ligase buffer, 1ul digested pZERO vector, and 1 ul of high concentrate
ligase. I modified it to 6ul of concatemers, 2ul of 5x ligase buffer,
0.3-0.5ul of digested pZERO vector, 1 ul high concentrate ligase and filled
the missing volume with water. My intention was to favor the concatemer to
pZERO ligation reaction relative to the competing pZERO to
pZERO ligation reaction.
Most gels during the procedure showed weak amounts of product for
visualization and the concatemer gels showed no visible product via the
naked eye (we cut out certain fractions regardless).

### EXAMPLE 7

Microarray Analysis. Custom 50 nucleotide oligomer arrays were constructed containing 606 unique gene elements. The 606 genes were derived from tumor and normal induced genes from both colon and brain data (328 genes), as well as 278 genes from both literature reviews and housekeeping genes. Arrays were interrogated with Cy3 and Cy5 dye-swapped labelled aRNA samples comparing HMVECs grown on plastic, collagen, fibrin, or Matrigel.

### EXAMPLE 8

In situ Hybridizations and Imunohistochemistry. In situ hybridizations for PV I, VEGFR2 and vWF were carried out as desribed previously (10). Co-staining of PV1 and CD31 was carried out as follows: Four 500 nucleotide riboprobe fragments specific for PV1 were transcribed and used to probe formalin fixed 5 micron tissue sections. Final detection of the bound riboprobes were delayed until after the CD31 IHC staining. After PV1 hybridization and washing, tissue sections were fixed for 20 minutes in 4% formaldehyde. After a brief rinse in TBS, antigen retrieval was carried out using DAKO target retrieval solution (DAKO, Cat#S 1699) according to manufacturer's instructions. After a five minute wash in TBS, slides were digested with Proteinase K at 20 ng/ml in TBS for 20 minutes at 37T, then blocked for 20 minutes at room temperature in block (10% Goat serum/0.5% Casein/0.05% Tween-20/PBS). Slides were incubated with DAKO CD31 (Cat#M0823) at a final concentration of 1 microgram/slide in block solution, for 60 minutes at room temperature. After two 5 minute TBST (DAKO, Cat#S3306) washes at room temperature, PV1 riboprobe and CD31 antibody were detected with Streptavidin-Cy2 (Jackson ImmunoResearch, Cat#016-220-084) at 5 micrograms/slide for the PV1 riboprobe, and goat anti-mouse-Cy3 (Jackson ImmunoResearch, Cat# 115-165 -146) at 2.5 micrograms/slide for CD3 1, for 60 minutes at room temperature. After three Ywashes in TBST, the slides were mounted with antifade medium containing DAPI nuclear counter-stain, cover-slipped and stored at -20'C until viewing. Single images of DAPI, Cy2 and Cy3 images were acquired separately on a Zeiss Axioplan at 40x with a Hammamatsu camera, then merged together to form a composite image using universal imaging metamorph software, and stored at -20C until viewing.

### EXAMPLE 9

Capillary-like tubule formation assay. The formation of capillary-like tubular structures was assessed in Matrigel-coated multiwell plates essentially as described previously (12). Briefly, 300 microliters of Matrigel (BD, Bedford, MA) was added to each well of a 24well plate and allowed to polymerize at 37 'C for 30 minutes. HMVECs (BioWhittaker) were infected with adenovius harboring Tem.1 or GFP gene or empty vector (EV) for 67 hours at 300 MOI (Multiplicity Of Infection). Cells were then seeded at a density of 30 x 103 cells/well in 500 microliters EGM-2 medium with supplements (BioWhittaker) in Matrigel-coated plates and incubated at 37 'C for 24 hours and viewed using a Nikon Eclipse TE200 microscope under a phase contrast and photographed. Images were analyzed using software Scion Image (Scion Corporation, Frederick, MD) under the mode of integrated density.

### EXAMPLE 9

Cell Proliferation Assay. HMVEC proliferation was assessed by the Cell Titer-Glo Luminescent Cell Viability Assay (Promega, Madison, WI) in 96-well cell culture plates. HMVECs were seeded at 2,000 cells per well in 100 microliters medium and plates were incubated at 37 'C for 48 hours. Reagent was added to each well according to manufacture's instruction, and fluorescence was measured using the Millipore CytoFluor2350.

### EXAMPLE 10

Five independent endothelial cell populations were purified from glioma tumor tissue and normal brain tissue. In this study, the tissue defined as normal is derived from patients with epilepsy who have undergone a temporal lobectomy. The samples are summarized in Table 3. Samples N1, T1 and T2 were ultimately P1H12-selected and samples N2 and T3 were UEA-I selected. Prior to SAGE analysis, each sample was assessed for the relative mRNA abundance for vWF, Glial fibrillary acidic protein (GFAP) and EF1 by RT-PCR. Abundant levels of vWF and the control housekeeper EF1, and low levels of the glial cell-specific gene GFAP suggested the cell population was primarily endothelial (data not shown). SAGE analysis was performed to a depth of approximately 50,000 tags (Table 3). For data analysis, each SAGE project was normalized to exactly 50,000 tags. Pairwise comparisons between expression data derived from tumor samples selected with P1H12 or UEA-I showed correlation coefficients around 80%, slightly higher than a comparison between two tumor samples both selected with P1H12. This suggests that selecting endothelial cells with either P1H12 or UEA-I results in highly similar cell populations. Moreover, nearly half of the tumor specific markers revealed in this study are induced 4 fold in each of the normal samples used, suggesting the normal samples are similar populations as well. With this in mind, we felt that combining data for the two normal samples and for the three tumor samples was appropriate.

**Table 3. Samples used in this study.**

| **Sample** | **Description** | **Tags Generated** | **EC Selection** |
|---|---|---|---|
| **Brain N1** | Normal temporal lobectomy ECs | 43,000 | P1H12 |
| **Brain N2** | Normal temporal lobectomy ECs | 49,000 | UEA-I |
| **Brain T1** | Grade IV Glioma Ecs | 46,000 | P1H12 |
| **Brain T2** | Grade III Glioma Ecs | 50,000 | P1H12 |
| **Brain T3** | Grade IV Glioma Ecs | 58,000 | UEA-I |
| | | | |
| **Colon N*** | Normal colon Ecs | 96,000 | P1H12 |
| **Colon T*** | Tumor colon Ecs | 96,000 | P1H12 |
| | | | |
| **Fetal Brain** | Normal bulk | 204,000 | - |
| **Fetal Kidney** | Normal bulk | 50,000+ | - |

Genes specific for endothelial cells showed expression levels consistent with the previously examined colon endothelial SAGE data (Table 4). Additionally, markers specific for epithelial, hematopoeitic or glial cells showed limited or no expression in the brain endothelial libraries suggesting little contamination from non-endothelial cell populations (Table 4). Finally, the data generated here allow for the derivation of a12 gene EC prediction class of which 6 have been previously described as EC-specific (Huminiecki, L., and Bicknell, R. (2000). In silico cloning of novel endothelial-specific genes. Genome Res 10, 1796-806.) (data not shown). This provides further evidence of pure EC populations used for this study.

**Table 4. Cell specificity markers.**

| **Gene** | **Specificity** | **Colon N** | **Colon T** | **Brain N1** | **Brain N2** | **Brain T1** | **Brain T2** | **Brain T3** |
|---|---|---|---|---|---|---|---|---|
| **Hevin** | EC | 161 | 69 | 51 | 99 | 223 | 121 | 48 |
| **VWF** | EC | 35 | 33 | 12 | 53 | 37 | 51 | 110 |
| **Tie2/Tek** | EC | 4 | 2 | 2 | 4 | 1 | 4 | 3 |
| **CD34** | EC | 5 | 2 | 3 | 10 | 12 | 4 | 11 |
| | | | | | | | | |
| **CD14** | Hematopoeitic | 1 | 1 | 1 | 2 | 0 | 0 | 1 |
| **CK8** | Epithelial | 1 | 2 | 0 | 0 | 2 | 1 | 1 |
| **GLUT1** | Brain EC | 0 | 1 | 8 | 37 | 2 | 25 | 8 |
| **GFAP** | Glial | 0 | 0 | 0 | 0 | 0 | 0 | 0 |

Genes expressed preferentially in glioma derived endothelial cells as opposed to normal endothelial cells are potentially involved in regulating angiogenesis-dependent tumor growth. Specific parameters for the sorting of SAGE data and the layering of additional statistical filters allowed for a conservative estimate of legitimate differentially expressed genes (see Methods). Excluding mitochondrial genes, 131 genes were observed to be induced in the glioma endothelial cells based on a four fold induction ratio. Only 14 genes can be entertained as glioma-specific when additional statistical filters are applied (Table 5). In this case, a two fold parameter family of distributions was used to estabish a 90% probability of observing at least a 2 fold difference in values. Only one of these twelve genes, apolipoprotein D, shows higher expression in the stage III glioma than at least one of the stage IV tumors. This suggests that many of the highly induced glioma endothelial genes revealed in this analysis may be involved in later stages of angiogenesis where the initiation of vascular sprouting has already occurred or are glioma type specific showing representation in the astrocytoma and not oligodendroglioma-derived ECs.. Less highly induced genes, or genes primarily induced in the less aggressive tumor stage, may be more reflective of angiogenesis initiation. Several genes regulating extracellular matrix architecture are revealed as highly induced in this study. HSPG2 (perlecan), several type IV collagen transcript variants, and matrix metalloprotease 14 (MMP14) have all been shown to play a role in remodeling the extracelullar matrix. Interestingly, other genes that play roles in either cellular signaling or cell-cell communication are also highly expressed exclusively in glioma-associated endothelial cells. Melanoma associated antigen (MG50), endothelin receptor, the G-protein coupled receptor RDC-1, and integrin αV are all cell surface proteins previously demonstrated to play a role in signaling cascades. Although the endothelin receptor, RDC-1 and integrin αV have previously been shown to regulate angiogenesis, MG50 does not have an association with angiogenesis. Moreover, MG50 was previously shown to be selectively associated with several types of tumor cells with a function yet to be defined. It is noteworthy that the p53-induced, brain-specific angiogenesis inhibitor (BAI-1) was expressed to significant levels but restricted to the earlier stage tumor present in this study (data not shown). It is possible that the loss of expression of BAI-1 in the later tumor stages reflects the need to more aggressively advance vascular development. Other than the detection of a differential HEYL SAGE tag, no other colon endothelial markers were observed to be preferentially expressed in the grade III tumor. In total, of the 14 tumor induced genes listed, 12 are either present on the cell surface or secreted. The localization of the remaining two gene products has yet to be determined as these genes remain uncharacterized. Finally, it is noteworthy that only a select few genes show significant (>2 tags) expression in a fetal brain library where angiogenesis is expected to be robust.

In contrast to the highly biased localization of glioma-induced endothelial cell gene expression defined above, genes that are induced in the normal endothelial cells relative to glioma endothelial cells show a radically different cellular distribution. Twenty-one genes are induced 4 fold or greater in the normal endothelial cells. Filtering for genes with a 50% or greater chance of having greater than 2 fold difference in transcript abundance reduces this list to 14 genes (Table 6). Protein products predicted for these 14 genes show a range of cellular localizations with 4 gene products being intracellular, 5 being integral membrane proteins, 3 extracellular, and one each either secreted, on the cell surface or a nuclear membrane receptor. Several of these genes have functions consistent with either tumor suppressor or anti-angiogenic functions. These anti-proliferative functions have been ascribed to the early growth response gene 1 (EGR1), BTG2, Fruppel-like factor 4 (KLF4), and the serine protease inhibitor SPINT2 although associations with angiogenesis are limited to SPINT2. The down-regulation of these genes in each of the three glioma tumors suggests that these genes may function to encode proteins with anti-angiogenic properties. Both SPINT2 and BTG2 are secreted and may act via paracrine mechanisms. Also noteworthy is the preferential expression of the secreted protein MT1A as this metalothionein may serve as an antioxidant potentially attenuating DNA damage within adjacent cells. Interestingly, EGR1 and KLF4 encode transcription factors suggesting that some part of the anti-angiogenic pathway revealed here may be initiated by these gene products. With the exception of MT1A, none of the above genes show differential expression in colon tumor ECs and may therefore be glioma-specific EC markers.

The specificity of gene expression for tumor EC subtypes is important to define and can be addressed with the glioma EC data integrated with data obtained previously for colon EC populations. A limited number of genes are preferentially expressed in both brain and colon normal EC populations. In contrast, 16 genes were induced at least 4 fold in both colon and brain tumor EC fractions. 12 of these genes also met the criteria of having a greater than 50% chance of being at least 2 fold differential (Table 7). The majority of these genes (7) are collagen transcripts. However, tumor endothalial marker 1 (TEM1), THY1, and RDC-1 also show consistent induction in the different tumor EC cells. This limited conservation of tumor-induced EC expression suggests highly specific EC expression profiles dependent on the tissue source. TEM1 expression has been validated on tissue arrays harboring tissue slices from astrocytomas (data not shown).

Defining the specificity of gene expression to particular cell types can assist in determining function and designing therapeutics. Our non-endothelial cell SAGE database currently contains 76 libraries encoding 255,000 unique SAGE transcripts. The epithelial cell lines derive from lung, ovary, kidney, prostate, breast, colon, pancreas. Additional non-epithelial sources include cardiomyocytes, melanocytes, glioblastoma and monocytes. Genes which show induction in glioma ECs and demonstrate a restricted expression in non-EC cells may be ideal targets for anti-angiogenic therapies. Allowing for 1 or fewer tags in any non-EC library and at least a four-fold induction in glioma ECs yielded only 5 genes (Table 8). Some of these genes are likely not EC-specific due to the relatively limited number of cell types included within the non-EC database. However, both PV-1 and Plexin A2 (PLXNA2) are interesting genes with potential functional relevance to angiogenesis regulation.

The SAGE tag that defines PLXNA2 falls outside of the current mRNA boundaries residing 3' of the ultimate exon. RT-PCR results, however, have confirmed transcription of mRNA containing this tag in the tumor samples used to derive the SAGE data. Plexins share homology with the scatter factor/hepatocyte growth factor (SF/HGF) family of receptors encoded by the MET gene family [Tamagnone, L., Artigiani, S., Chen, H., He, Z., Ming, G. I., Song, H., Chedotal, A., Winberg, M. L., Goodman, C. S., Poo, M., Tessier-Lavigne, M., and Comoglio, P. M. (1999). Plexins are a large family of receptors for transmembrane, secreted, and GPI-anchored semaphorins in vertebrates. Cell 99, 71-80.] Earlier results have demonstrated a link between SF/HGF expression and increase tumorigencity [Bowers, D. C., Fan, S., Walter, K. A., Abounader, R., Williams, J. A., Rosen, E. M., and Laterra, J. (2000). Scatter factor/hepatocyte growth factor protects against cytotoxic death in human glioblastoma via phosphatidylinositol 3-kinase- and AKT-dependent pathways. Cancer Res 60, 4277-83.] Moreover, SF/HGF promotes this increased tumorigencity with concordant stimulation in angiogenesis [Lamszus, K., Laterra, J., Westphal, M., and Rosen, E. M. (1999). Scatter factor/hepatocyte growth factor (SF/HGF) content and function in human gliomas. Int J Dev Neurosci 17, 517-30.] In vivo targeting of SF/HGF was demonstrated to inhibit glioma growth and angiogenesis [Abounader, R., Lal, B., Luddy, C., Koe, G., Davidson, B., Rosen, E. M., and Laterra, J. (2002). In vivo targeting of SF/HGF and c-met expression via UlsnRNA/ribozymes inhibits glioma growth and angiogenesis and promotes apoptosis. Faseb J 16, 108-10.]. Plexins are known to function as coreceptors with neuropilin 1 functioning as a receptor for semaphorin and, in turn, regulating neuronal guidance and cell association [Tamagnone, 1999, *supra*]*.* As neuropilin-1 and Plexin association can serve to receive signals from semaphorins to guide neuronal growth, it is conceivable that a Plexin-neuropilin association may regulate angiogenic growth in a manner analogous to KDR-neuropilin complexes signaling VEGF responses. Plexin A2 shows very low level expression in colon ECs and is not differentially induced in colon tumor ECs. It is noteoworthy that another plexin, plexin B2 (PLXNB2), also showed a five fold increase in glioma EC expression but did not make the statistical threshold demanded for Table 8. Plexin B2 was previously shown to be differentially induced in brain tumors [Shinoura, N., Shamraj, O. I., Hugenholz, H., Zhu, J. G., McBlack, P., Warnick, R., Tew, J. J., Wani, M. A., and Menon, A. G. (1995). Identification and partial sequence of a cDNA that is differentially expressed in human brain tumors. Cancer Lett 89, 215-21.] The upregulation of plexins in glioma ECs allows for a hypothesis whereby SF/HGF directly stimulates EC migration and proliferation. The novel discovery of a consistently upregulated level of Plexin A2 in gliomas requires further evidence for a functional link between tumor levels of plexin A2 and angiogenesis regulation, particularly in the brain.

PV-1 (also called PLVAP for plasmallema vesicle associated protein), is a recently discovered type II integral membrane glycoprotein shown to colocalize with caveolin-1. Stan, R. V., Arden, K. C., and Palade, G. E. (2001). cDNA and protein sequence, genomic organization, and analysis of cis regulatory elements of mouse and human PLVAP genes. Genomics 72, 304-13. Interestingly, this protein was the first to be shown to localize to the stomatal diaphragms and transendothelial channels within caveolae. The specific function of PV-1 remains unknown. PV-1 is expressed at substantial levels in colon ECs but is not expressed differentially between normal and tumor colon ECs. The upregulation of this caveolae-associated protein in gliomas may provide a means for specifically targeting glioma-associated endothelial cells as well as potentially providing a therapeutic delivery mechanism to the underlying tumorigenic cells (Marx, J. (2001). Caveolae: a once-elusive structure gets some respect. Science 294, 1862-5.))

From this study there is also the potential to define brain EC specific genes irrespective of function or differential expression in normal or tumor tissue. Applying the same criteria as that applied for defining EC restricted glioma induced genes, only two genes, TNFα-induced protein 3 and JCTNB, show consistent expression in the brain EC samples but severely limited expression in non-EC databases.

The blood brain barrier within brain capillary endothelial cells results in a restricted diffusion of both small and large molecules as compared to non-brain EC junction complexes. As a result of this, brain capillary ECs facilitate molecular exchange via a tightly regulated, or catalyzed transport system. Any differential expression of catalyzed membrane transporters between normal and tumor tissue may provide a means to selectively deliver therapies to tumor cells. The insulin receptor (IR) has been known for some time to be a marker for brain capillary ECs and to facilitate delivery of drugs. One of the most highly induced, glioma-specific genes in this study is the IR (Table 8). The high induction of IR transcripts in gliomas was not previously recognized and may provide a selective delivery mechanism to cancer cells as these receptors are also proposed to reside within caveolae structures [Smith, R. M., Jarret, L. (1988). Lab. Invest. 58, 613-629.] Overall, very few transporters showed a differential induction in glioma-associated ECs as compared to their normal counterpart (Table 9). This is counter to previous suggestions linking altered expression of transporters with histologic grade of CNS tumors [Guerin, C., Wolff, J. E., Laterra, J., Drewes, L. R., Brem, H., and Goldstein, G. W. (1992). Vascular differentiation and glucose transporter expression in rat gliomas: effects of steroids. Ann Neurol 31, 481-7.] Only one other gene, SLC1A5 Solute carrier family 1 member 5 (neutral amino acid transporter), showed a greater than 4 fold induction in glioma-derived ECs. It should be stated, however, that the standard SAGE tag for integrin αV is shared with aquaporin. Long tag derivations of these two genes revealed that both integrin αV and aquaporin are induced in glioma ECs. Aquaporin may play a role in caveolae swelling that accompanies VEGF stimulated EC growth [Roberts, W. G., and Palade, G. E. (1997). Neovasculature induced by vascular endothelial growth factor is fenestrated. Cancer Res 57, 765-72.] Only one membrane transporter, Na+/K+ transporting ATP1A2 ATPase, was reciprocally repressed in glioma-derived ECs. It remains possible that certain transporters were missed in this analysis due to incorrect functional assignment. Nonetheless, the low number of differentially regulated transport facilitators suggests a small number of these genes need to be transcriptionally activated to accommodate any necessary increase in protein abundance required for tumor growth.
Table 10 shows genes induced in glioma endothelial cells but not in colon tumor or breast tumor endothelial cells.
Table 11 shows genes which encode transporters which are repressed in glioma endothelial cells.
Table 12 shows genes which encode proteins which are localized to the nucleus of both brain and colon tumor endothelial cells.
Table 13 shows genes which encode proteins which are localized to the cytoplasm of both brain and colon tumor endothelial cells.
Table 14 shows genes which encode proteins which are extracellular from both brain and colon tumor endothelial cells.
Table 15 shows genes which encode proteins which are localized to the membrane of both brain and colon tumor endothelial cells.
Table 16 shows genes which encode proteins which are induced in both brain and colon tumor endothelial cells.
Table 17 shows additional tumor endothelial markers in brain.
Table 18 shows tumor endothelial markers in the brain which are cytoplasmic.
Table 19 shows tumor endothelial markers in the brain which are nuclear.
Table 20 shows tumor endothelial markers in the brain which are membrane associated.
Table 21 shows tumor endothelial markers in the brain which are extracellular.
Table 22 shows tumor endothelial markers in the brain which are unsorted with respect to cellular localization.

### References

Abounader, R., Lal, B., Luddy, C., Koe, G., Davidson, B., Rosen, E. M., and Laterra, J. (2002). In vivo targeting of SF/HGF and c-met expression via U1snRNA/ribozymes inhibits glioma growth and angiogenesis and promotes apoptosis. Faseb J 16, 108-10.
Bart, J., Groen, H. J., Hendrikse, N. H., van der Graaf, W. T., Vaalburg, W., and de Vries, E. G. (2000). The blood-brain barrier and oncology: new insights into function and modulation. Cancer Treat Rev 26, 449-62.
Bernsen, H. J., Rijken, P. F., Oostendorp, T., and van der Kogel, A. J. (1995). Vascularity and perfusion of human gliomas xenografted in the athymic nude mouse. Br J Cancer 71, 721-6.
Bowers, D. C., Fan, S., Walter, K. A., Abounader, R., Williams, J. A., Rosen, E. M., and Laterra, J. (2000). Scatter factor/hepatocyte growth factor protects against cytotoxic death in human glioblastoma via phosphatidylinositol 3-kinase- and AKT- dependent pathways. Cancer Res 60, 4277-83.
Chen, H., Centola, M., Altschul, S. F., and Metzger, H. (1998). Characterization of gene expression in resting and activated mast cells. J Exp Med 188, 1657-68.
Guerin, C., Wolff, J. E., Laterra, J., Drewes, L. R., Brem, H., and Goldstein, G. W. (1992). Vascular differentiation and glucose transporter expression in rat gliomas: effects of steroids. Ann Neurol 31, 481-7.
Hobbs, S. K., Monsky, W. L., Yuan, F., Roberts, W. G., Griffith, L., Torchilin, V. P., and Jain, R. K. (1998). Regulation of transport pathways in tumor vessels: role of tumor type and microenvironment. Proc Natl Acad Sci U S A 95, 4607-12.
Holash, J., Maisonpierre, P. C., Compton, D., Boland, P., Alexander, C. R., Zagzag, D., Yancopoulos, G. D., and Wiegand, S. J. (1999). Vessel cooption, regression, and growth in tumors mediated by angiopoietins and VEGF. Science 284, 1994-8.
Huminiecki, L., and Bicknell, R. (2000). In silico cloning of novel endothelial-specific genes. Genome Res 10, 1796-806.
Lamszus, K., Laterra, J., Westphal, M., and Rosen, E. M. (1999). Scatter factor/hepatocyte growth factor (SF/HGF) content and function in human gliomas. Int J Dev Neurosci 17, 517-30.
Marx, J. (2001). Caveolae: a once-elusive structure gets some respect. Science 294, 1862-5.
Roberts, W. G., and Palade, G. E. (1997). Neovasculature induced by vascular endothelial growth factor is fenestrated. Cancer Res 57, 765-72.
Shinoura, N., Shamraj, O. I., Hugenholz, H., Zhu, J. G., McBlack, P., Warnick, R., Tew, J. J., Wani, M. A., and Menon, A. G. (1995). Identification and partial sequence of a cDNA that is differentially expressed in human brain tumors. Cancer Lett 89, 215-21.
Smith, R. M., Jarret, L. (1988). Lab. Invest. 58, 613-629.
St Croix, B., Rago, C., Velculescu, V., Traverso, G., Romans, K. E., Montgomery, E., Lal, A., Riggins, G. J., Lengauer, C., Vogelstein, B., and Kinzler, K. W. (2000). Genes expressed in human tumor endothelium. Science 289, 1197-202.
Stan, R. V., Arden, K. C., and Palade, G. E. (2001). cDNA and protein sequence, genomic organization, and analysis of cis regulatory elements of mouse and human PLVAP genes. Genomics 72, 304-13.
Tamagnone, L., Artigiani, S., Chen, H., He, Z., Ming, G. I., Song, H., Chedotal, A., Winberg, M. L., Goodman, C. S., Poo, M., Tessier-Lavigne, M., and Comoglio, P. M. (1999). Plexins are a large family of receptors for transmembrane, secreted, and GPI-anchored semaphorins in vertebrates. Cell 99, 71-80.
Vajkoczy, P., and Menger, M. D. (2000). Vascular microenvironment in gliomas. J Neurooncol 50, 99-108.
Vajkoczy, P., Schilling, L., Ullrich, A., Schmiedek, P., and Menger, M. D. (1998). Characterization of angiogenesis and microcirculation of high-grade glioma: an intravital multifluorescence microscopic approach in the athymic nude mouse. J Cereb Blood Flow Metab 18, 510-20.
Vick, N. A., and Bigner, D. D. (1972). Microvascular abnormalities in virally-induced canine brain tumors. Structural bases for altered blood-brain barrier function. J Neurol Sci 17, 29-39.

Clauses:
1. A method to aid in diagnosing glioma, comprising the steps of:
   detecting an expression product of at least one gene in a first brain tissue sample suspected of being neoplastic wherein said at least one gene is selected from the group consisting of signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PR00628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(spl); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; S100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds;
      and
   comparing expression of the at least one gene in the first brain tissue sample with expression of the at least one gene in a second brain tissue sample which is normal, wherein increased expression of the at least one gene in the first brain tissue sample relative to the second tissue sample identifies the first brain tissue sample as likely to be neoplastic.
2. The method of clause 1 wherein the increased expression of the at least one gene in the first brain tissue sample relative to the second tissue sample is at least two-fold higher.
3. The method of clause 1 wherein the increased expression of the at least one gene in the first brain tissue sample relative to the second tissue sample is at least five-fold higher.
4. The method of clause 1 wherein the increased expression of the at least one gene in the first brain tissue sample relative to the second tissue sample is at least ten-fold higher.
5. The method of clause 1 wherein the expression product is RNA.
6. The method of clause 1 wherein the expression product is protein.
7. The method of clause 1 wherein the first and second tissue samples are from a human.
8. The method of clause 1 wherein the first and second tissue samples are from the same human.
9. The method of clause 6 wherein the step of detecting is performed using a Western blot.
10. The method of clause 6 wherein the step of detecting is performed using an immunoassay.
11. The method of clause 6 wherein the step of detecting is performed using an immunohistochemical assay.
12. The method of clause 5 wherein the step of detecting is performed using SAGE.
13. The method of clause 5 wherein the step of detecting is performed using hybridization to a microarray.
14. A method of treating a glioma, comprising the step of:
   contacting cells of the glioma with an antibody, wherein the antibody specifically binds to an extracellular epitope of a protein selected from the group consisting of plasmalemma vesicle associated protein; KIAA0726 gene product; osteonectin: laminin, alpha 5; collagen, type IV, alpha 1; insulin-like growth factor binding protein 7; Thy-1 I cell surface antigen; dysferlin, limb girdle muscular dystrophy 2B; integrin, alpha 5; matrix metalloproteinase 9; Lutjheran blood group, integrink, alpha 10, collagen, type VI, alpha 2; glioma endothelial marker 1 precursor; translocase of inner mitochondrial membrane 17 homolog A; heparan sulfate proteoglycan 2; annexin A2; matrix metalloproteinase 10; G protein-coupled receptor; matrix metalloproteinase 14; solute carrier family 29, member 1; CD59 antigen p18-20; KIAA 1870 protein; plexin B2; lectin, glactoside-binding, soluble, 8; integrin beta 4 binding protein; acetyl LDL receptor; laminin, gamma 3; macrophage migration inhibitory factor; gap junction p roein, alpha 1, 43 kD; aquaporin 1; protease, serine, 11; collagen, type IV, alpha 2; apolipoprotein D; plasminogen activator, urokinase; insulin-like growth factor binding protein 3; regulator of G-protein signaling 12; prosaposin; laminin, alpha 4; lectin, galactoside-binding, soluble, 3 binding protein; glycophorin C; endothelin receptor type B; biglycan; transmembrane 4 superfamilyh member 2; lysyl osidase-like 2; TEK tyrosine kinase, endothelial; insulin receptore; cell membrane glycoprotein, 110000M( r ); jagged 1; plasmalemma vesicle associated protein; TEM13, Thy-1 cell surface antigen; coagulation factor II (thrombin) receptor-like 3; dysferlin, limb girdle muscular dystrophy 2B (autosomal recessive); sema domain, transmembrane domain (TM), and cytoplasmic domain, (semaphorin) 6B; integrin, alpha 5 (fibronectin receptor, alpha polypeptide); likely ortholog of rat vacuole membrane protein 1; nerve growth factor receptor (TNFR superfamily, member 16); degenerative spermatocyte homolog, lipid desaturase (Drosophila); TEM1, endosialin; heme oxygenase (decycling) 1; G protein-coupled receptor; C-type (calcium dependent, carbohydrate-recognition domain) lectin, superfamily member 9; matrix metalloproteinase 14 (membrane-inserted); solute carrier family 29 (nucleoside transporters), member 1; likely ortholog of mouse embryonic epithelial gene 1; major histocompatibility complex, class I, C; likely ortholog of mouse fibronectin type III repeat containing protein 1; sprouty homolog 4 (Drosophila); KIAA0620 protein; coagulation factor III (thromboplastin, tissue factor); aquaporin 1 (channel-forming integral protein, 28kDa); major histocompatibility complex, class I, B; Lysosomal-associated multispanning membrane protein-5; endothelin receptor type B; insulin receptor; complement component 1, q subcomponent, receptor 1; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16) and complement component 1, q subcomponent, receptor 1; whereby immune destruction of cells of the glioma is triggered.
15. The method of clause 14 wherein the antibody is conjugated to a diagnostic or therapeutic reagent.
16. The method of clause 14 wherein the glioma is multidrug-sensitive.
17. The method of clause 15 wherein the reagent is a chemotherapeutic agent.
18. The method of clause 15 wherein the reagent is a cytotoxin.
19. The method of clause 15 wherein the reagent is a non-radioactive label.
20. The method of clause 15 wherein the reagent is a radioactive compound.
21. The method of clause 14 wherein the glioma is in a human.
22. A method of identifying a test compound as a potential anti-cancer or anti-glioma drug, comprising the step of:
   contacting a test compound with a cell which expresses at least one gene selected from the group consisting of signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 I cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(sp1); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; S100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds;
   monitoring an expression product of the at least one gene; and
   identifying the test compound as a potential anti-cancer drug if it decreases the expression of the at least one gene.
23. The method of clause 22 wherein the cell is a human cell.
24. The method of clause 22 wherein the cell is a glioma cell.
25. The method of clause 22 wherein the cell is a human glioma cell.
26. The method of clause 22 wherein the expression product is RNA.
27. The method of clause 22 wherein the expression product is protein.
28. The method of clause 22 wherein the cell overexpresses the at least one gene relative to a normal cell of the same tissue.
29. The method of clause 22 wherein expression of at least two of said genes is monitored.
30. The method of clause 22 wherein expression of at least three of said genes is monitored.
31. The method of clause 22 wherein expression of at least four of said genes is monitored.
32. The method of clause 22 wherein the test compound is identified if the decrease in expression is at least 50 %.
33. The method of clause 22 wherein the test compound is identified if the decrease in expression is at least 80 %.
34. The method of clause 22 wherein the decrease in expression is at least 90 %.
35. The method of clause 22 wherein the test compound is identified as an anti-glioma drug.
36. A method to aid in diagnosing glioma, comprising the steps of:
   detecting an mRNA of at least one gene in a first brain tissue sample suspected of being neoplastic wherein said at least one gene is identified by a tag selected from the group consisting of SEQ ID NO: 1-32; and
   comparing expression of the at least one gene in the first brain tissue sample with expression of the at least one gene in a second brain tissue sample which is normal, wherein increased expression of the at least one gene in the first brain tissue sample relative to the second tissue sample identifies the first brain tissue sample as likely to be neoplastic.
37. The method of clause 36 wherein the increased expression of the at least one gene in the first brain tissue sample relative to the second tissue sample is at least two-fold higher.
38. The method of clause 36 wherein the increased expression of the at least one gene in the first brain tissue sample relative to the second tissue sample is at least five-fold higher.
39. The method of clause 36 wherein the increased expression of the at least one gene in the first brain tissue sample relative to the second tissue sample is at least ten-fold higher.
40. The method of clause 36 wherein the first and second tissue samples are from a human.
41. The method of clause 36 wherein the first and second tissue samples are from the same human.
42. The method of clause 36 wherein the step of detecting is performed using a Western blot.
43. The method of clause 36 wherein the step of detecting is performed using an immunoassay.
44. The method of clause 36 wherein the step of detecting is performed using an immunohistochemical assay.
45. The method of clause 36 wherein the step of detecting is performed using SAGE.
46. The method of clause 36 wherein the step of detecting is performed using hybridization to a microarray.
47. A method of identifying a test compound as a potential anti-cancer or anti-glioma drug, comprising the step of:
   contacting a test compound with a cell which expresses an mRNA of at least one gene identified by a tag selected from the group consisting of SEQ ID NO: 1-32;
   monitoring an mRNA of the at least one gene; and
   identifying the test compound as a potential anti-cancer drug if it decreases the expression of the at least one gene.
48. The method of clause 47 wherein the cell is a human cell.
49. The method of clause 47 wherein the cell is a glioma cell.
50. The method of clause 47 wherein the cell is a human glioma cell.
51. The method of clause 47 wherein the expression product is RNA.
52. The method of clause 47 wherein the expression product is protein.
53. The method of clause 47 wherein the cell overexpresses the at least one gene relative to a normal cell of the same tissue.
54. The method of clause 47 wherein expression of at least two of said genes is monitored.
55. The method of clause 47 wherein expression of at least three of said genes is monitored.
56. The method of clause 47 wherein expression of at least four of said genes is monitored.
57. The method of clause 47 wherein the test compound is identified if the decrease in expression is at least 50 %.
58. The method of clause 47 wherein the test compound is identified if the decrease in expression is at least 80 %.
59. The method of clause 47 wherein the decrease in expression is at least 90 %.
60. The method of clause 47 wherein the test compound is identified as an anti-glioma drug.
61. A method to induce an immune response to glioma, comprising:
   administering to a mammal a protein or nucleic acid encoding a protein selected from the group consisting of:
      signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(sp1); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC:17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; 5100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds, whereby an immune response to the protein is induced.
62. The method of clause 61 wherein a protein is administered.
63. The method of clause 61 wherein a nucleic acid is administered.
64. The method of clause 63 wherein the nucleic acid is administered intramuscularly.
65. The method of clause 62 further comprising administering an immune adjuvant to the mammal.
66. The method of clause 61 wherein the mammal has a glioma.
67. The method of clause 61 wherein the mammal has had a glioma surgically removed.

## Claims

1. A method to aid in diagnosing glioma, comprising the steps of:
detecting an expression product of at least one gene in a first brain tissue sample suspected of being neoplastic wherein said at least one gene is plasmalemma vesicle associated protein
and
comparing expression of the at least one gene in the first brain tissue sample with expression of the at least one gene in a second brain tissue sample which is normal, wherein increased expression of the at least one gene in the first brain tissue sample relative to the second tissue sample identifies the first brain tissue sample as likely to be neoplastic.

2. An antibody, optionally conjugated to a diagnostic or therapeutic reagent, which specifically binds to an extracellular epitope of plasmalemma vesicle associated protein for use in treating a glioma, especially whereby immune destruction of cells of the glioma is triggered.

3. A method of identifying a test compound as a potential anti-cancer or anti-glioma drug, comprising the step of:
contacting a test compound with a cell which expresses plasmalemma vesicle associated protein monitoring an expression product of the plasmalemma vesicle associated protein; and
identifying the test compound as a potential anti-cancer drug if it decreases the expression of the plasmalemma vesicle associated protein.

4. A method to aid in diagnosing glioma, comprising the steps of:
detecting an mRNA of at least one gene in a first brain tissue sample suspected of being neoplastic wherein said at least one gene is identified by a tag selected from the group consisting of SEQ ID NO: 1-32; and
comparing expression of the at least one gene in the first brain tissue sample with expression of the at least one gene in a second brain tissue sample which is normal, wherein increased expression of the at least one gene in the first brain tissue sample relative to the second tissue sample identifies the first brain tissue sample as likely to be neoplastic.

5. A method of identifying a test compound as a potential anti-cancer or anti-glioma drug, comprising the step of:
contacting a test compound with a cell which expresses an mRNA of at least one gene identified by a tag selected from the group consisting of SEQ ID NO: 1-32;
monitoring an mRNA of the at least one gene; and
identifying the test compound as a potential anti-cancer drug if it decreases the expression of the at least one gene.

6. Plasmalemma vesicle associated protein or a nucleic acid encoding plasmalemma vesicle associated protein for use in inducing an immune response to a glioma.

7. The method of claim 1 or 4 wherein the increased expression of the at least one gene in the first brain tissue sample relative to the second tissue sample is at least two-fold higher; preferably at least five-fold higher; more preferably at least ten-fold higher.

8. The method of claim 1, 2 or 5 wherein the expression product is RNA or protein.

9. The method of claim 1 or 3 wherein the first and second tissue samples are from a human, especially from the same human.

10. The antibody of claim 2 wherein the antibody is conjugated to a diagnostic or therapeutic reagent.

11. The antibody of claim 2 wherein the glioma is multidrug-sensitive, and/or wherein the glioma is in a human.

12. The antibody of claim 2 wherein the reagent is a chemotherapeutic agent; or a cytotoxin; or a non-radioactive label; or a radioactive compound.

13. The method of claim 2 or 5 wherein the cell is a human cell and/or a glioma cell,

14. The method of claim 2 or 5 wherein the cell overexpresses the at least one gene relative to a normal cell of the same tissue.

15. The method of claim 2 or 5 wherein expression of at least one, preferably at least two, more preferably three additional genes selected from the group consisting of signal sequence receptor, delta (translocon-associated protein delta); DC2 protein; KIAA0404 protein; symplekin; Huntingtin interacting protein I; plasmalemma vesicle associated protein; KIAA0726 gene product; latexin protein; transforming growth factor, beta 1; hypothetical protein FLJ22215; Rag C protein; hypothetical protein FLJ23471; N-myristoyltransferase 1; hypothetical protein dJ1181N3.1; ribosomal protein L27; secreted protein, acidic, cysteine-rich (osteonectin); Hs 111988; Hs 112238; laminin, alpha 5; protective protein for beta-galactosidase (galactosialidosis); Melanoma associated gene; Melanoma associated gene; E3 ubiquitin ligase SMURF1; collagen, type IV, alpha 1; collagen, type IV, alpha 1; collagen, type IV, alpha 1; insulin-like growth factor binding protein 7; gene predicted from cDNA with a complete coding sequence; Thy-1 cell surface antigen; Hs 127824; GTP binding protein 2; Homo sapiens mRNA; cDNA DKFZp586D0918 (from clone DKFZp586D0918); cutaneous T-cell lymphoma-associated tumor antigen se20-4; differentially expressed nucleolar TGF-betal target protein (DENTT); dysferlin, limb girdle muscular dystrophy 2B (autosomal recessive); smoothelin; integrin, alpha 5 (fibronectin receptor, alpha polypeptide); putative translation initiation factor; retinoic acid induced 14; matrix metalloproteinase 9 (gelatinase B, 92kD gelatinase, 92kD type IV collagenase); Lutheran blood group (Auberger b antigen included); stanniocalcin 2; nuclear factor (erythroid-derived 2)-like 2; protein tyrosine phosphatase, non-receptor type 1; integrin, alpha 10; collagen, type VI, alpha 2; chromosome 21 open reading frame 25; CDC37 (cell division cycle 37, S. cerevisiae, homolog); Hs 16450; Rho guanine nucleotide exchange factor (GEF) 7; creatine kinase, brain; hypothetical protein FLJ10297; hypothetical protein FLJ10350; TNF-induced protein; tumor necrosis factor receptor superfamily, member 12 (translocating chain-association membrane protein); cofilin 1 (non-muscle); splicing factor proline/glutamine rich (polypyrimidine tract-binding protein-associated); splicing factor proline/glutamine rich (polypyrimidine tract-binding protein-associated); v-ets avian erythroblastosis virus E26 oncogene homolog 1; protease, cysteine, 1 (legumain); ribosomal protein L13; chromosome 22 open reading frame 5; zinc finger protein 144 (Mel-18); degenerative spermatocyte (homolog Drosophila; lipid desaturase); eukaryotic translation initiation factor 2C, 2; mitochondrial ribosomal protein L45; prostate tumor over expressed gene 1; NADH dehydrogenase (ubiquinone) 1 alpha subcomplex, 7 (14.5kD, B14.5a); glioma endothelial marker 1 precursor; NS1-binding protein; ribosomal protein L38; tuftelin-interacting protein; HLA class II region expressed gene KE2; translocase of inner mitochondrial membrane 17 homolog A (yeast); sudD (suppressor of bimD6, Aspergillus nidulans) homolog; heparan sulfate proteoglycan 2 (perlecan); SEC24 (S. cerevisiae) related gene family, member A; NADH dehydrogenase (ubiquinone) Fe-S protein 7 (20kD) (NADH-coenzyme Q reductase); DNA segment on chromosome X and Y (unique) 155 expressed sequence; annexin A2; Homo sapiens clone 24670 mRNA sequence; hypothetical protein; matrix metalloproteinase 10 (stromelysin 2); KIAA1049 protein; G protein-coupled receptor; hypothetical protein FLJ20401; matrix metalloproteinase 14 (membrane-inserted); KIAA0470 gene product; solute carrier family 29 (nucleoside transporters), member 1; stanniocalcin 1; stanniocalcin 1; stanniocalcin 1; tumor suppressor deleted in oral cancer-related 1; tumor suppressor deleted in oral cancer-related 1; apolipoprotein C-I; glutathione peroxidase 4 (phospholipid hydroperoxidase); Hs 272106; transcription factor binding to IGHM enhancer 3; hypothetical protein DKFZp762A227; hypothetical protein FLJ22362; CD59 antigen p18-20 (antigen identified by monoclonal antibodies 16.3A5, EJ16, EJ30, EL32 and G344); PRO0628 protein; melanoma-associated antigen recognised by cytotoxic T lymphocytes; LOC88745; Homo sapiens beta-1,3-galactosyltransferase-6 (B3GALT6) mRNA, complete cds; sprouty (Drosophila) homolog 4; sprouty (Drosophila) homolog 4; Homo sapiens mRNA; cDNA DKFZp434E1515 (from clone DKFZp434E1515); coactosin-like protein; hypothetical protein FLJ21865; Hs296234; KIAA0685 gene product; hypothetical protein FLJ10980; ribosomal protein L10; ribosomal protein S19; Hs 299251; Huntingtin interacting protein K; Homo sapiens mRNA full length insert cDNA clone EUROIMAGE 50374; Hs 311780; Hs 212191; v-akt murine thymoma viral oncogene homolog 2; Hs 328774; transducin-like enhancer of split 2, homolog of Drosophila E(sp1); KIAA1870 protein; ribosomal protein L10a; peptidylprolyl isomerase A (cyclophilin A); Hs 344224; hypothetical protein FLJ23239; hypothetical protein DKFZp761H221; KIAA1887 protein; Homo sapiens mRNA full length insert cDNA clone EUROIMAGE 701679; Homo sapiens cDNA FLJ30634 fis, clone CTONG2002453; Homo sapiens cDNA FLJ32203 fis, clone PLACE6003038, weakly similar to ZINC FINGER PROTEIN 84; Homo sapiens mRNA full length insert cDNA clone EUROIMAGE 1035904; hypothetical protein LOC57333; myosin ID; plexin B2; lectin, galactoside-binding, soluble, 8 (galectin 8); double ring-finger protein, Dorfin; DKFZP434B168 protein; LIM domain binding 2; integrin beta 4 binding protein; synaptopodin; Hs 54828; insulin induced gene 1; acetyl LDL receptor; SREC; excision repair cross-complementing rodent repair deficiency, complementation group 1 (includes overlapping antisense sequence); hypothetical protein FLJ22329; schwannomin-interacting protein 1; PTEN induced putative kinase 1; myosin X; Homo sapiens cDNA FLJ32424 fis, clone SKMUS2000954, moderately similar to Homo sapiens F-box protein Fbx25 (FBX25) 97; golgi phosphoprotein 1; splicing factor, arginine/serine-rich 6; laminin, gamma 3; cysteine-rich protein 2; U6 snRNA-associated Sm-like protein LSm7; hypothetical protein FLJ10707; Homo sapiens, Similar to RIKEN cDNA 2310012N15 gene, clone IMAGE:3342825, mRNA, partial cds; macrophage migration inhibitory factor (glycosylation-inhibiting factor); ubiquinol-cytochrome c reductase hinge protein; gap junction protein, alpha 1, 43kD (connexin 43); dihydropyrimidinase-like 3; aquaporin 1 (channel-forming integral protein, 28kD); protein expressed in thyroid; macrophage myristoylated alanine-rich C kinase substrate; procollagen-lysine, 2-oxoglutarate 5-dioxygenase (lysine hydroxylase, Ehlers-Danlos syndrome type VI); protease, serine, 11 (IGF binding); 24-dehydrocholesterol reductase; collagen, type IV, alpha 2; profilin 1; apolipoprotein D; hyaluronoglucosaminidase 2; hypothetical protein FLJ22678; quiescin Q6; ras homolog gene family, member A; ras homolog gene family, member A; plasminogen activator, urokinase; insulin-like growth factor binding protein 3; uridine phosphorylase; KIAA0638 protein; B7 homolog 3; lamin A/C; lamin A/C; lamin A/C; regulator of G-protein signalling 12; proteasome (prosome, macropain) 26S subunit, non-ATPase, 8; Homo sapiens, Similar to RIKEN cDNA 5730528L13 gene, clone MGC: 17337 IMAGE:4213591, mRNA, complete cds; prosaposin (variant Gaucher disease and variant metachromatic leukodystrophy); laminin, alpha 4; transcription elongation factor A (SII), 1; lectin, galactoside-binding, soluble, 3 binding protein; ribosomal protein S16; glycophorin C (Gerbich blood group); endothelin receptor type B; serine (or cysteine) proteinase inhibitor, clade E (nexin, plasminogen activator inhibitor type 1), member 1; biglycan; small nuclear ribonucleoprotein polypeptide B"; transmembrane 4 superfamily member 2; TAF11 RNA polymerase II, TATA box binding protein (TBP)-associated factor, 28 kD; lysyl oxidase-like 2; SRY (sex determining region Y)-box 4; SOX4 SRY (sex determining region Y)-box 4; SRY (sex determining region Y)-box 4; actin related protein 2/3 complex, subunit 2 (34 kD); Homo sapiens cDNA: FLJ23507 fis, clone LNG03128; hypothetical protein FLJ12442; Fas (TNFRSF6)-associated via death domain; mitogen-activated protein kinase kinase kinase 11; TEK tyrosine kinase, endothelial (venous malformations, multiple cutaneous and mucosal); insulin receptor; cell membrane glycoprotein, 110000M(r) (surface antigen); Homo sapiens cDNA FLJ11863 fis, clone HEMBA1006926; jagged 1 (Alagille syndrome); KIAA0304 gene product; pre-B-cell leukemia transcription factor 2; Homo sapiens cDNA FLJ31238 fis, clone KIDNE2004864; p53-induced protein; complement component 1, q subcomponent, receptor 1; complement component 1, q subcomponent, receptor 1; apolipoprotein E; chemokine (C-C motif) ligand 3; coagulation factor II (thrombin) receptor-like 3; coagulation factor III (thromboplastin, tissue factor); collagen, type I, alpha 1; collagen, type III, alpha 1 (Ehlers-Danlos syndrome type IV, autosomal dominant); C-type (calcium dependent, carbohydrate-recognition domain) lectin, superfamily member 9; cystatin C (amyloid angiopathy and cerebral hemorrhage); endoplasmic reticulum associated protein 140 kDa; ESTs; ESTs; ESTs, Highly similar to hypothetical protein FLJ10350 [Homo sapiens] [H.sapiens]; ESTs, Highly similar to ITB1_HUMAN Integrin beta-1 precursor (Fibronectin receptor beta subunit) (CD29) (Integrin VLA-4 beta subunit) [H.sapiens]; ESTs, Weakly similar to hypothetical protein FLJ20489 [Homo sapiens] [H.sapiens]; ESTs, Weakly similar to T17346 hypothetical protein DKFZp586O1624.1 - human (fragment) [H.sapiens]; ESTs, Weakly similar to T21371 hypothetical protein F25H8.3 - Caenorhabditis elegans [C.elegans]; eukaryotic translation initiation factor 4A, isoform 1; heme oxygenase (decycling) 1; Hermansky-Pudlak syndrome 4; Homo sapiens cDNA FLJ34888 fis, clone NT2NE2017332; Homo sapiens cDNA FLJ39848 fis, clone SPLEN2014669; Homo sapiens mRNA full length insert cDNA clone EUROIMAGE 1977059; Homo sapiens, clone IMAGE:4845226, mRNA; hypothetical protein FLJ22329; hypothetical protein FLJ32205; hypothetical protein MGC4677; inhibin, beta B (activin AB beta polypeptide); insulin-like growth factor binding protein 5; junction plakoglobin; KIAA0620 protein; KIAA0943 protein; likely ortholog of rat vacuole membrane protein 1; Lysosomal-associated multispanning membrane protein-5; major histocompatibility complex, class I, B; major histocompatibility complex, class I, C; matrix Gla protein; matrix metalloproteinase 1 (interstitial collagenase); microtubule-associated protein 1 light chain 3 beta; nerve growth factor receptor (TNFR superfamily, member 16); ribosomal protein S9; ring finger protein 40; S100 calcium binding protein, beta (neural); sema domain, transmembrane domain (TM), and cytoplasmic domain, (semaphorin) 6B; SPARC-like 1 (mast9, hevin); tumor necrosis factor, alpha-induced protein 3; UDP-Gal:betaGlcNAc beta 1,4- galactosyltransferase, polypeptide 3; UDP-GlcNAc:betaGal beta-1,3-N-acetylglucosaminyltransferase 5; von Willebrand factor; v-akt murine thymoma vial oncogene homolog 2; cyclin-dependent kinase (cdc2-like) 10; ortholog mouse myocytic induction/differentiation originator; brain-specific angiogenesis inhibitor 1 ; EGF-TM7 latrophilin-related protein; sema domain ; integrin, alpha 5 ; likely ortholog of mouse fibronectin type III; Lutheran blood group (Auberger b antigen included); SSR4, TRAPD; nerve growth factor receptor (TNFR superfamily, member 16); insulin-like growth factor binding protein; leukemia inhibitory factor; protein tyrosine phosphatase, nonreceptor type I; and Homo sapiens, clone IMAGE:3908182, mRNA, partial cds; is monitored.

16. The method of claim 2 or 5 wherein the test compound is identified if the decrease in expression is at least 50%, preferably at least 80%, more preferably at least 90%.

17. The method of claim 2 or 5 wherein the test compound is identified as an anti-glioma drug.

18. A nucleic acid encoding plasmalemma vesicle associated protein, according to claim 6, wherein the nucleic acid is for intramuscular administration.

19. Plasmalemma vesicle associated protein or a nucleic acid encoding plasmalemma vesicle associated protein, according to claim 6, wherein the plasmalemma vesicle associated protein or nucleic acid encoding the plasmalemma vesicle associated protein is for administration with an immune adjuvant.

20. Plasmalemma vesicle associated protein or a nucleic acid encoding plasmalemma vesicle associated protein, according to claim 6, wherein the plasmalemma vesicle associated protein or nucleic acid encoding the plasmalemma vesicle associated protein is for administration to a mammal, or a mammal from which a glioma has been surgically removed.


SEQUENCE LISTING

<110> Genzyme Corporation
      The Johns Hopkins University

<120> BRAIN ENDOTHELIAL EXPRESSION PATTERNS


<130> 003482.00010

<140> PCT/US03/25614
<141> 2003-08-15

<150> US 60/403,390
<151> 2002-08-15

<150> US 60/458,978
<151> 2003-04-01

<160> 869

<170> FastSEQ for Windows Version 4.0

<210> 1
<211> 10
<212> DNA
<213> Homo sapiens

<400> 1
aaaccattct                                                              10

<210> 2
<211> 10
<212> DNA
<213> Homo sapiens

<400> 2
aaggcaggga                                                              10

<210> 3
<211> 10
<212> DNA
<213> Homo sapiens

<400> 3
acacagcaag                                                              10

<210> 4
<211> 10
<212> DNA
<213> Homo sapiens

<400> 4
agctggagtc                                                              10

<210> 5
<211> 10
<212> DNA
<213> Homo sapiens

<400> 5
agctggcacc                                                              10

<210> 6
<211> 10
<212> DNA
<213> Homo sapiens

<400> 6
ataaatgagg                                                              10

<210> 7
<211> 10
<212> DNA
<213> Homo sapiens

<400> 7
caagcacccc                                                              10

<210> 8
<211> 10
<212> DNA
<213> Homo sapiens

<400> 8
cactacccac                                                              10

<210> 9
<211> 10
<212> DNA
<213> Homo sapiens

<400> 9
cactactcac                                                              10

<210> 10
<211> 10
<212> DNA
<213> Homo sapiens

<400> 10
cccacctcca                                                              10

<210> 11
<211> 10
<212> DNA
<213> Homo sapiens

<400> 11
cccgcctctt                                                              10

<210> 12
<211> 10
<212> DNA
<213> Homo sapiens

<400> 12
cctcagatgt                                                              10

<210> 13
<211> 10
<212> DNA
<213> Homo sapiens

<400> 13
cgctactcac                                                              10

<210> 14
<211> 10
<212> DNA
<213> Homo sapiens

<400> 14
ctaagacctc                                                              10

<210> 15
<211> 10

<212> DNA
<213> Homo sapiens

<400> 15
ctaagacttc                                                              10

<210> 16
<211> 10
<212> DNA
<213> Homo sapiens

<400> 16
gagtgggtgc                                                              10

<210> 17
<211> 10
<212> DNA
<213> Homo sapiens

<400> 17
gggacagctg                                                              10

<210> 18
<211> 10
<212> DNA
<213> Homo sapiens

<400> 18
gggttggctt                                                              10

<210> 19
<211> 10
<212> DNA
<213> Homo sapiens

<400> 19
gtaagtgtac                                                              10

<210> 20
<211> 10
<212> DNA
<213> Homo sapiens

<400> 20
gtaagtgtac                                                              10

<210> 21
<211> 10
<212> DNA
<213> Homo sapiens

<400> 21
gtaggggtaa                                                              10

<210> 22
<211> 10
<212> DNA
<213> Homo sapiens

<400> 22
taaccactgc                                                              10

<210> 23
<211> 10
<212> DNA
<213> Homo sapiens

<400> 23
tactgctcgg                                                              10

<210> 24
<211> 10
<212> DNA
<213> Homo sapiens

<400> 24
tcaggctgaa                                                              10

<210> 25
<211> 10
<212> DNA
<213> Homo sapiens

<400> 25
tccatacacc                                                              10

<210> 26
<211> 10
<212> DNA
<213> Homo sapiens

<400> 26
tccttttaaa                                                              10

<210> 27
<211> 10
<212> DNA
<213> Homo sapiens

<400> 27
tgattaaggt                                                              10

<210> 28
<211> 10
<212> DNA
<213> Homo sapiens

<400> 28
tggtatcaca                                                              10

<210> 29
<211> 10
<212> DNA
<213> Homo sapiens

<400> 29
tggtgtatgc                                                              10

<210> 30
<211> 10
<212> DNA
<213> Homo sapiens

<400> 30
tgtcactggg                                                              10

<210> 31
<211> 10
<212> DNA
<213> Homo sapiens

<400> 31
tgtgggaggc                                                              10

<210> 32
<211> 10
<212> DNA
<213> Homo sapiens

<400> 32
tttaacggcc                                                              10

<210> 33
<211> 10
<212> DNA
<213> Homo sapiens

<400> 33
gctctctatg                                                              10

<210> 34
<211> 10
<212> DNA
<213> Homo sapiens

<400> 34
agaatgaaac                                                              10

<210> 35
<211> 10
<212> DNA
<213> Homo sapiens

<400> 35
aagtggaata                                                              10

<210> 36
<211> 10
<212> DNA
<213> Homo sapiens

<400> 36
gatgacgact                                                              10

<210> 37
<211> 10
<212> DNA
<213> Homo sapiens

<400> 37
ccctttcaca                                                              10

<210> 38
<211> 10
<212> DNA
<213> Homo sapiens

<400> 38
tcctggggca                                                              10

<210> 39
<211> 10
<212> DNA
<213> Homo sapiens

<400> 39
tctattgatg                                                              10

<210> 40
<211> 10
<212> DNA
<213> Homo sapiens

<400> 40
ggggctgtat                                                              10

<210> 41

<211> 10
<212> DNA
<213> Homo sapiens

<400> 41
cccaggacac                                                              10

<210> 42
<211> 10
<212> DNA
<213> Homo sapiens

<400> 42
ggagctgctg                                                              10

<210> 43
<211> 10
<212> DNA
<213> Homo sapiens

<400> 43
tggacagcag                                                              10

<210> 44
<211> 10
<212> DNA
<213> Homo sapiens

<400> 44
tctgggaaca                                                              10

<210> 45
<211> 10
<212> DNA
<213> Homo sapiens

<400> 45
cctgtgtatg                                                              10

<210> 46
<211> 10
<212> DNA
<213> Homo sapiens

<400> 46
ggcaagaaga                                                              10

<210> 47
<211> 10
<212> DNA
<213> Homo sapiens

<400> 47
aaatgcttgg                                                              10

<210> 48
<211> 10
<212> DNA
<213> Homo sapiens

<400> 48
ctaaaaacct                                                              10

<210> 49
<211> 10
<212> DNA
<213> Homo sapiens

<400> 49

gagcattgca                                                              10

<210> 50
<211> 10
<212> DNA
<213> Homo sapiens

<400> 50
ggtggacacg                                                              10

<210> 51
<211> 10
<212> DNA
<213> Homo sapiens

<400> 51
gctcctgagc                                                              10

<210> 52
<211> 10
<212> DNA
<213> Homo sapiens

<400> 52
aagaagtgga                                                              10

<210> 53
<211> 10
<212> DNA
<213> Homo sapiens

<400> 53
tgggaagtgg                                                              10

<210> 54
<211> 10
<212> DNA
<213> Homo sapiens

<400> 54
actcgctctg                                                              10

<210> 55
<211> 10
<212> DNA
<213> Homo sapiens

<400> 55
tttcagggga                                                              10

<210> 56
<211> 10
<212> DNA
<213> Homo sapiens

<400> 56
acaacgtcca                                                              10

<210> 57
<211> 10
<212> DNA
<213> Homo sapiens

<400> 57
gtctcagtgc                                                              10

<210> 58
<211> 10
<212> DNA

<213> Homo sapiens

<400> 58
ccccctgccc                                                              10

<210> 59
<211> 10
<212> DNA
<213> Homo sapiens

<400> 59
agaaaccacg                                                              10

<210> 60
<211> 10
<212> DNA
<213> Homo sapiens

<400> 60
gaccgcagga                                                              10

<210> 61
<211> 10
<212> DNA
<213> Homo sapiens

<400> 61
gtgctacttc                                                              10

<210> 62
<211> 10
<212> DNA
<213> Homo sapiens

<400> 62
gataactaca                                                              10

<210> 63
<211> 10
<212> DNA
<213> Homo sapiens

<400> 63
tggctgtgac                                                              10

<210> 64
<211> 10
<212> DNA
<213> Homo sapiens

<400> 64
gagtgagacc                                                              10

<210> 65
<211> 10
<212> DNA
<213> Homo sapiens

<400> 65
gagtggctac                                                              10

<210> 66
<211> 10
<212> DNA
<213> Homo sapiens

<400> 66
gactcaggga                                                              10

<210> 67
<211> 10
<212> DNA
<213> Homo sapiens

<400> 67
gttatatgcc                                                              10

<210> 68
<211> 10
<212> DNA
<213> Homo sapiens

<400> 68
gaggcgctgc                                                              10

<210> 69
<211> 10
<212> DNA
<213> Homo sapiens

<400> 69
gagctctgag                                                              10

<210> 70
<211> 10
<212> DNA
<213> Homo sapiens

<400> 70
gccagccagt                                                              10

<210> 71
<211> 10
<212> DNA
<213> Homo sapiens

<400> 71
atggcaacag                                                              10

<210> 72
<211> 10
<212> DNA
<213> Homo sapiens

<400> 72
aaggagttac                                                              10

<210> 73
<211> 10
<212> DNA
<213> Homo sapiens

<400> 73
tcccacaagg                                                              10

<210> 74
<211> 10
<212> DNA
<213> Homo sapiens

<400> 74
taaatcccca                                                              10

<210> 75
<211> 10
<212> DNA
<213> Homo sapiens

<400> 75
cccgcccccg                                                              10

<210> 76
<211> 10
<212> DNA
<213> Homo sapiens

<400> 76
cccgaggcag                                                              10

<210> 77
<211> 10
<212> DNA
<213> Homo sapiens

<400> 77
ctacgtgatg                                                              10

<210> 78
<211> 10
<212> DNA
<213> Homo sapiens

<400> 78
atgggtttgc                                                              10

<210> 79
<211> 10
<212> DNA
<213> Homo sapiens

<400> 79
ggcattgtct                                                              10

<210> 80
<211> 10
<212> DNA
<213> Homo sapiens

<400> 80
gtgctaagcg                                                              10

<210> 81
<211> 10
<212> DNA
<213> Homo sapiens

<400> 81
accgtttgca                                                              10

<210> 82
<211> 10
<212> DNA
<213> Homo sapiens

<400> 82
cagcgctgca                                                              10

<210> 83
<211> 10
<212> DNA
<213> Homo sapiens

<400> 83
gaagacactt                                                              10

<210> 84
<211> 10

<212> DNA
<213> Homo sapiens

<400> 84
cgctgggcgt                                                              10

<210> 85
<211> 10
<212> DNA
<213> Homo sapiens

<400> 85
cacccctgat                                                              10

<210> 86
<211> 10
<212> DNA
<213> Homo sapiens

<400> 86
gcccccctgc                                                              10

<210> 87
<211> 10
<212> DNA
<213> Homo sapiens

<400> 87
   ccccctgccc                                                              10

<210> 88
<211> 10
<212> DNA
<213> Homo sapiens

<400> 88
agcataaaaa                                                              10

<210> 89
<211> 10
<212> DNA
<213> Homo sapiens

<400> 89
gggctggacg                                                              10

<210> 90
<211> 10
<212> DNA
<213> Homo sapiens

<400> 90
ctgccaactt                                                              10

<210> 91
<211> 10
<212> DNA
<213> Homo sapiens

<400> 91
aagtggatag                                                              10

<210> 92
<211> 10
<212> DNA
<213> Homo sapiens

<400> 92
cgtactgagc                                                              10

<210> 93
<211> 10
<212> DNA
<213> Homo sapiens

<400> 93
ccgcttactc                                                              10

<210> 94
<211> 10
<212> DNA
<213> Homo sapiens

<400> 94
ggggcttctg                                                              10

<210> 95
<211> 10
<212> DNA
<213> Homo sapiens

<400> 95
cccgtccgga                                                              10

<210> 96
<211> 10
<212> DNA
<213> Homo sapiens

<400> 96
agttccacca                                                              10

<210> 97
<211> 10
<212> DNA
<213> Homo sapiens

<400> 97
ggcctccagc                                                              10

<210> 98
<211> 10
<212> DNA
<213> Homo sapiens

<400> 98
ggaggctgag                                                              10

<210> 99
<211> 10
<212> DNA
<213> Homo sapiens

<400> 99
cagaggcgtc                                                              10

<210> 100
<211> 10
<212> DNA
<213> Homo sapiens

<400> 100
gaccagcctt                                                              10

<210> 101
<211> 10
<212> DNA
<213> Homo sapiens

<400> 101
gaggatggtg                                                              10

<210> 102
<211> 10
<212> DNA
<213> Homo sapiens

<400> 102
tcgtcgcaga                                                              10

<210> 103
<211> 10
<212> DNA
<213> Homo sapiens

<400> 103
ggggctgccc                                                              10

<210> 104
<211> 10
<212> DNA
<213> Homo sapiens

<400> 104
ctgtacatac                                                              10

<210> 105
<211> 10
<212> DNA
<213> Homo sapiens

<400> 105
gcgacgaggc                                                              10

<210> 106
<211> 10
<212> DNA
<213> Homo sapiens

<400> 106
gccaagtgaa                                                              10

<210> 107
<211> 10
<212> DNA
<213> Homo sapiens

<400> 107
aagataaact                                                              10

<210> 108
<211> 10
<212> DNA
<213> Homo sapiens

<400> 108
gagagtgtac                                                              10

<210> 109
<211> 10
<212> DNA
<213> Homo sapiens

<400> 109
ccactgcact                                                              10

<210> 110

<211> 10
<212> DNA
<213> Homo sapiens

<400> 110
ccaccctcac                                                              10

<210> 111
<211> 10
<212> DNA
<213> Homo sapiens

<400> 111
cagaccattg                                                              10

<210> 112
<211> 10
<212> DNA
<213> Homo sapiens

<400> 112
gggagctgcg                                                              10

<210> 113
<211> 10
<212> DNA
<213> Homo sapiens

<400> 113
gggatttctg                                                              10

<210> 114
<211> 10
<212> DNA
<213> Homo sapiens

<400> 114
cttccagcta                                                              10

<210> 115
<211> 10
<212> DNA
<213> Homo sapiens

<400> 115
cagaaacaga                                                              10

<210> 116
<211> 10
<212> DNA
<213> Homo sapiens

<400> 116
tctgtgctca                                                              10

<210> 117
<211> 10
<212> DNA
<213> Homo sapiens

<400> 117
tgcaataggt                                                              10

<210> 118
<211> 10
<212> DNA
<213> Homo sapiens

<400> 118

atggccaact                                                              10

<210> 119
<211> 10
<212> DNA
<213> Homo sapiens

<400> 119
tcacacagtg                                                              10

<210> 120
<211> 10
<212> DNA
<213> Homo sapiens

<400> 120
ggcttaggat                                                              10

<210> 121
<211> 10
<212> DNA
<213> Homo sapiens

<400> 121
gggaggggtg                                                              10

<210> 122
<211> 10
<212> DNA
<213> Homo sapiens

<400> 122
gaagtagaag                                                              10

<210> 123
<211> 10
<212> DNA
<213> Homo sapiens

<400> 123
caccctgtac                                                              10

<210> 124
<211> 10
<212> DNA
<213> Homo sapiens

<400> 124
atgtttacaa                                                              10

<210> 125
<211> 10
<212> DNA
<213> Homo sapiens

<400> 125
caaactggtc                                                              10

<210> 126
<211> 10
<212> DNA
<213> Homo sapiens

<400> 126
gtaatgacag                                                              10

<210> 127
<211> 10
<212> DNA

<213> Homo sapiens

<400> 127
acctgccgac                                                              10

<210> 128
<211> 10
<212> DNA
<213> Homo sapiens

<400> 128
tgatgcgcgc                                                              10

<210> 129
<211> 10
<212> DNA
<213> Homo sapiens

<400> 129
tggccccagg                                                              10

<210> 130
<211> 10
<212> DNA
<213> Homo sapiens

<400> 130
gcctgctggg                                                              10

<210> 131
<211> 10
<212> DNA
<213> Homo sapiens

<400> 131
tgcctgtggt                                                              10

<210> 132
<211> 10
<212> DNA
<213> Homo sapiens

<400> 132
gagggtatac                                                              10

<210> 133
<211> 10
<212> DNA
<213> Homo sapiens

<400> 133
ggagccagct                                                              10

<210> 134
<211> 10
<212> DNA
<213> Homo sapiens

<400> 134
gagcctcagg                                                              10

<210> 135
<211> 10
<212> DNA
<213> Homo sapiens

<400> 135
tacttcacat                                                              10

<210> 136
<211> 10
<212> DNA
<213> Homo sapiens

<400> 136
taatcccagc                                                              10

<210> 137
<211> 10
<212> DNA
<213> Homo sapiens

<400> 137
caccttccag                                                              10

<210> 138
<211> 10
<212> DNA
<213> Homo sapiens

<400> 138
gagtctgttc                                                              10

<210> 139
<211> 10
<212> DNA
<213> Homo sapiens

<400> 139
ggattttggt                                                              10

<210> 140
<211> 10
<212> DNA
<213> Homo sapiens

<400> 140
tgcctgtagt                                                              10

<210> 141
<211> 10
<212> DNA
<213> Homo sapiens

<400> 141
ttacaaacag                                                              10

<210> 142
<211> 10
<212> DNA
<213> Homo sapiens

<400> 142
tcttctttca                                                              10

<210> 143
<211> 10
<212> DNA
<213> Homo sapiens

<400> 143
agcacatttg                                                              10

<210> 144
<211> 10
<212> DNA
<213> Homo sapiens

<400> 144
cagggctcgc                                                              10

<210> 145
<211> 10
<212> DNA
<213> Homo sapiens

<400> 145
gctggtccca                                                              10

<210> 146
<211> 10
<212> DNA
<213> Homo sapiens

<400> 146
tccacgccct                                                              10

<210> 147
<211> 10
<212> DNA
<213> Homo sapiens

<400> 147
ttgcaatagc                                                              10

<210> 148
<211> 10
<212> DNA
<213> Homo sapiens

<400> 148
agggcttcca                                                              10

<210> 149
<211> 10
<212> DNA
<213> Homo sapiens

<400> 149
ctgggttaat                                                              10

<210> 150
<211> 10
<212> DNA
<213> Homo sapiens

<400> 150
aacctgggag                                                              10

<210> 151
<211> 10
<212> DNA
<213> Homo sapiens

<400> 151
ggcaacgtgg                                                              10

<210> 152
<211> 10
<212> DNA
<213> Homo sapiens

<400> 152
ggatgcgcag                                                              10

<210> 153
<211> 10

<212> DNA
<213> Homo sapiens

<400> 153
cacctgtagt                                                              10

<210> 154
<211> 10
<212> DNA
<213> Homo sapiens

<400> 154
gtggtgggcg                                                              10

<210> 155
<211> 10
<212> DNA
<213> Homo sapiens

<400> 155
gcagggtggg                                                              10

<210> 156
<211> 10
<212> DNA
<213> Homo sapiens

<400> 156
caagcatccc                                                              10

<210> 157
<211> 10
<212> DNA
<213> Homo sapiens

<400> 157
tgggggccga                                                              10

<210> 158
<211> 10
<212> DNA
<213> Homo sapiens

<400> 158
tcagtgtatt                                                              10

<210> 159
<211> 10
<212> DNA
<213> Homo sapiens

<400> 159
ggcaagcccc                                                              10

<210> 160
<211> 10
<212> DNA
<213> Homo sapiens

<400> 160
cctagctgga                                                              10

<210> 161
<211> 10
<212> DNA
<213> Homo sapiens

<400> 161
gcaaaaccct                                                              10

<210> 162
<211> 10
<212> DNA
<213> Homo sapiens

<400> 162
gctggttcct                                                              10

<210> 163
<211> 10
<212> DNA
<213> Homo sapiens

<400> 163
gcacctcagc                                                              10

<210> 164
<211> 10
<212> DNA
<213> Homo sapiens

<400> 164
accagctgtc                                                              10

<210> 165
<211> 10
<212> DNA
<213> Homo sapiens

<400> 165
tttgaatcag                                                              10

<210> 166
<211> 10
<212> DNA
<213> Homo sapiens

<400> 166
agactagggg                                                              10

<210> 167
<211> 10
<212> DNA
<213> Homo sapiens

<400> 167
agctcagtga                                                              10

<210> 168
<211> 10
<212> DNA
<213> Homo sapiens

<400> 168
ggccaacatt                                                              10

<210> 169
<211> 10
<212> DNA
<213> Homo sapiens

<400> 169
tttgtgggca                                                              10

<210> 170
<211> 10
<212> DNA
<213> Homo sapiens

<400> 170
attgtagaca                                                              10

<210> 171
<211> 10
<212> DNA
<213> Homo sapiens

<400> 171
ccctaggttg                                                              10

<210> 172
<211> 10
<212> DNA
<213> Homo sapiens

<400> 172
aaatcaccaa                                                              10

<210> 173
<211> 10
<212> DNA
<213> Homo sapiens

<400> 173
ggctgcagtc                                                              10

<210> 174
<211> 10
<212> DNA
<213> Homo sapiens

<400> 174
gtggcaggcg                                                              10

<210> 175
<211> 10
<212> DNA
<213> Homo sapiens

<400> 175
taaaggcaca                                                              10

<210> 176
<211> 10
<212> DNA
<213> Homo sapiens

<400> 176
ggctcctggc                                                              10

<210> 177
<211> 10
<212> DNA
<213> Homo sapiens

<400> 177
atattaggaa                                                              10

<210> 178
<211> 10
<212> DNA
<213> Homo sapiens

<400> 178
gcttcagtgg                                                              10

<210> 179

<211> 10
<212> DNA
<213> Homo sapiens

<400> 179
tgattaaaac                                                              10

<210> 180
<211> 10
<212> DNA
<213> Homo sapiens

<400> 180
agccaccacg                                                              10

<210> 181
<211> 10
<212> DNA
<213> Homo sapiens

<400> 181
ggcggctgca                                                              10

<210> 182
<211> 10
<212> DNA
<213> Homo sapiens

<400> 182
tgtttggggg                                                              10

<210> 183
<211> 10
<212> DNA
<213> Homo sapiens

<400> 183
cctgcctcgt                                                              10

<210> 184
<211> 10
<212> DNA
<213> Homo sapiens

<400> 184
aggcctgggc                                                              10

<210> 185
<211> 10
<212> DNA
<213> Homo sapiens

<400> 185
caaaactgtt                                                              10

<210> 186
<211> 10
<212> DNA
<213> Homo sapiens

<400> 186
gagaggacat                                                              10

<210> 187
<211> 10
<212> DNA
<213> Homo sapiens

<400> 187

gagttaggca                                                              10

<210> 188
<211> 10
<212> DNA
<213> Homo sapiens

<400> 188
ccgtagtgcc                                                              10

<210> 189
<211> 10
<212> DNA
<213> Homo sapiens

<400> 189
cataaacggg                                                              10

<210> 190
<211> 10
<212> DNA
<213> Homo sapiens

<400> 190
tccctggcag                                                              10

<210> 191
<211> 10
<212> DNA
<213> Homo sapiens

<400> 191
gaggccatcc                                                              10

<210> 192
<211> 10
<212> DNA
<213> Homo sapiens

<400> 192
ttgcctggga                                                              10

<210> 193
<211> 10
<212> DNA
<213> Homo sapiens

<400> 193
ctgtcagcgg                                                              10

<210> 194
<211> 10
<212> DNA
<213> Homo sapiens

<400> 194
aacgcggcca                                                              10

<210> 195
<211> 10
<212> DNA
<213> Homo sapiens

<400> 195
ggtttggctt                                                              10

<210> 196
<211> 10
<212> DNA

<213> Homo sapiens

<400> 196
gatttttgtg                                                              10

<210> 197
<211> 10
<212> DNA
<213> Homo sapiens

<400> 197
ggctgccctg                                                              10

<210> 198
<211> 10
<212> DNA
<213> Homo sapiens

<400> 198
atggcaacag                                                              10

<210> 199
<211> 10
<212> DNA
<213> Homo sapiens

<400> 199
cgctgtgggg                                                              10

<210> 200
<211> 10
<212> DNA
<213> Homo sapiens

<400> 200
ggcagccaga                                                              10

<210> 201
<211> 10
<212> DNA
<213> Homo sapiens

<400> 201
agagcaaacc                                                              10

<210> 202
<211> 10
<212> DNA
<213> Homo sapiens

<400> 202
tttccctcaa                                                              10

<210> 203
<211> 10
<212> DNA
<213> Homo sapiens

<400> 203
tccccgtggc                                                              10

<210> 204
<211> 10
<212> DNA
<213> Homo sapiens

<400> 204
ttctcccaaa                                                              10

<210> 205
<211> 10
<212> DNA
<213> Homo sapiens

<400> 205
ggctgggggc                                                              10

<210> 206
<211> 10
<212> DNA
<213> Homo sapiens

<400> 206
ccctaccctg                                                              10

<210> 207
<211> 10
<212> DNA
<213> Homo sapiens

<400> 207
taggaccctg                                                              10

<210> 208
<211> 10
<212> DNA
<213> Homo sapiens

<400> 208
gtttttgctt                                                              10

<210> 209
<211> 10
<212> DNA
<213> Homo sapiens

<400> 209
cttgattccc                                                              10

<210> 210
<211> 10
<212> DNA
<213> Homo sapiens

<400> 210
gcttggctcc                                                              10

<210> 211
<211> 10
<212> DNA
<213> Homo sapiens

<400> 211
ggtggcactc                                                              10

<210> 212
<211> 10
<212> DNA
<213> Homo sapiens

<400> 212
acctgtgacc                                                              10

<210> 213
<211> 10
<212> DNA
<213> Homo sapiens

<400> 213
actgaggaaa                                                              10

<210> 214
<211> 10
<212> DNA
<213> Homo sapiens

<400> 214
tgcagcgcct                                                              10

<210> 215
<211> 10
<212> DNA
<213> Homo sapiens

<400> 215
   ctggggggaa                                                               10

<210> 216
<211> 10
<212> DNA
<213> Homo sapiens

<400> 216
gtgctattct                                                              10

<210> 217
<211> 10
<212> DNA
<213> Homo sapiens

<400> 217
ggagggggct                                                              10

<210> 218
<211> 10
<212> DNA
<213> Homo sapiens

<400> 218
gtgcctgaga                                                              10

<210> 219
<211> 10
<212> DNA
<213> Homo sapiens

<400> 219
tcacagggtc                                                              10

<210> 220
<211> 10
<212> DNA
<213> Homo sapiens

<400> 220
gggctccctg                                                              10

<210> 221
<211> 10
<212> DNA
<213> Homo sapiens

<400> 221
gccccaggta                                                              10

<210> 222
<211> 10

<212> DNA
<213> Homo sapiens

<400> 222
gaaagtggct                                                              10

<210> 223
<211> 10
<212> DNA
<213> Homo sapiens

<400> 223
tccctggctg                                                              10

<210> 224
<211> 10
<212> DNA
<213> Homo sapiens

<400> 224
acagagcaca                                                              10

<210> 225
<211> 10
<212> DNA
<213> Homo sapiens

<400> 225
ctttgcactc                                                              10

<210> 226
<211> 10
<212> DNA
<213> Homo sapiens

<400> 226
atgctccctg                                                              10

<210> 227
<211> 10
<212> DNA
<213> Homo sapiens

<400> 227
ccgtccaagg                                                              10

<210> 228
<211> 10
<212> DNA
<213> Homo sapiens

<400> 228
gggcccctg                                                               10

<210> 229
<211> 10
<212> DNA
<213> Homo sapiens

<400> 229
cttatgctgc                                                              10

<210> 230
<211> 10
<212> DNA
<213> Homo sapiens

<400> 230
ggttattttg                                                              10

<210> 231
<211> 10
<212> DNA
<213> Homo sapiens

<400> 231
gcctgtccct                                                              10

<210> 232
<211> 10
<212> DNA
<213> Homo sapiens

<400> 232
aagatgaggg                                                              10

<210> 233
<211> 10
<212> DNA
<213> Homo sapiens

<400> 233
ccaacaagaa                                                              10

<210> 234
<211> 10
<212> DNA
<213> Homo sapiens

<400> 234
aaggatgcgg                                                              10

<210> 235
<211> 10
<212> DNA
<213> Homo sapiens

<400> 235
tgtcatcaca                                                              10

<210> 236
<211> 10
<212> DNA
<213> Homo sapiens

<400> 236
caggcttttt                                                              10

<210> 237
<211> 10
<212> DNA
<213> Homo sapiens

<400> 237
tcaagttcac                                                              10

<210> 238
<211> 10
<212> DNA
<213> Homo sapiens

<400> 238
tccctgggca                                                              10

<210> 239
<211> 10
<212> DNA
<213> Homo sapiens

<400> 239
caggagttca                                                              10

<210> 240
<211> 10
<212> DNA
<213> Homo sapiens

<400> 240
caggtggttc                                                              10

<210> 241
<211> 10
<212> DNA
<213> Homo sapiens

<400> 241
gcccacatcc                                                              10

<210> 242
<211> 10
<212> DNA
<213> Homo sapiens

<400> 242
gctggggtgg                                                              10

<210> 243
<211> 10
<212> DNA
<213> Homo sapiens

<400> 243
gacctcctgc                                                              10

<210> 244
<211> 10
<212> DNA
<213> Homo sapiens

<400> 244
agtgaataaa                                                              10

<210> 245
<211> 10
<212> DNA
<213> Homo sapiens

<400> 245
aaggttcttc                                                              10

<210> 246
<211> 10
<212> DNA
<213> Homo sapiens

<400> 246
agcctggact                                                              10

<210> 247
<211> 10
<212> DNA
<213> Homo sapiens

<400> 247
caacccagat                                                              10

<210> 248

<211> 10
<212> DNA
<213> Homo sapiens

<400> 248
tgcttctgcc                                                              10

<210> 249
<211> 10
<212> DNA
<213> Homo sapiens

<400> 249
caggtgacaa                                                              10

<210> 250
<211> 10
<212> DNA
<213> Homo sapiens

<400> 250
ggccgggggc                                                              10

<210> 251
<211> 10
<212> DNA
<213> Homo sapiens

<400> 251
gtgcgctagg                                                              10

<210> 252
<211> 10
<212> DNA
<213> Homo sapiens

<400> 252
aggctgtcca                                                              10

<210> 253
<211> 10
<212> DNA
<213> Homo sapiens

<400> 253
tgttatgtcc                                                              10

<210> 254
<211> 10
<212> DNA
<213> Homo sapiens

<400> 254
tttcccaaac                                                              10

<210> 255
<211> 10
<212> DNA
<213> Homo sapiens

<400> 255
ggggatgggg                                                              10

<210> 256
<211> 17
<212> DNA
<213> Homo sapiens

<400> 256

aaaccattct cctccgc                                                    17

<210> 257
<211> 17
<212> DNA
<213> Homo sapiens

<400> 257
aaggcaggga gggaggg                                                    17

<210> 258
<211> 17
<212> DNA
<213> Homo sapiens

<400> 258
acacagcaag acgagaa                                                    17

<210> 259
<211> 17
<212> DNA
<213> Homo sapiens

<400> 259
agctggagtc ctaggca                                                    17

<210> 260
<211> 17
<212> DNA
<213> Homo sapiens

<400> 260
agctggcacc agagccc                                                    17

<210> 261
<211> 17
<212> DNA
<213> Homo sapiens

<400> 261
ataaatgagg taaggtc                                                    17

<210> 262
<211> 17
<212> DNA
<213> Homo sapiens

<400> 262
caagcacccc cgttcca                                                    17

<210> 263
<211> 17
<212> DNA
<213> Homo sapiens

<400> 263
cactacccac cagacgc                                                    17

<210> 264
<211> 17
<212> DNA
<213> Homo sapiens

<400> 264
cactactcac caggcgc                                                    17

<210> 265
<211> 17
<212> DNA

<213> Homo sapiens

<400> 265
cccacctcca gtccagc                                                    17

<210> 266
<211> 17
<212> DNA
<213> Homo sapiens

<400> 266
cccgcctctt cacgggc                                                    17

<210> 267
<211> 17
<212> DNA
<213> Homo sapiens

<400> 267
cctcagatgt ttgaaaa                                                    17

<210> 268
<211> 17
<212> DNA
<213> Homo sapiens

<400> 268
cgctactcac cagacgc                                                    17

<210> 269
<211> 17
<212> DNA
<213> Homo sapiens

<400> 269
ctaagacctc accagtc                                                    17

<210> 270
<211> 17
<212> DNA
<213> Homo sapiens

<400> 270
ctaagacttc accggtc                                                    17

<210> 271
<211> 17
<212> DNA
<213> Homo sapiens

<400> 271
gagtgggtgc agcctcc                                                    17

<210> 272
<211> 17
<212> DNA
<213> Homo sapiens

<400> 272
gggacagctg tctgtgg                                                    17

<210> 273
<211> 17
<212> DNA
<213> Homo sapiens

<400> 273
gggttggctt gaaacca                                                    17

<210> 274
<211> 17
<212> DNA
<213> Homo sapiens

<400> 274
gtaagtgtac tggaagt                                                      17

<210> 275
<211> 17
<212> DNA
<213> Homo sapiens

<400> 275
gtaagtgtac tggtaag                                                      17

<210> 276
<211> 17
<212> DNA
<213> Homo sapiens

<400> 276
gtaggggtaa aaggagg                                                      17

<210> 277
<211> 17
<212> DNA
<213> Homo sapiens

<400> 277
taaccactgc actttcc                                                      17

<210> 278
<211> 17
<212> DNA
<213> Homo sapiens

<400> 278
tactgctcgg aggtcgg                                                      17

<210> 279
<211> 17
<212> DNA
<213> Homo sapiens

<400> 279
tcaggctgaa gtcaggc                                                      17

<210> 280
<211> 17
<212> DNA
<213> Homo sapiens

<400> 280
tccatacacc tatcccc                                                      17

<210> 281
<211> 17
<212> DNA
<213> Homo sapiens

<400> 281
   tccttttaaa acaaaac                                                      17

<210> 282
<211> 17
<212> DNA
<213> Homo sapiens

<400> 282
tgattaaggt cggcgct                                                    17

<210> 283
<211> 17
<212> DNA
<213> Homo sapiens

<400> 283
tggtatcaca caagggg                                                    17

<210> 284
<211> 17
<212> DNA
<213> Homo sapiens

<400> 284
tggtgtatgc atcgggg                                                    17

<210> 285
<211> 17
<212> DNA
<213> Homo sapiens

<400> 285
tgtcactggg caggcgg                                                    17

<210> 286
<211> 17
<212> DNA
<213> Homo sapiens

<400> 286
tgtgggaggc tgatggg                                                    17

<210> 287
<211> 17
<212> DNA
<213> Homo sapiens

<400> 287
tttaacggcc gcggtac                                                    17

<210> 288
<211> 17
<212> DNA
<213> Homo sapiens

<400> 288
gctctctatg ctgacgt                                                    17

<210> 289
<211> 17
<212> DNA
<213> Homo sapiens

<400> 289
agaatgaaac tgccggg                                                    17

<210> 290
<211> 17
<212> DNA
<213> Homo sapiens

<400> 290
aagtggaata aactgcc                                                    17

<210> 291
<211> 17

<212> DNA
<213> Homo sapiens

<400> 291
gatgacgact cggggct                                                      17

<210> 292
<211> 17
<212> DNA
<213> Homo sapiens

<400> 292
ccctttcaca cacactt                                                      17

<210> 293
<211> 17
<212> DNA
<213> Homo sapiens

<400> 293
tcctggggca ggggcgg                                                      17

<210> 294
<211> 17
<212> DNA
<213> Homo sapiens

<400> 294
tctattgatg tgtatgc                                                      17

<210> 295
<211> 17
<212> DNA
<213> Homo sapiens

<400> 295
ggggctgtat ttaagga                                                      17

<210> 296
<211> 17
<212> DNA
<213> Homo sapiens

<400> 296
cccaggacac cagctgg                                                      17

<210> 297
<211> 17
<212> DNA
<213> Homo sapiens

<400> 297
ggagctgctg cttgtgg                                                      17

<210> 298
<211> 17
<212> DNA
<213> Homo sapiens

<400> 298
tggacagcag ggacctg                                                      17

<210> 299
<211> 17
<212> DNA
<213> Homo sapiens

<400> 299
tctgggaaca gggacgg                                                      17

<210> 300
<211> 17
<212> DNA
<213> Homo sapiens

<400> 300
cctgtgtatg tgtgtaa                                                      17

<210> 301
<211> 17
<212> DNA
<213> Homo sapiens

<400> 301
ggcaagaaga agatcgc                                                      17

<210> 302
<211> 17
<212> DNA
<213> Homo sapiens

<400> 302
aaatgcttgg aggtgaa                                                      17

<210> 303
<211> 17
<212> DNA
<213> Homo sapiens

<400> 303
ctaaaaacct tatgaca                                                      17

<210> 304
<211> 17
<212> DNA
<213> Homo sapiens

<400> 304
gagcattgca ccacccg                                                      17

<210> 305
<211> 17
<212> DNA
<213> Homo sapiens

<400> 305
ggtggacacg gatctgc                                                      17

<210> 306
<211> 17
<212> DNA
<213> Homo sapiens

<400> 306
gctcctgagc cccggcc                                                      17

<210> 307
<211> 17
<212> DNA
<213> Homo sapiens

<400> 307
aagaagtgga gattgtc                                                      17

<210> 308
<211> 17
<212> DNA
<213> Homo sapiens

<400> 308
tgggaagtgg gctcctt                                                    17

<210> 309
<211> 17
<212> DNA
<213> Homo sapiens

<400> 309
actcgctctg tggaggt                                                    17

<210> 310
<211> 17
<212> DNA
<213> Homo sapiens

<400> 310
tttcagggga gggggaa                                                    17

<210> 311
<211> 17
<212> DNA
<213> Homo sapiens

<400> 311
acaacgtcca gctggtg                                                    17

<210> 312
<211> 17
<212> DNA
<213> Homo sapiens

<400> 312
gtctcagtgc tgaggcg                                                    17

<210> 313
<211> 17
<212> DNA
<213> Homo sapiens

<400> 313
ccccctgccc ctctgcc                                                    17

<210> 314
<211> 17
<212> DNA
<213> Homo sapiens

<400> 314
agaaaccacg gaaatgg                                                    17

<210> 315
<211> 17
<212> DNA
<213> Homo sapiens

<400> 315
gaccgcagga gggcaga                                                    17

<210> 316
<211> 17
<212> DNA
<213> Homo sapiens

<400> 316
gtgctacttc ttcttct                                                    17

<210> 317

<211> 17
<212> DNA
<213> Homo sapiens

<400> 317
gataactaca ttacctg                                                      17

<210> 318
<211> 17
<212> DNA
<213> Homo sapiens

<400> 318
tggctgtgac tgtgact                                                      17

<210> 319
<211> 17
<212> DNA
<213> Homo sapiens

<400> 319
gagtgagacc caggagc                                                      17

<210> 320
<211> 17
<212> DNA
<213> Homo sapiens

<400> 320
gagtggctac ccgccgc                                                      17

<210> 321
<211> 17
<212> DNA
<213> Homo sapiens

<400> 321
gactcaggga tttgttg                                                      17

<210> 322
<211> 17
<212> DNA
<213> Homo sapiens

<400> 322
gttatatgcc cgggaga                                                      17

<210> 323
<211> 17
<212> DNA
<213> Homo sapiens

<400> 323
gaggcgctgc tgccacc                                                      17

<210> 324
<211> 17
<212> DNA
<213> Homo sapiens

<400> 324
gagctctgag atcaccc                                                      17

<210> 325
<211> 17
<212> DNA
<213> Homo sapiens

<400> 325

gccagccagt ggcaagc                                                    17

<210> 326
<211> 17
<212> DNA
<213> Homo sapiens

<400> 326
atggcaacag atctgga                                                    17

<210> 327
<211> 17
<212> DNA
<213> Homo sapiens

<400> 327
aaggagttac actagtc                                                    17

<210> 328
<211> 17
<212> DNA
<213> Homo sapiens

<400> 328
tcccacaagg ctgcttg                                                    17

<210> 329
<211> 17
<212> DNA
<213> Homo sapiens

<400> 329
taaatcccca ctgggac                                                    17

<210> 330
<211> 17
<212> DNA
<213> Homo sapiens

<400> 330
cccgcccccg ccttccc                                                    17

<210> 331
<211> 17
<212> DNA
<213> Homo sapiens

<400> 331
cccgaggcag agtcggg                                                    17

<210> 332
<211> 17
<212> DNA
<213> Homo sapiens

<400> 332
ctacgtgatg aagatgg                                                    17

<210> 333
<211> 17
<212> DNA
<213> Homo sapiens

<400> 333
atgggtttgc attttag                                                    17

<210> 334
<211> 17
<212> DNA

<213> Homo sapiens

<400> 334
ggcattgtct ctgtttc                                                    17

<210> 335
<211> 17
<212> DNA
<213> Homo sapiens

<400> 335
gtgctaagcg ggcccgg                                                    17

<210> 336
<211> 17
<212> DNA
<213> Homo sapiens

<400> 336
accgtttgca ttcgaaa                                                    17

<210> 337
<211> 17
<212> DNA
<213> Homo sapiens

<400> 337
cagcgctgca ttgactc                                                    17

<210> 338
<211> 17
<212> DNA
<213> Homo sapiens

<400> 338
gaagacactt ggtttga                                                    17

<210> 339
<211> 17
<212> DNA
<213> Homo sapiens

<400> 339
cgctgggcgt ctgggac                                                    17

<210> 340
<211> 17
<212> DNA
<213> Homo sapiens

<400> 340
cacccctgat gttcgcc                                                    17

<210> 341
<211> 17
<212> DNA
<213> Homo sapiens

<400> 341
gcccccctgc cccgtgc                                                    17

<210> 342
<211> 17
<212> DNA
<213> Homo sapiens

<400> 342
ccccctgccc tcgcctg                                                    17

<210> 343
<211> 17
<212> DNA
<213> Homo sapiens

<400> 343
agcataaaaa tgcgtgc                                                      17

<210> 344
<211> 17
<212> DNA
<213> Homo sapiens

<400> 344
gggctggacg gctgcgt                                                      17

<210> 345
<211> 17
<212> DNA
<213> Homo sapiens

<400> 345
ctgccaactt ctaaccg                                                      17

<210> 346
<211> 17
<212> DNA
<213> Homo sapiens

<400> 346
aagtggatag atacttc                                                      17

<210> 347
<211> 17
<212> DNA
<213> Homo sapiens

<400> 347
cgtactgagc gctttgg                                                      17

<210> 348
<211> 17
<212> DNA
<213> Homo sapiens

<400> 348
ccgcttactc tgttggg                                                      17

<210> 349
<211> 17
<212> DNA
<213> Homo sapiens

<400> 349
ggggcttctg tagcccc                                                      17

<210> 350
<211> 17
<212> DNA
<213> Homo sapiens

<400> 350
cccgtccgga acgtcta                                                      17

<210> 351
<211> 17
<212> DNA
<213> Homo sapiens

<400> 351
agttccacca gaaagcc                                                    17

<210> 352
<211> 17
<212> DNA
<213> Homo sapiens

<400> 352
ggcctccagc cacccac                                                    17

<210> 353
<211> 17
<212> DNA
<213> Homo sapiens

<400> 353
ggaggctgag gtgggag                                                    17

<210> 354
<211> 17
<212> DNA
<213> Homo sapiens

<400> 354
cagaggcgtc cgcaggt                                                    17

<210> 355
<211> 17
<212> DNA
<213> Homo sapiens

<400> 355
gaccagcctt cagatgg                                                    17

<210> 356
<211> 17
<212> DNA
<213> Homo sapiens

<400> 356
gaggatggtg tcctgag                                                    17

<210> 357
<211> 17
<212> DNA
<213> Homo sapiens

<400> 357
tcgtcgcaga aggcgct                                                    17

<210> 358
<211> 17
<212> DNA
<213> Homo sapiens

<400> 358
ggggctgccc agctgga                                                    17

<210> 359
<211> 17
<212> DNA
<213> Homo sapiens

<400> 359
ctgtacatac tttttgg                                                    17

<210> 360
<211> 17

<212> DNA
<213> Homo sapiens

<400> 360
gcgacgaggc gcgctgg                                                    17

<210> 361
<211> 17
<212> DNA
<213> Homo sapiens

<400> 361
gccaagtgaa ctgtggc                                                    17

<210> 362
<211> 17
<212> DNA
<213> Homo sapiens

<400> 362
aagataaact ctgggcc                                                    17

<210> 363
<211> 17
<212> DNA
<213> Homo sapiens

<400> 363
gagagtgtac tggcact                                                    17

<210> 364
<211> 17
<212> DNA
<213> Homo sapiens

<400> 364
ccactgcact ccggcct                                                    17

<210> 365
<211> 17
<212> DNA
<213> Homo sapiens

<400> 365
ccaccctcac acacaca                                                    17

<210> 366
<211> 17
<212> DNA
<213> Homo sapiens

<400> 366
cagaccattg tttgatc                                                    17

<210> 367
<211> 17
<212> DNA
<213> Homo sapiens

<400> 367
gggagctgcg ccaacgg                                                    17

<210> 368
<211> 17
<212> DNA
<213> Homo sapiens

<400> 368
gggatttctg tgtctgc                                                    17

<210> 369
<211> 17
<212> DNA
<213> Homo sapiens

<400> 369
cttccagcta acaggtc                                                      17

<210> 370
<211> 17
<212> DNA
<213> Homo sapiens

<400> 370
cagaaacaga ctggggg                                                      17

<210> 371
<211> 17
<212> DNA
<213> Homo sapiens

<400> 371
tctgtgctca ggaagag                                                      17

<210> 372
<211> 17
<212> DNA
<213> Homo sapiens

<400> 372
tgcaataggt gagagaa                                                      17

<210> 373
<211> 17
<212> DNA
<213> Homo sapiens

<400> 373
atggccaact tccacct                                                      17

<210> 374
<211> 17
<212> DNA
<213> Homo sapiens

<400> 374
tcacacagtg cctgtcg                                                      17

<210> 375
<211> 17
<212> DNA
<213> Homo sapiens

<400> 375
ggcttaggat gtgaatg                                                      17

<210> 376
<211> 17
<212> DNA
<213> Homo sapiens

<400> 376
gggaggggtg ggggtgg                                                      17

<210> 377
<211> 17
<212> DNA
<213> Homo sapiens

<400> 377
gaagtagaag gtaagga                                                    17

<210> 378
<211> 17
<212> DNA
<213> Homo sapiens

<400> 378
caccctgtac agttgcc                                                    17

<210> 379
<211> 17
<212> DNA
<213> Homo sapiens

<400> 379
atgtttacaa gatggcg                                                    17

<210> 380
<211> 17
<212> DNA
<213> Homo sapiens

<400> 380
caaactggtc taggtca                                                    17

<210> 381
<211> 17
<212> DNA
<213> Homo sapiens

<400> 381
gtaatgacag atgcaag                                                    17

<210> 382
<211> 17
<212> DNA
<213> Homo sapiens

<400> 382
acctgccgac agtgttg                                                    17

<210> 383
<211> 17
<212> DNA
<213> Homo sapiens

<400> 383
tgatgcgcgc tttgttg                                                    17

<210> 384
<211> 17
<212> DNA
<213> Homo sapiens

<400> 384
tggccccagg tgccacc                                                    17

<210> 385
<211> 17
<212> DNA
<213> Homo sapiens

<400> 385
gcctgctggg cttggct                                                    17

<210> 386

<211> 17
<212> DNA
<213> Homo sapiens

<400> 386
tgcctgtggt cccagct                                                      17

<210> 387
<211> 17
<212> DNA
<213> Homo sapiens

<400> 387
gagggtatac tgagggg                                                      17

<210> 388
<211> 17
<212> DNA
<213> Homo sapiens

<400> 388
ggagccagct gacctgc                                                      17

<210> 389
<211> 17
<212> DNA
<213> Homo sapiens

<400> 389
gagcctcagg tgctccc                                                      17

<210> 390
<211> 17
<212> DNA
<213> Homo sapiens

<400> 390
tacttcacat acagtgc                                                      17

<210> 391
<211> 17
<212> DNA
<213> Homo sapiens

<400> 391
taatcccagc actttgg                                                      17

<210> 392
<211> 17
<212> DNA
<213> Homo sapiens

<400> 392
caccttccag cccgggg                                                      17

<210> 393
<211> 17
<212> DNA
<213> Homo sapiens

<400> 393
gagtctgttc gtgactc                                                      17

<210> 394
<211> 17
<212> DNA
<213> Homo sapiens

<400> 394

ggatttggt ctctgtc                                                    17

<210> 395
<211> 17
<212> DNA
<213> Homo sapiens

<400> 395
tgcctgtagt cctagtt                                                   17

<210> 396
<211> 17
<212> DNA
<213> Homo sapiens

<400> 396
ttacaaacag aaaagct                                                   17

<210> 397
<211> 17
<212> DNA
<213> Homo sapiens

<400> 397
tcttctttca gaatggg                                                   17

<210> 398
<211> 17
<212> DNA
<213> Homo sapiens

<400> 398
agcacatttg atatagc                                                   17

<210> 399
<211> 17
<212> DNA
<213> Homo sapiens

<400> 399
cagggctcgc gtgcggg                                                   17

<210> 400
<211> 17
<212> DNA
<213> Homo sapiens

<400> 400
gctggtccca gggccag                                                   17

<210> 401
<211> 17
<212> DNA
<213> Homo sapiens

<400> 401
tccacgccct tcctggc                                                   17

<210> 402
<211> 17
<212> DNA
<213> Homo sapiens

<400> 402
ttgcaatagc aaaaccc                                                   17

<210> 403
<211> 17
<212> DNA

<213> Homo sapiens

<400> 403
agggcttcca atgtgct                                                    17

<210> 404
<211> 17
<212> DNA
<213> Homo sapiens

<400> 404
ctgggttaat aaattgc                                                    17

<210> 405
<211> 17
<212> DNA
<213> Homo sapiens

<400> 405
aacctgggag gtggagg                                                    17

<210> 406
<211> 17
<212> DNA
<213> Homo sapiens

<400> 406
ggcaacgtgg tagaggc                                                    17

<210> 407
<211> 17
<212> DNA
<213> Homo sapiens

<400> 407
ggatgcgcag gggaggc                                                    17

<210> 408
<211> 17
<212> DNA
<213> Homo sapiens

<400> 408
cacctgtagt cctagct                                                    17

<210> 409
<211> 17
<212> DNA
<213> Homo sapiens

<400> 409
gtggtgggcg cctgtag                                                    17

<210> 410
<211> 17
<212> DNA
<213> Homo sapiens

<400> 410
gcagggtggg gaggggt                                                    17

<210> 411
<211> 17
<212> DNA
<213> Homo sapiens

<400> 411
caagcatccc cgttcca                                                    17

<210> 412
<211> 17
<212> DNA
<213> Homo sapiens

<400> 412
tgggggccga tgggcag                                                      17

<210> 413
<211> 17
<212> DNA
<213> Homo sapiens

<400> 413
tcagtgtatt aaaaccc                                                      17

<210> 414
<211> 17
<212> DNA
<213> Homo sapiens

<400> 414
ggcaagcccc agcgcct                                                      17

<210> 415
<211> 17
<212> DNA
<213> Homo sapiens

<400> 415
cctagctgga ttgcaga                                                      17

<210> 416
<211> 17
<212> DNA
<213> Homo sapiens

<400> 416
gcaaaaccct gctctcc                                                      17

<210> 417
<211> 17
<212> DNA
<213> Homo sapiens

<400> 417
gctggttcct gagtggc                                                      17

<210> 418
<211> 17
<212> DNA
<213> Homo sapiens

<400> 418
gcacctcagc caggggt                                                      17

<210> 419
<211> 17
<212> DNA
<213> Homo sapiens

<400> 419
accagctgtc caggggc                                                      17

<210> 420
<211> 17
<212> DNA
<213> Homo sapiens

<400> 420
tttgaatcag tgctaga                                                    17

<210> 421
<211> 17
<212> DNA
<213> Homo sapiens

<400> 421
agactagggg ccggagc                                                    17

<210> 422
<211> 17
<212> DNA
<213> Homo sapiens

<400> 422
agctcagtga gaagggc                                                    17

<210> 423
<211> 17
<212> DNA
<213> Homo sapiens

<400> 423
ggccaacatt tggtcca                                                    17

<210> 424
<211> 17
<212> DNA
<213> Homo sapiens

<400> 424
tttgtgggca gtcaggc                                                    17

<210> 425
<211> 17
<212> DNA
<213> Homo sapiens

<400> 425
attgtagaca atgaggg                                                    17

<210> 426
<211> 17
<212> DNA
<213> Homo sapiens

<400> 426
ccctaggttg ggcccct                                                    17

<210> 427
<211> 17
<212> DNA
<213> Homo sapiens

<400> 427
aaatcaccaa tcaaggc                                                    17

<210> 428
<211> 17
<212> DNA
<213> Homo sapiens

<400> 428
ggctgcagtc ttcttcc                                                    17

<210> 429
<211> 17

<212> DNA
<213> Homo sapiens

<400> 429
gtggcaggcg cctgtag                                                      17

<210> 430
<211> 17
<212> DNA
<213> Homo sapiens

<400> 430
taaaggcaca gtggctc                                                      17

<210> 431
<211> 17
<212> DNA
<213> Homo sapiens

<400> 431
ggctcctggc tctggac                                                      17

<210> 432
<211> 17
<212> DNA
<213> Homo sapiens

<400> 432
atattaggaa gtcgggg                                                      17

<210> 433
<211> 17
<212> DNA
<213> Homo sapiens

<400> 433
gcttcagtgg gggagag                                                      17

<210> 434
<211> 17
<212> DNA
<213> Homo sapiens

<400> 434
tgattaaaac aagttgc                                                      17

<210> 435
<211> 17
<212> DNA
<213> Homo sapiens

<400> 435
agccaccacg cctggtc                                                      17

<210> 436
<211> 17
<212> DNA
<213> Homo sapiens

<400> 436
ggcggctgca gagcctg                                                      17

<210> 437
<211> 17
<212> DNA
<213> Homo sapiens

<400> 437
tgtttggggg cttttag                                                      17

<210> 438
<211> 17
<212> DNA
<213> Homo sapiens

<400> 438
cctgcctcgt agtgaag                                                    17

<210> 439
<211> 17
<212> DNA
<213> Homo sapiens

<400> 439
aggcctgggc ctctgcg                                                    17

<210> 440
<211> 17
<212> DNA
<213> Homo sapiens

<400> 440
caaaactgtt tgttggc                                                    17

<210> 441
<211> 17
<212> DNA
<213> Homo sapiens

<400> 441
gagaggacat tggaggg                                                    17

<210> 442
<211> 17
<212> DNA
<213> Homo sapiens

<400> 442
gagttaggca cttcctg                                                    17

<210> 443
<211> 17
<212> DNA
<213> Homo sapiens

<400> 443
ccgtagtgcc tttatgg                                                    17

<210> 444
<211> 17
<212> DNA
<213> Homo sapiens

<400> 444
cataaacggg cacaccc                                                    17

<210> 445
<211> 17
<212> DNA
<213> Homo sapiens

<400> 445
tccctggcag agggctt                                                    17

<210> 446
<211> 17
<212> DNA
<213> Homo sapiens

<400> 446
gaggccatcc ccaaccc                                                      17

<210> 447
<211> 17
<212> DNA
<213> Homo sapiens

<400> 447
ttgcctggga tgctggt                                                      17

<210> 448
<211> 17
<212> DNA
<213> Homo sapiens

<400> 448
ctgtcagcgg ctgcccc                                                      17

<210> 449
<211> 17
<212> DNA
<213> Homo sapiens

<400> 449
aacgcggcca atgtggg                                                      17

<210> 450
<211> 17
<212> DNA
<213> Homo sapiens

<400> 450
ggtttggctt aggctgg                                                      17

<210> 451
<211> 17
<212> DNA
<213> Homo sapiens

<400> 451
gatttttgtg gtgtggg                                                      17

<210> 452
<211> 17
<212> DNA
<213> Homo sapiens

<400> 452
ggctgccctg ggcagcc                                                      17

<210> 453
<211> 17
<212> DNA
<213> Homo sapiens

<400> 453
atggcaacag aaaccaa                                                      17

<210> 454
<211> 17
<212> DNA
<213> Homo sapiens

<400> 454
cgctgtgggg tgcagac                                                      17

<210> 455

<211> 17
<212> DNA
<213> Homo sapiens

<400> 455
ggcagccaga gctccaa                                                      17

<210> 456
<211> 17
<212> DNA
<213> Homo sapiens

<400> 456
agagcaaacc gtagtcc                                                      17

<210> 457
<211> 17
<212> DNA
<213> Homo sapiens

<400> 457
tttccctcaa agactct                                                      17

<210> 458
<211> 17
<212> DNA
<213> Homo sapiens

<400> 458
tccccgtggc tgtgggg                                                      17

<210> 459
<211> 17
<212> DNA
<213> Homo sapiens

<400> 459
ttctcccaaa taccgtt                                                      17

<210> 460
<211> 17
<212> DNA
<213> Homo sapiens

<400> 460
ggctgggggc cagggct                                                      17

<210> 461
<211> 17
<212> DNA
<213> Homo sapiens

<400> 461
ccctaccctg ttacctt                                                      17

<210> 462
<211> 17
<212> DNA
<213> Homo sapiens

<400> 462
taggaccctg caggggg                                                      17

<210> 463
<211> 17
<212> DNA
<213> Homo sapiens

<400> 463

gtttttgctt cagcggc                                                    17

<210> 464
<211> 17
<212> DNA
<213> Homo sapiens

<400> 464
cttgattccc acgctac                                                    17

<210> 465
<211> 17
<212> DNA
<213> Homo sapiens

<400> 465
gcttggctcc caaaggg                                                    17

<210> 466
<211> 17
<212> DNA
<213> Homo sapiens

<400> 466
ggtggcactc agtctct                                                    17

<210> 467
<211> 17
<212> DNA
<213> Homo sapiens

<400> 467
acctgtgacc agcactg                                                    17

<210> 468
<211> 17
<212> DNA
<213> Homo sapiens

<400> 468
actgaggaaa ggagctc                                                    17

<210> 469
<211> 17
<212> DNA
<213> Homo sapiens

<400> 469
tgcagcgcct gcggcct                                                    17

<210> 470
<211> 17
<212> DNA
<213> Homo sapiens

<400> 470
ctgggggaa gggactg                                                     17

<210> 471
<211> 17
<212> DNA
<213> Homo sapiens

<400> 471
gtgctattct ggggctg                                                    17

<210> 472
<211> 17
<212> DNA

<213> Homo sapiens

<400> 472
ggagggggct tgaagcc                                                      17

<210> 473
<211> 17
<212> DNA
<213> Homo sapiens

<400> 473
gtgcctgaga ggcaggc                                                      17

<210> 474
<211> 17
<212> DNA
<213> Homo sapiens

<400> 474
tcacagggtc cccgggg                                                      17

<210> 475
<211> 17
<212> DNA
<213> Homo sapiens

<400> 475
gggctccctg gccctgg                                                      17

<210> 476
<211> 17
<212> DNA
<213> Homo sapiens

<400> 476
gccccaggta gggggac                                                      17

<210> 477
<211> 17
<212> DNA
<213> Homo sapiens

<400> 477
gaaagtggct gtcctgg                                                      17

<210> 478
<211> 17
<212> DNA
<213> Homo sapiens

<400> 478
tccctggctg ttgaggc                                                      17

<210> 479
<211> 17
<212> DNA
<213> Homo sapiens

<400> 479
acagagcaca gctgccc                                                      17

<210> 480
<211> 17
<212> DNA
<213> Homo sapiens

<400> 480
ctttgcactc tcctttg                                                      17

<210> 481
<211> 17
<212> DNA
<213> Homo sapiens

<400> 481
atgctccctg aggagct                                                      17

<210> 482
<211> 17
<212> DNA
<213> Homo sapiens

<400> 482
ccgtccaagg gtccgct                                                      17

<210> 483
<211> 17
<212> DNA
<213> Homo sapiens

<400> 483
   gggccccctg ggcagtg                                                       17

<210> 484
<211> 17
<212> DNA
<213> Homo sapiens

<400> 484
cttatgctgc tggtgcc                                                      17

<210> 485
<211> 17
<212> DNA
<213> Homo sapiens

<400> 485
ggttattttg gagtgta                                                      17

<210> 486
<211> 17
<212> DNA
<213> Homo sapiens

<400> 486
gcctgtccct ccaagac                                                      17

<210> 487
<211> 17
<212> DNA
<213> Homo sapiens

<400> 487
aagatgaggg ggcaggc                                                      17

<210> 488
<211> 17
<212> DNA
<213> Homo sapiens

<400> 488
ccaacaagaa tgcattg                                                      17

<210> 489
<211> 17
<212> DNA
<213> Homo sapiens

<400> 489
aaggatgcgg tgatggc                                                    17

<210> 490
<211> 17
<212> DNA
<213> Homo sapiens

<400> 490
tgtcatcaca gacactt                                                    17

<210> 491
<211> 17
<212> DNA
<213> Homo sapiens

<400> 491
caggcttttt ggcttcc                                                    17

<210> 492
<211> 17
<212> DNA
<213> Homo sapiens

<400> 492
tcaagttcac tgcctgt                                                    17

<210> 493
<211> 17
<212> DNA
<213> Homo sapiens

<400> 493
tccctgggca gcttcag                                                    17

<210> 494
<211> 17
<212> DNA
<213> Homo sapiens

<400> 494
caggagttca aagaagg                                                    17

<210> 495
<211> 17
<212> DNA
<213> Homo sapiens

<400> 495
caggtggttc tgccatc                                                    17

<210> 496
<211> 17
<212> DNA
<213> Homo sapiens

<400> 496
gcccacatcc gctgagg                                                    17

<210> 497
<211> 17
<212> DNA
<213> Homo sapiens

<400> 497
gctggggtgg gggtggg                                                    17

<210> 498
<211> 17

<212> DNA
<213> Homo sapiens

<400> 498
gacctcctgc cctgggg                                                      17

<210> 499
<211> 17
<212> DNA
<213> Homo sapiens

<400> 499
agtgaataaa tgtcttg                                                      17

<210> 500
<211> 17
<212> DNA
<213> Homo sapiens

<400> 500
aaggttcttc tcaaggg                                                      17

<210> 501
<211> 17
<212> DNA
<213> Homo sapiens

<400> 501
agcctggact gagccac                                                      17

<210> 502
<211> 17
<212> DNA
<213> Homo sapiens

<400> 502
caacccagat tggggtg                                                      17

<210> 503
<211> 17
<212> DNA
<213> Homo sapiens

<400> 503
tgcttctgcc accctgc                                                      17

<210> 504
<211> 17
<212> DNA
<213> Homo sapiens

<400> 504
caggtgacaa gggccct                                                      17

<210> 505
<211> 17
<212> DNA
<213> Homo sapiens

<400> 505
ggccgggggc agttctc                                                      17

<210> 506
<211> 17
<212> DNA
<213> Homo sapiens

<400> 506
gtgcgctagg gccccgg                                                      17

<210> 507
<211> 17
<212> DNA
<213> Homo sapiens

<400> 507
aggctgtcca ggctctg                                                      17

<210> 508
<211> 17
<212> DNA
<213> Homo sapiens

<400> 508
tgttatgtcc attttgc                                                      17

<210> 509
<211> 17
<212> DNA
<213> Homo sapiens

<400> 509
tttcccaaac tgtgagg                                                      17

<210> 510
<211> 17
<212> DNA
<213> Homo sapiens

<400> 510
ggggatgggg tactgcc                                                      17

<210> 511
<211> 10
<212> DNA
<213> Homo sapiens

<400> 511
gtctcagtgc                                                              10

<210> 512
<211> 10
<212> DNA
<213> Homo sapiens

<400> 512
cttatgctgc                                                              10

<210> 513
<211> 10
<212> DNA
<213> Homo sapiens

<400> 513
ccaccctcac                                                              10

<210> 514
<211> 10
<212> DNA
<213> Homo sapiens

<400> 514
gtgctacttc                                                              10

<210> 515
<211> 10
<212> DNA
<213> Homo sapiens

<400> 515
gagtgagacc                                                              10

<210> 516
<211> 10
<212> DNA
<213> Homo sapiens

<400> 516
atggcaacag                                                              10

<210> 517
<211> 10
<212> DNA
<213> Homo sapiens

<400> 517
tcacacagtg                                                              10

<210> 518
<211> 9
<212> DNA
<213> Homo sapiens

<400> 518
gaccgcagg                                                                9

<210> 519
<211> 10
<212> DNA
<213> Homo sapiens

<400> 519
gggaggggtg                                                              10

<210> 520
<211> 10
<212> DNA
<213> Homo sapiens

<400> 520
ccctaccctg                                                              10

<210> 521
<211> 10
<212> DNA
<213> Homo sapiens

<400> 521
ttctcccaaa                                                              10

<210> 522
<211> 10
<212> DNA
<213> Homo sapiens

<400> 522
ggatgcgcag                                                              10

<210> 523
<211> 10
<212> DNA
<213> Homo sapiens

<400> 523
gtgctaagcg                                                              10

<210> 524

<211> 10
<212> DNA
<213> Homo sapiens

<400> 524
cccaggacac                                                              10

<210> 525
<211> 10
<212> DNA
<213> Homo sapiens

<400> 525
tagttggaaa                                                              10

<210> 526
<211> 10
<212> DNA
<213> Homo sapiens

<400> 526
aagggcgcgg                                                              10

<210> 527
<211> 10
<212> DNA
<213> Homo sapiens

<400> 527
agctgtgcca                                                              10

<210> 528
<211> 10
<212> DNA
<213> Homo sapiens

<400> 528
acaaaatcaa                                                              10

<210> 529
<211> 10
<212> DNA
<213> Homo sapiens

<400> 529
gcctgcagtc                                                              10

<210> 530
<211> 10
<212> DNA
<213> Homo sapiens

<400> 530
accaggtcca                                                              10

<210> 531
<211> 10
<212> DNA
<213> Homo sapiens

<400> 531
ggctaattat                                                              10

<210> 532
<211> 10
<212> DNA
<213> Homo sapiens

<400> 532

tttaaatagc                                                              10

<210> 533
<211> 10
<212> DNA
<213> Homo sapiens

<400> 533
cagttcatta                                                              10

<210> 534
<211> 10
<212> DNA
<213> Homo sapiens

<400> 534
ctgccgtgac                                                              10

<210> 535
<211> 10
<212> DNA
<213> Homo sapiens

<400> 535
ttttaactta                                                              10

<210> 536
<211> 10
<212> DNA
<213> Homo sapiens

<400> 536
tagaaaccgg                                                              10

<210> 537
<211> 9
<212> DNA
<213> Homo sapiens

<400> 537
cttcttgcc                                                                9

<210> 538
<211> 10
<212> DNA
<213> Homo sapiens

<400> 538
tagaaaaaat                                                              10

<210> 539
<211> 10
<212> DNA
<213> Homo sapiens

<400> 539
gtgctacttc                                                              10

<210> 540
<211> 10
<212> DNA
<213> Homo sapiens

<400> 540
gagtgagacc                                                              10

<210> 541
<211> 10
<212> DNA

<213> Homo sapiens

<400> 541
tcacacagtg                                                              10

<210> 542
<211> 10
<212> DNA
<213> Homo sapiens

<400> 542
gaccgcagga                                                              10

<210> 543
<211> 10
<212> DNA
<213> Homo sapiens

<400> 543
gggaggggtg                                                              10

<210> 544
<211> 10
<212> DNA
<213> Homo sapiens

<400> 544
ggggctgccc                                                              10

<210> 545
<211> 10
<212> DNA
<213> Homo sapiens

<400> 545
ttctcccaaa                                                              10

<210> 546
<211> 10
<212> DNA
<213> Homo sapiens

<400> 546
ccacagggga                                                              10

<210> 547
<211> 10
<212> DNA
<213> Homo sapiens

<400> 547
tcaagttcac                                                              10

<210> 548
<211> 10
<212> DNA
<213> Homo sapiens

<400> 548
accaaaaacc                                                              10

<210> 549
<211> 10
<212> DNA
<213> Homo sapiens

<400> 549
gatcaggcca                                                              10

<210> 550
<211> 10
<212> DNA
<213> Homo sapiens

<400> 550
agaaaccacg                                                              10

<210> 551
<211> 10
<212> DNA
<213> Homo sapiens

<400> 551
aaggttcttc                                                              10

<210> 552
<211> 10
<212> DNA
<213> Homo sapiens

<400> 552
ccctttcaca                                                              10

<210> 553
<211> 10
<212> DNA
<213> Homo sapiens

<400> 553
agactagggg                                                              10

<210> 554
<211> 10
<212> DNA
<213> Homo sapiens

<400> 554
cataaacggg                                                              10

<210> 555
<211> 10
<212> DNA
<213> Homo sapiens

<400> 555
ggccaacatt                                                              10

<210> 556
<211> 10
<212> DNA
<213> Homo sapiens

<400> 556
gtacgtccca                                                              10

<210> 557
<211> 10
<212> DNA
<213> Homo sapiens

<400> 557
gcaatttaac                                                              10

<210> 558
<211> 10
<212> DNA
<213> Homo sapiens

<400> 558
aggtgcgggg                                                              10

<210> 559
<211> 10
<212> DNA
<213> Homo sapiens

<400> 559
tttggggctg                                                              10

<210> 560
<211> 10
<212> DNA
<213> Homo sapiens

<400> 560
caccctgtac                                                              10

<210> 561
<211> 10
<212> DNA
<213> Homo sapiens

<400> 561
gggtgggcgt                                                              10

<210> 562
<211> 17
<212> DNA
<213> Homo sapiens

<400> 562
gtacgtccca ccctgtc                                                      17

<210> 563
<211> 17
<212> DNA
<213> Homo sapiens

<400> 563
gcaatttaac cacattt                                                      17

<210> 564
<211> 17
<212> DNA
<213> Homo sapiens

<400> 564
aggtgcgggg ggcagac                                                      17

<210> 565
<211> 17
<212> DNA
<213> Homo sapiens

<400> 565
tttggggctg gcctcac                                                      17

<210> 566
<211> 17
<212> DNA
<213> Homo sapiens

<400> 566
caccctgtac agttgcc                                                      17

<210> 567
<211> 17

<212> DNA
<213> Homo sapiens

<400> 567
gggtgggcgt gcaggga                                                    17

<210> 568
<211> 17
<212> DNA
<213> Homo sapiens

<400> 568
actcagcccg gctgatg                                                    17

<210> 569
<211> 17
<212> DNA
<213> Homo sapiens

<400> 569
gctctctatg ctgacgt                                                    17

<210> 570
<211> 17
<212> DNA
<213> Homo sapiens

<400> 570
agaatgaaac tgccggg                                                    17

<210> 571
<211> 17
<212> DNA
<213> Homo sapiens

<400> 571
aagtggaata aactgcc                                                    17

<210> 572
<211> 17
<212> DNA
<213> Homo sapiens

<400> 572
tttgaatcag tgctaga                                                    17

<210> 573
<211> 17
<212> DNA
<213> Homo sapiens

<400> 573
ttctgctctt gtgccct                                                    17

<210> 574
<211> 17
<212> DNA
<213> Homo sapiens

<400> 574
tgggaagtgg gctcctt                                                    17

<210> 575
<211> 17
<212> DNA
<213> Homo sapiens

<400> 575
tgggcccgtg tctggcc                                                    17

<210> 576
<211> 17
<212> DNA
<213> Homo sapiens

<400> 576
acaacgtcca gctggtg                                                    17

<210> 577
<211> 17
<212> DNA
<213> Homo sapiens

<400> 577
ccccctgccc ctctgcc                                                    17

<210> 578
<211> 17
<212> DNA
<213> Homo sapiens

<400> 578
gtctatgcct cccagga                                                    17

<210> 579
<211> 17
<212> DNA
<213> Homo sapiens

<400> 579
ggctggagcc gctttgg                                                    17

<210> 580
<211> 17
<212> DNA
<213> Homo sapiens

<400> 580
catacctcct gccccgc                                                    17

<210> 581
<211> 17
<212> DNA
<213> Homo sapiens

<400> 581
tgcctgcacc aggagac                                                    17

<210> 582
<211> 17
<212> DNA
<213> Homo sapiens

<400> 582
ttccttgtaa tcaaaga                                                    17

<210> 583
<211> 17
<212> DNA
<213> Homo sapiens

<400> 583
tggcggcaga ggcagag                                                    17

<210> 584
<211> 17
<212> DNA
<213> Homo sapiens

<400> 584
ccacgtggct ggctggg                                                    17

<210> 585
<211> 17
<212> DNA
<213> Homo sapiens

<400> 585
ctggaggctg cctcggg                                                    17

<210> 586
<211> 17
<212> DNA
<213> Homo sapiens

<400> 586
atggcaacag atctgga                                                    17

<210> 587
<211> 17
<212> DNA
<213> Homo sapiens

<400> 587
ggtcccctac ccttccc                                                    17

<210> 588
<211> 17
<212> DNA
<213> Homo sapiens

<400> 588
cccgcccccg ccttccc                                                    17

<210> 589
<211> 17
<212> DNA
<213> Homo sapiens

<400> 589
cccgaggcag agtcggg                                                    17

<210> 590
<211> 17
<212> DNA
<213> Homo sapiens

<400> 590
ctacgtgatg aagatgg                                                    17

<210> 591
<211> 17
<212> DNA
<213> Homo sapiens

<400> 591
atgggtttgc attttag                                                    17

<210> 592
<211> 17
<212> DNA
<213> Homo sapiens

<400> 592
atggaagtct gcgtaac                                                    17

<210> 593

<211> 17
<212> DNA
<213> Homo sapiens

<400> 593
tggacagcag ggacctg                                                      17

<210> 594
<211> 17
<212> DNA
<213> Homo sapiens

<400> 594
tcatagaaac cttgatt                                                      17

<210> 595
<211> 17
<212> DNA
<213> Homo sapiens

<400> 595
cagcgctgca ttgactc                                                      17

<210> 596
<211> 17
<212> DNA
<213> Homo sapiens

<400> 596
ctgggtgccc cagcctg                                                      17

<210> 597
<211> 17
<212> DNA
<213> Homo sapiens

<400> 597
cgaccccacg ccacccc                                                      17

<210> 598
<211> 17
<212> DNA
<213> Homo sapiens

<400> 598
cgctgggcgt ctgggac                                                      17

<210> 599
<211> 17
<212> DNA
<213> Homo sapiens

<400> 599
attagtcaga aactgcc                                                      17

<210> 600
<211> 17
<212> DNA
<213> Homo sapiens

<400> 600
gatagcacag ttgtcag                                                      17

<210> 601
<211> 17
<212> DNA
<213> Homo sapiens

<400> 601

cgtactgagc gctttgg                                                    17

<210> 602
<211> 17
<212> DNA
<213> Homo sapiens

<400> 602
ggggcttctg tagcccc                                                    17

<210> 603
<211> 17
<212> DNA
<213> Homo sapiens

<400> 603
cccgtccgga acgtcta                                                    17

<210> 604
<211> 17
<212> DNA
<213> Homo sapiens

<400> 604
ccagtggccc ggagctg                                                    17

<210> 605
<211> 17
<212> DNA
<213> Homo sapiens

<400> 605
actgtactcc agcctag                                                    17

<210> 606
<211> 17
<212> DNA
<213> Homo sapiens

<400> 606
agctccagac ccccagc                                                    17

<210> 607
<211> 17
<212> DNA
<213> Homo sapiens

<400> 607
gactcgcaga caccggg                                                    17

<210> 608
<211> 17
<212> DNA
<213> Homo sapiens

<400> 608
ggcctccagc cacccac                                                    17

<210> 609
<211> 17
<212> DNA
<213> Homo sapiens

<400> 609
gaccagcctt cagatgg                                                    17

<210> 610
<211> 17
<212> DNA

<213> Homo sapiens

<400> 610
gcccccaggg gcaggac                                                    17

<210> 611
<211> 17
<212> DNA
<213> Homo sapiens

<400> 611
gaggatggtg tcctgag                                                    17

<210> 612
<211> 17
<212> DNA
<213> Homo sapiens

<400> 612
aagatcaaga tcattgc                                                    17

<210> 613
<211> 17
<212> DNA
<213> Homo sapiens

<400> 613
agcacagggt cgggggg                                                    17

<210> 614
<211> 17
<212> DNA
<213> Homo sapiens

<400> 614
gccaagtgaa ctgtggc                                                    17

<210> 615
<211> 17
<212> DNA
<213> Homo sapiens

<400> 615
cgtgggtggg gagggag                                                    17

<210> 616
<211> 17
<212> DNA
<213> Homo sapiens

<400> 616
ctcccctatg gactggc                                                    17

<210> 617
<211> 17
<212> DNA
<213> Homo sapiens

<400> 617
agtatgagga aatctct                                                    17

<210> 618
<211> 17
<212> DNA
<213> Homo sapiens

<400> 618
gccccttcc tcccttg                                                     17

<210> 619
<211> 17
<212> DNA
<213> Homo sapiens

<400> 619
gggatttctg tgtctgc                                                      17

<210> 620
<211> 17
<212> DNA
<213> Homo sapiens

<400> 620
cttccagcta acaggtc                                                      17

<210> 621
<211> 17
<212> DNA
<213> Homo sapiens

<400> 621
gccttgggtg acaaatt                                                      17

<210> 622
<211> 17
<212> DNA
<213> Homo sapiens

<400> 622
gcctcccgcc acggggc                                                      17

<210> 623
<211> 17
<212> DNA
<213> Homo sapiens

<400> 623
gtgtgggggg ctggggg                                                      17

<210> 624
<211> 17
<212> DNA
<213> Homo sapiens

<400> 624
gactcttcag tctggag                                                      17

<210> 625
<211> 17
<212> DNA
<213> Homo sapiens

<400> 625
gccacaccca ccgcccc                                                      17

<210> 626
<211> 17
<212> DNA
<213> Homo sapiens

<400> 626
ccagggcaac agaatga                                                      17

<210> 627
<211> 17
<212> DNA
<213> Homo sapiens

<400> 627
caccctgtac agttgcc                                                    17

<210> 628
<211> 17
<212> DNA
<213> Homo sapiens

<400> 628
gacgaatatg aatgtca                                                    17

<210> 629
<211> 17
<212> DNA
<213> Homo sapiens

<400> 629
caaactggtc taggtca                                                    17

<210> 630
<211> 17
<212> DNA
<213> Homo sapiens

<400> 630
gtaatgacag atgcaag                                                    17

<210> 631
<211> 17
<212> DNA
<213> Homo sapiens

<400> 631
tggccccagg tgccacc                                                    17

<210> 632
<211> 17
<212> DNA
<213> Homo sapiens

<400> 632
aacacaatca tatgatg                                                    17

<210> 633
<211> 17
<212> DNA
<213> Homo sapiens

<400> 633
gagggtatac tgagggg                                                    17

<210> 634
<211> 17
<212> DNA
<213> Homo sapiens

<400> 634
ggagccagct gacctgc                                                    17

<210> 635
<211> 17
<212> DNA
<213> Homo sapiens

<400> 635
gagcctcagg tgctccc                                                    17

<210> 636
<211> 17

<212> DNA
<213> Homo sapiens

<400> 636
tacttcacat acagtgc                                                      17

<210> 637
<211> 17
<212> DNA
<213> Homo sapiens

<400> 637
cggtgggacc accctgc                                                      17

<210> 638
<211> 17
<212> DNA
<213> Homo sapiens

<400> 638
ggagaaacag ctgctga                                                      17

<210> 639
<211> 17
<212> DNA
<213> Homo sapiens

<400> 639
gctcaggtct gccgggg                                                      17

<210> 640
<211> 17
<212> DNA
<213> Homo sapiens

<400> 640
tctgcactga gaaactg                                                      17

<210> 641
<211> 17
<212> DNA
<213> Homo sapiens

<400> 641
tccacgccct tcctggc                                                      17

<210> 642
<211> 17
<212> DNA
<213> Homo sapiens

<400> 642
ttgcaatagc aaaaccc                                                      17

<210> 643
<211> 17
<212> DNA
<213> Homo sapiens

<400> 643
tccaaatcga tgtggat                                                      17

<210> 644
<211> 17
<212> DNA
<213> Homo sapiens

<400> 644
agggcttcca atgtgct                                                      17

<210> 645
<211> 17
<212> DNA
<213> Homo sapiens

<400> 645
aacctgggag gtggagg                                                      17

<210> 646
<211> 17
<212> DNA
<213> Homo sapiens

<400> 646
ggccaacatt tggtcca                                                      17

<210> 647
<211> 17
<212> DNA
<213> Homo sapiens

<400> 647
ggggctggag gggggca                                                      17

<210> 648
<211> 17
<212> DNA
<213> Homo sapiens

<400> 648
ggatgcgcag gggaggc                                                      17

<210> 649
<211> 17
<212> DNA
<213> Homo sapiens

<400> 649
gaagacactt ggtttga                                                      17

<210> 650
<211> 17
<212> DNA
<213> Homo sapiens

<400> 650
gagagaagag tgatctg                                                      17

<210> 651
<211> 17
<212> DNA
<213> Homo sapiens

<400> 651
gcagggtggg gaggggt                                                      17

<210> 652
<211> 17
<212> DNA
<213> Homo sapiens

<400> 652
tcagtgtatt aaaaccc                                                      17

<210> 653
<211> 17
<212> DNA
<213> Homo sapiens

<400> 653
atactataat tgtgaga                                                    17

<210> 654
<211> 17
<212> DNA
<213> Homo sapiens

<400> 654
gctggttcct gagtggc                                                    17

<210> 655
<211> 17
<212> DNA
<213> Homo sapiens

<400> 655
agccactgcg cccggcc                                                    17

<210> 656
<211> 17
<212> DNA
<213> Homo sapiens

<400> 656
agactagggg ccggagc                                                    17

<210> 657
<211> 17
<212> DNA
<213> Homo sapiens

<400> 657
gggacagctg tctgtgg                                                    17

<210> 658
<211> 17
<212> DNA
<213> Homo sapiens

<400> 658
aacccaggag gcggagc                                                    17

<210> 659
<211> 17
<212> DNA
<213> Homo sapiens

<400> 659
cagcctgagg ctcttgg                                                    17

<210> 660
<211> 17
<212> DNA
<213> Homo sapiens

<400> 660
cctcccctgc acctggg                                                    17

<210> 661
<211> 17
<212> DNA
<213> Homo sapiens

<400> 661
gcttcagtgg gggagag                                                    17

<210> 662

<211> 17
<212> DNA
<213> Homo sapiens

<400> 662
tgtttggggg cttttag                                                      17

<210> 663
<211> 17
<212> DNA
<213> Homo sapiens

<400> 663
ttttaaatta ggttttg                                                      17

<210> 664
<211> 17
<212> DNA
<213> Homo sapiens

<400> 664
atctcaaaga tacacag                                                      17

<210> 665
<211> 17
<212> DNA
<213> Homo sapiens

<400> 665
tttgtgggca gtcaggc                                                      17

<210> 666
<211> 17
<212> DNA
<213> Homo sapiens

<400> 666
attgtagaca atgaggg                                                      17

<210> 667
<211> 17
<212> DNA
<213> Homo sapiens

<400> 667
gcaaaaccct gctctcc                                                      17

<210> 668
<211> 17
<212> DNA
<213> Homo sapiens

<400> 668
gtctcagtgc tgaggcg                                                      17

<210> 669
<211> 17
<212> DNA
<213> Homo sapiens

<400> 669
agccactgtg cccggcc                                                      17

<210> 670
<211> 17
<212> DNA
<213> Homo sapiens

<400> 670

tgattaaggt cggcgct                                                    17

<210> 671
<211> 17
<212> DNA
<213> Homo sapiens

<400> 671
ttacaaacag aaaagct                                                    17

<210> 672
<211> 17
<212> DNA
<213> Homo sapiens

<400> 672
tttattattg ttcaata                                                    17

<210> 673
<211> 17
<212> DNA
<213> Homo sapiens

<400> 673
cgggcctcag gtggcag                                                    17

<210> 674
<211> 17
<212> DNA
<213> Homo sapiens

<400> 674
taaaggcaca gtggctc                                                    17

<210> 675
<211> 17
<212> DNA
<213> Homo sapiens

<400> 675
atattaggaa gtcgggg                                                    17

<210> 676
<211> 17
<212> DNA
<213> Homo sapiens

<400> 676
tgattaaaac aagttgc                                                    17

<210> 677
<211> 17
<212> DNA
<213> Homo sapiens

<400> 677
ttgtgcacgc atcagtg                                                    17

<210> 678
<211> 17
<212> DNA
<213> Homo sapiens

<400> 678
cctgcctcgt agtgaag                                                    17

<210> 679
<211> 17
<212> DNA

<213> Homo sapiens

<400> 679
caaaactgtt tgttggc                                                      17

<210> 680
<211> 17
<212> DNA
<213> Homo sapiens

<400> 680
taggaaagta aaatgga                                                      17

<210> 681
<211> 17
<212> DNA
<213> Homo sapiens

<400> 681
ctccatcggc tgtgagg                                                      17

<210> 682
<211> 17
<212> DNA
<213> Homo sapiens

<400> 682
caagcatccc cgttcca                                                      17

<210> 683
<211> 17
<212> DNA
<213> Homo sapiens

<400> 683
gagttaggca cttcctg                                                      17

<210> 684
<211> 17
<212> DNA
<213> Homo sapiens

<400> 684
cataaacggg cacaccc                                                      17

<210> 685
<211> 17
<212> DNA
<213> Homo sapiens

<400> 685
ttgcctggga tgctggt                                                      17

<210> 686
<211> 17
<212> DNA
<213> Homo sapiens

<400> 686
aacgcggcca atgtggg                                                      17

<210> 687
<211> 17
<212> DNA
<213> Homo sapiens

<400> 687
ggtttggctt aggctgg                                                      17

<210> 688
<211> 17
<212> DNA
<213> Homo sapiens

<400> 688
gatttttgtg gtgtggg                                                      17

<210> 689
<211> 17
<212> DNA
<213> Homo sapiens

<400> 689
ggctgccctg ggcagcc                                                      17

<210> 690
<211> 17
<212> DNA
<213> Homo sapiens

<400> 690
atggcaacag aaaccaa                                                      17

<210> 691
<211> 17
<212> DNA
<213> Homo sapiens

<400> 691
agagcaaacc gtagtcc                                                      17

<210> 692
<211> 17
<212> DNA
<213> Homo sapiens

<400> 692
tgcacttcaa gaaaatg                                                      17

<210> 693
<211> 17
<212> DNA
<213> Homo sapiens

<400> 693
ccctaccctg ttacctt                                                      17

<210> 694
<211> 17
<212> DNA
<213> Homo sapiens

<400> 694
ggaaaaatgt tggaatg                                                      17

<210> 695
<211> 17
<212> DNA
<213> Homo sapiens

<400> 695
gtttttgctt cagcggc                                                      17

<210> 696
<211> 17
<212> DNA
<213> Homo sapiens

<400> 696
gaggacccaa caggagg                                                      17

<210> 697
<211> 17
<212> DNA
<213> Homo sapiens

<400> 697
actgaggaaa ggagctc                                                      17

<210> 698
<211> 17
<212> DNA
<213> Homo sapiens

<400> 698
tgcagcgcct gcggcct                                                      17

<210> 699
<211> 17
<212> DNA
<213> Homo sapiens

<400> 699
   ctggggggaa gggactg                                                       17

<210> 700
<211> 17
<212> DNA
<213> Homo sapiens

<400> 700
gtgcctgaga ggcaggc                                                      17

<210> 701
<211> 17
<212> DNA
<213> Homo sapiens

<400> 701
tcacagggtc cccgggg                                                      17

<210> 702
<211> 17
<212> DNA
<213> Homo sapiens

<400> 702
ggagggggct tgaagcc                                                      17

<210> 703
<211> 17
<212> DNA
<213> Homo sapiens

<400> 703
ggaggaattc atcttca                                                      17

<210> 704
<211> 17
<212> DNA
<213> Homo sapiens

<400> 704
gaaagtggct gtcctgg                                                      17

<210> 705
<211> 17

<212> DNA
<213> Homo sapiens

<400> 705
tccctggctg ttgaggc                                                      17

<210> 706
<211> 17
<212> DNA
<213> Homo sapiens

<400> 706
aagatgaggg ggcaggc                                                      17

<210> 707
<211> 17
<212> DNA
<213> Homo sapiens

<400> 707
ccaacaagaa tgcattg                                                      17

<210> 708
<211> 17
<212> DNA
<213> Homo sapiens

<400> 708
aaggatgcgg tgatggc                                                      17

<210> 709
<211> 17
<212> DNA
<213> Homo sapiens

<400> 709
tgcagtcact ggtgtca                                                      17

<210> 710
<211> 17
<212> DNA
<213> Homo sapiens

<400> 710
gccgtgtaga ccctaac                                                      17

<210> 711
<211> 17
<212> DNA
<213> Homo sapiens

<400> 711
caggcttttt ggcttcc                                                      17

<210> 712
<211> 17
<212> DNA
<213> Homo sapiens

<400> 712
tccctgggca gcttcag                                                      17

<210> 713
<211> 17
<212> DNA
<213> Homo sapiens

<400> 713
gagcgcagcg agctagc                                                      17

<210> 714
<211> 17
<212> DNA
<213> Homo sapiens

<400> 714
caggtggttc tgccatc                                                      17

<210> 715
<211> 17
<212> DNA
<213> Homo sapiens

<400> 715
gcccacatcc gctgagg                                                      17

<210> 716
<211> 17
<212> DNA
<213> Homo sapiens

<400> 716
aaggttcttc tcaaggg                                                      17

<210> 717
<211> 10
<212> DNA
<213> Homo sapiens

<400> 717
actcagcccg                                                              10

<210> 718
<211> 10
<212> DNA
<213> Homo sapiens

<400> 718
gctctctatg                                                              10

<210> 719
<211> 10
<212> DNA
<213> Homo sapiens

<400> 719
agaatgaaac                                                              10

<210> 720
<211> 10
<212> DNA
<213> Homo sapiens

<400> 720
aagtggaata                                                              10

<210> 721
<211> 10
<212> DNA
<213> Homo sapiens

<400> 721
tttgaatcag                                                              10

<210> 722
<211> 10
<212> DNA
<213> Homo sapiens

<400> 722
ttctgctctt                                                              10

<210> 723
<211> 10
<212> DNA
<213> Homo sapiens

<400> 723
tgggaagtgg                                                              10

<210> 724
<211> 10
<212> DNA
<213> Homo sapiens

<400> 724
tgggcccgtg                                                              10

<210> 725
<211> 10
<212> DNA
<213> Homo sapiens

<400> 725
acaacgtcca                                                              10

<210> 726
<211> 10
<212> DNA
<213> Homo sapiens

<400> 726
ccccctgccc                                                              10

<210> 727
<211> 10
<212> DNA
<213> Homo sapiens

<400> 727
gtctatgcct                                                              10

<210> 728
<211> 10
<212> DNA
<213> Homo sapiens

<400> 728
ggctggagcc                                                              10

<210> 729
<211> 10
<212> DNA
<213> Homo sapiens

<400> 729
catacctcct                                                              10

<210> 730
<211> 10
<212> DNA
<213> Homo sapiens

<400> 730
tgcctgcacc                                                              10

<210> 731

<211> 10
<212> DNA
<213> Homo sapiens

<400> 731
ttccttgtaa                                                              10

<210> 732
<211> 10
<212> DNA
<213> Homo sapiens

<400> 732
tggcggcaga                                                              10

<210> 733
<211> 10
<212> DNA
<213> Homo sapiens

<400> 733
ccacgtggct                                                              10

<210> 734
<211> 10
<212> DNA
<213> Homo sapiens

<400> 734
ctggaggctg                                                              10

<210> 735
<211> 10
<212> DNA
<213> Homo sapiens

<400> 735
atggcaacag                                                              10

<210> 736
<211> 10
<212> DNA
<213> Homo sapiens

<400> 736
ggtcccctac                                                              10

<210> 737
<211> 10
<212> DNA
<213> Homo sapiens

<400> 737
cccgcccccg                                                              10

<210> 738
<211> 10
<212> DNA
<213> Homo sapiens

<400> 738
cccgaggcag                                                              10

<210> 739
<211> 10
<212> DNA
<213> Homo sapiens

<400> 739

ctacgtgatg                                                              10

<210> 740
<211> 10
<212> DNA
<213> Homo sapiens

<400> 740
atgggtttgc                                                              10

<210> 741
<211> 10
<212> DNA
<213> Homo sapiens

<400> 741
atggaagtct                                                              10

<210> 742
<211> 10
<212> DNA
<213> Homo sapiens

<400> 742
tggacagcag                                                              10

<210> 743
<211> 10
<212> DNA
<213> Homo sapiens

<400> 743
tcatagaaac                                                              10

<210> 744
<211> 10
<212> DNA
<213> Homo sapiens

<400> 744
cagcgctgca                                                              10

<210> 745
<211> 10
<212> DNA
<213> Homo sapiens

<400> 745
ctgggtgccc                                                              10

<210> 746
<211> 10
<212> DNA
<213> Homo sapiens

<400> 746
cgaccccacg                                                              10

<210> 747
<211> 10
<212> DNA
<213> Homo sapiens

<400> 747
cgctgggcgt                                                              10

<210> 748
<211> 10
<212> DNA

<213> Homo sapiens

<400> 748
attagtcaga                                                              10

<210> 749
<211> 10
<212> DNA
<213> Homo sapiens

<400> 749
gatagcacag                                                              10

<210> 750
<211> 10
<212> DNA
<213> Homo sapiens

<400> 750
cgtactgagc                                                              10

<210> 751
<211> 10
<212> DNA
<213> Homo sapiens

<400> 751
ggggcttctg                                                              10

<210> 752
<211> 10
<212> DNA
<213> Homo sapiens

<400> 752
cccgtccgga                                                              10

<210> 753
<211> 10
<212> DNA
<213> Homo sapiens

<400> 753
ccagtggccc                                                              10

<210> 754
<211> 10
<212> DNA
<213> Homo sapiens

<400> 754
actgtactcc                                                              10

<210> 755
<211> 10
<212> DNA
<213> Homo sapiens

<400> 755
agctccagac                                                              10

<210> 756
<211> 10
<212> DNA
<213> Homo sapiens

<400> 756
gactcgcaga                                                              10

<210> 757
<211> 10
<212> DNA
<213> Homo sapiens

<400> 757
ggcctccagc                                                              10

<210> 758
<211> 10
<212> DNA
<213> Homo sapiens

<400> 758
gaccagcctt                                                              10

<210> 759
<211> 10
<212> DNA
<213> Homo sapiens

<400> 759
gcccccaggg                                                              10

<210> 760
<211> 10
<212> DNA
<213> Homo sapiens

<400> 760
gaggatggtg                                                              10

<210> 761
<211> 10
<212> DNA
<213> Homo sapiens

<400> 761
aagatcaaga                                                              10

<210> 762
<211> 10
<212> DNA
<213> Homo sapiens

<400> 762
agcacagggt                                                              10

<210> 763
<211> 10
<212> DNA
<213> Homo sapiens

<400> 763
gccaagtgaa                                                              10

<210> 764
<211> 10
<212> DNA
<213> Homo sapiens

<400> 764
cgtgggtggg                                                              10

<210> 765
<211> 10
<212> DNA
<213> Homo sapiens

<400> 765
ctcccctatg                                                              10

<210> 766
<211> 10
<212> DNA
<213> Homo sapiens

<400> 766
agtatgagga                                                              10

<210> 767
<211> 10
<212> DNA
<213> Homo sapiens

<400> 767
   gcccccttcc                                                             10

<210> 768
<211> 10
<212> DNA
<213> Homo sapiens

<400> 768
gggatttctg                                                              10

<210> 769
<211> 10
<212> DNA
<213> Homo sapiens

<400> 769
cttccagcta                                                              10

<210> 770
<211> 10
<212> DNA
<213> Homo sapiens

<400> 770
gccttgggtg                                                              10

<210> 771
<211> 10
<212> DNA
<213> Homo sapiens

<400> 771
gcctcccgcc                                                              10

<210> 772
<211> 10
<212> DNA
<213> Homo sapiens

<400> 772
gtgtgggggg                                                              10

<210> 773
<211> 10
<212> DNA
<213> Homo sapiens

<400> 773
gactcttcag                                                              10

<210> 774
<211> 10

<212> DNA
<213> Homo sapiens

<400> 774
gccacaccca                                                              10

<210> 775
<211> 10
<212> DNA
<213> Homo sapiens

<400> 775
ccagggcaac                                                              10

<210> 776
<211> 10
<212> DNA
<213> Homo sapiens

<400> 776
caccctgtac                                                              10

<210> 777
<211> 10
<212> DNA
<213> Homo sapiens

<400> 777
gacgaatatg                                                              10

<210> 778
<211> 10
<212> DNA
<213> Homo sapiens

<400> 778
caaactggtc                                                              10

<210> 779
<211> 10
<212> DNA
<213> Homo sapiens

<400> 779
gtaatgacag                                                              10

<210> 780
<211> 10
<212> DNA
<213> Homo sapiens

<400> 780
tggccccagg                                                              10

<210> 781
<211> 10
<212> DNA
<213> Homo sapiens

<400> 781
aacacaatca                                                              10

<210> 782
<211> 10
<212> DNA
<213> Homo sapiens

<400> 782
gagggtatac                                                              10

<210> 783
<211> 10
<212> DNA
<213> Homo sapiens

<400> 783
ggagccagct                                                              10

<210> 784
<211> 10
<212> DNA
<213> Homo sapiens

<400> 784
gagcctcagg                                                              10

<210> 785
<211> 10
<212> DNA
<213> Homo sapiens

<400> 785
tacttcacat                                                              10

<210> 786
<211> 10
<212> DNA
<213> Homo sapiens

<400> 786
cggtgggacc                                                              10

<210> 787
<211> 10
<212> DNA
<213> Homo sapiens

<400> 787
ggagaaacag                                                              10

<210> 788
<211> 10
<212> DNA
<213> Homo sapiens

<400> 788
gctcaggtct                                                              10

<210> 789
<211> 10
<212> DNA
<213> Homo sapiens

<400> 789
tctgcactga                                                              10

<210> 790
<211> 10
<212> DNA
<213> Homo sapiens

<400> 790
tccacgccct                                                              10

<210> 791
<211> 10
<212> DNA
<213> Homo sapiens

<400> 791
ttgcaatagc                                                              10

<210> 792
<211> 10
<212> DNA
<213> Homo sapiens

<400> 792
tccaaatcga                                                              10

<210> 793
<211> 10
<212> DNA
<213> Homo sapiens

<400> 793
agggcttcca                                                              10

<210> 794
<211> 10
<212> DNA
<213> Homo sapiens

<400> 794
aacctgggag                                                              10

<210> 795
<211> 10
<212> DNA
<213> Homo sapiens

<400> 795
ggccaacatt                                                              10

<210> 796
<211> 10
<212> DNA
<213> Homo sapiens

<400> 796
ggggctggag                                                              10

<210> 797
<211> 10
<212> DNA
<213> Homo sapiens

<400> 797
ggatgcgcag                                                              10

<210> 798
<211> 10
<212> DNA
<213> Homo sapiens

<400> 798
gaagacactt                                                              10

<210> 799
<211> 10
<212> DNA
<213> Homo sapiens

<400> 799
gagagaagag                                                              10

<210> 800

<211> 10
<212> DNA
<213> Homo sapiens

<400> 800
gcagggtggg                                                              10

<210> 801
<211> 10
<212> DNA
<213> Homo sapiens

<400> 801
tcagtgtatt                                                              10

<210> 802
<211> 10
<212> DNA
<213> Homo sapiens

<400> 802
atactataat                                                              10

<210> 803
<211> 10
<212> DNA
<213> Homo sapiens

<400> 803
gctggttcct                                                              10

<210> 804
<211> 10
<212> DNA
<213> Homo sapiens

<400> 804
agccactgcg                                                              10

<210> 805
<211> 10
<212> DNA
<213> Homo sapiens

<400> 805
agactagggg                                                              10

<210> 806
<211> 10
<212> DNA
<213> Homo sapiens

<400> 806
gggacagctg                                                              10

<210> 807
<211> 10
<212> DNA
<213> Homo sapiens

<400> 807
aacccaggag                                                              10

<210> 808
<211> 10
<212> DNA
<213> Homo sapiens

<400> 808

cagcctgagg                                                              10

<210> 809
<211> 10
<212> DNA
<213> Homo sapiens

<400> 809
cctcccctgc                                                              10

<210> 810
<211> 10
<212> DNA
<213> Homo sapiens

<400> 810
gcttcagtgg                                                              10

<210> 811
<211> 10
<212> DNA
<213> Homo sapiens

<400> 811
tgtttggggg                                                              10

<210> 812
<211> 10
<212> DNA
<213> Homo sapiens

<400> 812
ttttaaatta                                                              10

<210> 813
<211> 10
<212> DNA
<213> Homo sapiens

<400> 813
atctcaaaga                                                              10

<210> 814
<211> 10
<212> DNA
<213> Homo sapiens

<400> 814
tttgtgggca                                                              10

<210> 815
<211> 10
<212> DNA
<213> Homo sapiens

<400> 815
attgtagaca                                                              10

<210> 816
<211> 10
<212> DNA
<213> Homo sapiens

<400> 816
gcaaaaccct                                                              10

<210> 817
<211> 10
<212> DNA

<213> Homo sapiens

<400> 817
gtctcagtgc                                                              10

<210> 818
<211> 10
<212> DNA
<213> Homo sapiens

<400> 818
agccactgtg                                                              10

<210> 819
<211> 10
<212> DNA
<213> Homo sapiens

<400> 819
tgattaaggt                                                              10

<210> 820
<211> 10
<212> DNA
<213> Homo sapiens

<400> 820
ttacaaacag                                                              10

<210> 821
<211> 10
<212> DNA
<213> Homo sapiens

<400> 821
tttattattg                                                              10

<210> 822
<211> 10
<212> DNA
<213> Homo sapiens

<400> 822
cgggcctcag                                                              10

<210> 823
<211> 10
<212> DNA
<213> Homo sapiens

<400> 823
taaaggcaca                                                              10

<210> 824
<211> 10
<212> DNA
<213> Homo sapiens

<400> 824
atattaggaa                                                              10

<210> 825
<211> 10
<212> DNA
<213> Homo sapiens

<400> 825
tgattaaaac                                                              10

<210> 826
<211> 10
<212> DNA
<213> Homo sapiens

<400> 826
ttgtgcacgc                                                              10

<210> 827
<211> 10
<212> DNA
<213> Homo sapiens

<400> 827
cctgcctcgt                                                              10

<210> 828
<211> 10
<212> DNA
<213> Homo sapiens

<400> 828
caaaactgtt                                                              10

<210> 829
<211> 10
<212> DNA
<213> Homo sapiens

<400> 829
taggaaagta                                                              10

<210> 830
<211> 10
<212> DNA
<213> Homo sapiens

<400> 830
ctccatcggc                                                              10

<210> 831
<211> 10
<212> DNA
<213> Homo sapiens

<400> 831
caagcatccc                                                              10

<210> 832
<211> 10
<212> DNA
<213> Homo sapiens

<400> 832
gagttaggca                                                              10

<210> 833
<211> 10
<212> DNA
<213> Homo sapiens

<400> 833
cataaacggg                                                              10

<210> 834
<211> 10
<212> DNA
<213> Homo sapiens

<400> 834
ttgcctggga                                                              10

<210> 835
<211> 10
<212> DNA
<213> Homo sapiens

<400> 835
aacgcggcca                                                              10

<210> 836
<211> 10
<212> DNA
<213> Homo sapiens

<400> 836
ggtttggctt                                                              10

<210> 837
<211> 10
<212> DNA
<213> Homo sapiens

<400> 837
gattttgtg                                                               10

<210> 838
<211> 10
<212> DNA
<213> Homo sapiens

<400> 838
ggctgccctg                                                              10

<210> 839
<211> 10
<212> DNA
<213> Homo sapiens

<400> 839
atggcaacag                                                              10

<210> 840
<211> 10
<212> DNA
<213> Homo sapiens

<400> 840
agagcaaacc                                                              10

<210> 841
<211> 10
<212> DNA
<213> Homo sapiens

<400> 841
tgcacttcaa                                                              10

<210> 842
<211> 10
<212> DNA
<213> Homo sapiens

<400> 842
ccctaccctg                                                              10

<210> 843
<211> 10

<212> DNA
<213> Homo sapiens

<400> 843
ggaaaaatgt                                                              10

<210> 844
<211> 10
<212> DNA
<213> Homo sapiens

<400> 844
gtttttgctt                                                              10

<210> 845
<211> 10
<212> DNA
<213> Homo sapiens

<400> 845
gaggacccaa                                                              10

<210> 846
<211> 10
<212> DNA
<213> Homo sapiens

<400> 846
actgaggaaa                                                              10

<210> 847
<211> 10
<212> DNA
<213> Homo sapiens

<400> 847
tgcagcgcct                                                              10

<210> 848
<211> 10
<212> DNA
<213> Homo sapiens

<400> 848
ctggggggaa                                                              10

<210> 849
<211> 10
<212> DNA
<213> Homo sapiens

<400> 849
gtgcctgaga                                                              10

<210> 850
<211> 10
<212> DNA
<213> Homo sapiens

<400> 850
tcacagggtc                                                              10

<210> 851
<211> 10
<212> DNA
<213> Homo sapiens

<400> 851
ggagggggct                                                              10

<210> 852
<211> 10
<212> DNA
<213> Homo sapiens

<400> 852
ggaggaattc                                                              10

<210> 853
<211> 10
<212> DNA
<213> Homo sapiens

<400> 853
gaaagtggct                                                              10

<210> 854
<211> 10
<212> DNA
<213> Homo sapiens

<400> 854
tccctggctg                                                              10

<210> 855
<211> 10
<212> DNA
<213> Homo sapiens

<400> 855
aagatgaggg                                                              10

<210> 856
<211> 10
<212> DNA
<213> Homo sapiens

<400> 856
ccaacaagaa                                                              10

<210> 857
<211> 10
<212> DNA
<213> Homo sapiens

<400> 857
aaggatgcgg                                                              10

<210> 858
<211> 10
<212> DNA
<213> Homo sapiens

<400> 858
tgcagtcact                                                              10

<210> 859
<211> 10
<212> DNA
<213> Homo sapiens

<400> 859
gccgtgtaga                                                              10

<210> 860
<211> 10
<212> DNA
<213> Homo sapiens

<400> 860
caggcttttt                                                                10

<210> 861
<211> 10
<212> DNA
<213> Homo sapiens

<400> 861
tccctgggca                                                                10

<210> 862
<211> 10
<212> DNA
<213> Homo sapiens

<400> 862
gagcgcagcg                                                                10

<210> 863
<211> 10
<212> DNA
<213> Homo sapiens

<400> 863
caggtggttc                                                                10

<210> 864
<211> 10
<212> DNA
<213> Homo sapiens

<400> 864
gcccacatcc                                                                10

<210> 865
<211> 10
<212> DNA
<213> Homo sapiens

<400> 865
aaggttcttc                                                                10

<210> 866
<211> 10
<212> DNA
<213> Homo sapiens

<400> 866
ggctaattat                                                                10

<210> 867
<211> 10
<212> DNA
<213> Homo sapiens

<400> 867
caaaaataaa                                                                10

<210> 868
<211> 17
<212> DNA
<213> Homo sapiens

<400> 868
ggctaattat catcaat                                                        17

<210> 869

<211> 16
<212> DNA
<213> Homo sapiens

<400> 869
caaaaataaa agccga                                                        16